# EUROPÄISCHE PATENTSCHRIFT

(11) **EP 2 349 989 B1**
(45) Veröffentlichungstag und Bekanntmachung des Hinweises auf die Patenterteilung: **18.06.2014**
(21) Anmeldenummer: 09736857.5
(22) Anmeldetag: 15.10.2009
(51) Int. Cl.: C07C 309/17, A61Q 1/02, A61Q 5/06, A61Q 15/00, A61Q 17/04, A61Q 19/04, A61K 8/46, A61K 31/255

(54) **MISCHUNGEN VON ALKYLSULFOSUCCINATEN UND IHRE VERWENDUNG**
MIXTURES OF ALKYLSULFOSUCCINATES AND THEIR USE
MÉLANGES DE SULFOSUCCINATES ALKYLÉS ET LEUR UTILISATION

(30) Priorität: 24.10.2008 EP 08018623; 05.08.2009 EP 09010116; 04.09.2009 EP 09011350
(43) Veröffentlichungstag der Anmeldung: 03.08.2011
(73) Patentinhaber: Cognis IP Management GmbH, 40589 Düsseldorf (DE)
(72) Erfinder: BEHLER, Ansgar, 46240 Bottrop (DE); ESKUCHEN, Rainer, 40764 Langenfeld (DE); FOLGE, Almud, 42657 Solingen (DE); GONDEK, Helga, 40589 Düsseldorf (DE); WEICHOLD, Catherine, 52072 Aachen (DE)
(86) Internationale Anmeldenummer: PCT/EP2009/007382
(87) Internationale Veröffentlichungsnummer: WO 2010/046048

(56) Entgegenhaltungen:
- WO-A1-01/89472
- DE-A1- 2 507 520
- DE-A1- 4 325 923
- DE-C- 868 154
- US-A- 2 176 423

## Beschreibung

Die vorliegende Erfindung betrifft Mischungen von Alkylsulfosuccinaten sowie ihre Verwendung in kosmetischen und/oder pharmazeutischen Zubereitungen.

### Stand der Technik

In kosmetischen und pharmazeutischen Zubereitungen müssen eine Vielzahl von Stoffen stabil und homogen vorliegen. So ist z.B. die stabile und einfache Emulgierung von Öl- und Wasser in verschiedenen Emulsionstypen eine andauernde Aufgabe. Weiterhin müssen auch eine Vielzahl von Wirk- oder Inhaltsstoffen, die z.T. als Feststoffe vorliegen, in den Zubereitungen homogen dispergiert bzw. stabilisiert vorliegen, um u.a. ein gleichmäßige Verteilung des Wirkstoffs im kosmetischen und/oder pharmazeutischen Träger zu gewährleisten. Gleiches gilt für flüssige Wirk- oder Inhaltsstoffe. Trotz der Vielzahl der bereits auf dem Markt erhältlichen Verbindungen besteht ein anhaltendes Interesse daran neuen Verbindungen bereit zu stellen, die diese Aufgabe lösen. Bei bekannten Produkten (wie z.B. Emulgatoren, Dispergatoren oder Stabilisatoren) sind vor allem die Elektrolytverträglichkeit, die Pigmentverträglichkeit sowie die Alkalistabilität verbesserungsbedürftig. Weiterhin ist von Interesse, dass sich die Produkte über einen Sprühtrocknungsprozess als Pulver formulieren lassen, da dies sowohl die Handhabbarkeit der Produkte als auch die Verarbeitbarkeit deutlich erleichtert. Weiterhin ist von Interesse, Produkte bereit zu stellen, des es ermöglichen kosmetische und/oder pharmazeutische Zubereitungen zu erhalten, die auch bei niedrigem pH-Wert stabil sind. Weiterhin ist insbesondere bei Zubereitungen in Emulsionsform die Lagerstabilität von Interesse: hierbei sind zu nennen: die Phasenstabilität (keine Phasentrennung), eine stabile Teilchengröße sowie die Viskositätsstabilität.

DE 25 07 520 offenbart die Herstellung einer Mischung von C16 und C18 Sulfosuccinatmonoestern, die als Bestandteil von Tensiden verwendet wird.

Die Aufgabe der vorliegenden Erfindung hat darin bestanden, neue Produkte bereit zu stellen, welche sich als Inhaltsstoffe, insbesondere als grenzflächenaktive Substanzen wie z.B. als Emulgatoren und/oder Dispergatoren in kosmetischen und/oder pharmazeutischen Zubereitungen eignen. Hierbei waren vor allem eine hohe Elektrolytverträglichkeit, eine gute Pigmentverträglichkeit sowie eine hohe Alkalistabilität von Interesse. Weiterhin sollten die Produkte einfach als Pulver herstellbar sein. Von Interesse war weiterhin, dass die mit den Produkten erhältlichen kosmetischen und/oder pharmazeutischen Zubereitungen über einen breiten pH-Bereich stabil sind. Weiterhin war von Interesse, dass die Zubereitungen eine hohe Lagerstabilität aufweisen: hierbei waren die Vermeidung der Phasenseparation und/oder eine stabile Teilchengröße der Emulsionen sowie die Stabilität der Viskosität von Interesse. Überraschenderweise wurde gefunden, dass die erfindungsgemäßen Mischungen der Alkylsulfosuccinate diese Aufgaben lösen.

### Beschreibung der Erfindung

### Mischungen von Alkylsulfosuccinatmonoestern

Der Begriff "Alkylsulfosuccinat" wird im Sinne der vorliegenden Erfindung synonym mit dem Begriff Alkylsulfosuccinatmonoester verwendet und bezeichnet alle Verbindungen der allgemeinen Formel (I) und/oder (II) 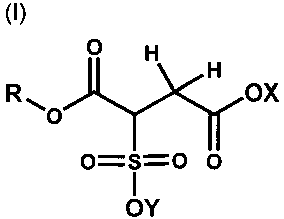 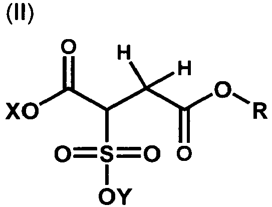 worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium.

Gegenstand der Erfindung sind Mischungen von Alkylsulfosuccinatmonoestern der allgemeinen Formel (I) und/oder (II) 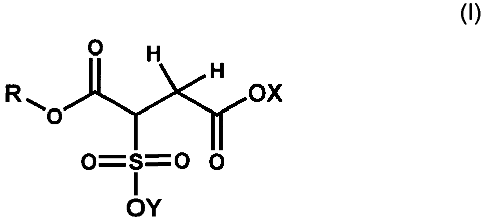 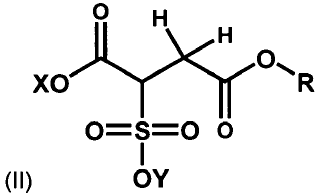
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Alkylsulfosuccinatmonoester der Formel (I) und (II).

Diese erfindungsgemäßen Mischungen können die Alkylsulfosuccinatmonoester der Formel (I) und (II) in beliebigen Anteilen enthalten. Die erfindungsgemäßen Mischungen können beispielsweise 0 bis 90%, 0 bis 80%, 0 bis 70%, 0 bis 60%, 0 bis 50%, 0 bis 40%, 0 bis 30%, 0 bis 20%, oder 0 bis 10% der Alkylsulfosuccinatmonoestern der allgemeinen Formel (I) enthalten. Die erfindungsgemäßen Mischungen können beispielsweise 10 bis 100%, 20 bis 100%, 30 bis 100%, 40 bis 100%, 50 bis 100%, 60 bis 100%, 70 bis 100%, 80 bis 100% oder 90 bis 100% der Alkylsulfosuccinatmonoestern der allgemeinen Formel (I) enthalten. Die erfindungsgemäßen Mischungen können beispielsweise 0 bis 90%, 0 bis 80%, 0 bis 70%, 0 bis 60%, 0 bis 50%, 0 bis 40%, 0 bis 30%, 0 bis 20%, oder 0 bis 10% der Alkylsulfosuccinatmonoestern der allgemeinen Formel (II) enthalten. Die erfindungsgemäßen Mischungen können beispielsweise 10 bis 100%, 20 bis 100%, 30 bis 100%, 40 bis 100%, 50 bis 100%, 60 bis 100%, 70 bis 100%, 80 bis 100% oder 90 bis 100% der Alkylsulfosuccinatmonoestern der allgemeinen Formel (II) enthalten.

Diese erfindungsgemäßen Mischungen können die Alkylsulfosuccinatmonoester der Formel (I) und (II) in beliebigen Anteilen enthalten. In einer bevorzugten Ausführungsform der Erfindung enthalten die erfindungsgemäßen Mischungen die Alkylsulfosuccinatmonoester der Formel (I) und die Alkylsulfosuccinatmonoester der Formel (II) im Gewichtsverhältnis [10 - 1] : 1, insbesondere [8 - 1] : 1, vorzugsweise [6 - 1,5] : 1, insbesondere [4 - 2] : 1, besonders bevorzugt [3,5 - 2,5] : 1, enthalten. In einer Ausführungsform der Erfindung enthalten die Mischungen die Alkylsulfosuccinate der Formel (I) und die Alkylsulfosuccinate der Formel (II) in einem Gewichtsverhältnis von 3 : 1.

Diese erfindungsgemäßen Mischungen können die Alkylsulfosuccinatmonoester der Formel (I) und (II) in beliebigen Anteilen enthalten. In einer weiteren Ausführungsform der Erfindung enthalten die erfindungsgemäßen Mischungen die Alkylsulfosuccinatmonoester der Formel **(II)** und die Alkylsulfosuccinatmonoester der Formel **(I)** im Gewichtsverhältnis [10 - 1] : 1, insbesondere [8 - 1] : 1, vorzugsweise [6 - 1,5] : 1, insbesondere [4 - 2] : 1, besonders bevorzugt [3,5 - 2,5] : 1, enthalten.

Entsprechend umfasst die vorliegende Erfindung als eine Ausführungsform Mischungen von Alkylsulfosuccinatmonoestern der allgemeinen Formel (I) 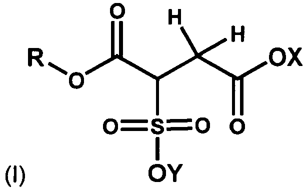
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I).

Entsprechend umfasst die vorliegende Erfindung als eine Ausführungsform Mischungen von Alkylsulfosuccinatmonoestern der allgemeinen Formel (II) 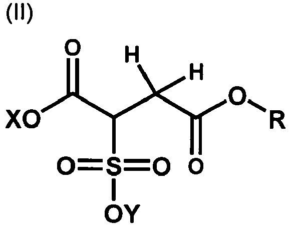
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (II).

In einer bevorzugten Ausführungsform der Erfindung sind X und Y unabhängig voneinander ausgewählt aus der Gruppe bestehend aus Na, K, Mg, Ca und NH₄. Besonders bevorzugt sind Mischungen, in denen X und Y identisch sind, vorzugsweise sind X und Y Na.

R steht für einen linearen oder verzweigten, gesättigten oder ungesättigten Alkylrest mit 6 bis 22, vorzugsweise 8 bis 18 C-Atomen. Exemplarisch seien genannt n-Octyl, i-Octyl, n- Nonyl-, i-Nonyl, n-Decyl, i-Decyl, n-Undecyl, i-Undecyl, n-Dodecyl (Trivialname Lauryl), i-Dodecyl, n-Tridecyl-, i-Tridecyl, n-Tetradecyl-, i-Tetradecyl, n-Pentadecyl, i-Pentadecyl, n-Hexadecyl (Trivialname Cetyl-), i-Hexadecyl, n-Heptadecyl, i-Heptadecyl, n-Octadecyl (Trivialname Stearyl-), i-Octadecyl und n-Octadecenyl (Oleyl-).

In einer bevorzugten Ausführungsform der Erfindung enthält die erfindungsgemäße Mischung weniger als 15, insbesondere weniger als 10, vorzugsweise weniger als 5 Gew.-% Alkylsulfosuccinatmonoester der allgemeinen Formel (I) und (II), in denen R einen verzweigten Alkylrest darstellt, bezogen auf die Gesamtmenge der Alkylsulfosuccinatmonoester.

In einer bevorzugten Ausführungsform der Erfindung enthält die erfindungsgemäße Mischung weniger als 15, insbesondere weniger als 10, vorzugsweise weniger als 5 Gew.-% - Alkylsulfosuccinatmonoester der allgemeinen Formel (I) und (II) in denen R einen ungesättigten Alkylrest darstellt, bezogen auf die Gesamtmenge der Alkylsulfosuccinatmonoester.

Die erfindungsgemäße Mischung enthält 30 bis 70, vorzugsweise 40 bis 60 Gew.-%, insbesondere 45 bis 55 Gew.-% C16-Alkylsulfosuccinatmonoester. Der Begriff C16-Alkylsulfosuccinatmonoester bezeichnet Verbindungen der allgemeinen Formel (I) und/oder (II) in der R einen Alkylrest mit 16 C Atomen darstellt. Vorzugsweise enthält die Mischung Verbindungen der allgemeinen Formel (I) und/oder (II), in der R einen linearen und gesättigten Alkylrest mit 16 C Atomen darstellt (= Hexadecylrest, Trivialname Cetylrest).

Die erfindungsgemäße Mischung enthält 30 bis 70 Gew.-%, vorzugsweise 40 bis 60 Gew.-%, insbesondere 45 bis 55 Gew.-% C18-Alkylsulfosuccinatmonoester, Der Begriff C18-Alkylsulfosuccinatmonoester bezeichnet Verbindungen der allgemeinen Formel (I) und/oder (II) in der R einen Alkylrest mit 18 C Atomen darstellt. Vorzugsweise enthält die Mischung Verbindungen der allgemeinen Formel (I) und/oder (II) in der R einen linearen und gesättigten Alkylrest mit 18 C Atomen darstellt (= Octadecylrest, Trivialname Stearylrest).

Die Gew.-% Angaben der C16-Alkylsulfosuccinatmonoester sowie der C18-Alkylsulfosuccinatmonoester beziehen sich auf die Gesamtmenge der in der Mischung vorhandenen Alkylsulfosuccinatmonoester der allgemeinen Formel (I) und (II)

Die erfindungsgemäßen Alkylsulfosuccinat Mischungen können herstellungsbedingt bis zu 30 Gew.-% Nebenbestandteile enthalten. Dies sind beispielsweise nicht umgesetzte Edukte aus der Veresterungsreaktion (wie Fettalkohole oder Maleinsäureanhydrid) oder Nebenprodukte, die bei der Herstellung entstehen, beispielsweise Diester. Der Begriff "Mischungen von Alklysulfosuccinatmonoestern" umfasst somit Mischungen, welche mindestens 70 Gew.-% Alkylsulfosuccinatmonoester der allgemeinen Formel (I) und (II) enthalten und 0 bis 30 Gew.-% Nebenbestandteile.

In einer bevorzugten Ausführungsform der Erfindung ist der Gehalt an Fettalkoholen kleiner gleich 10 Gew.-%, vorzugsweise kleiner gleich 5 Gew.-% - bezogen auf Gesamtmenge an Alkylsulfosuccinatmonoestern in der Mischung.

Als Fettalkohole im Sinne der Erfindung werden lineare, gesättigte oder ungesättigte primäre Alkohole (Alkan-1-ole) mit 6 bis 22 Kohlenstoffatomen verstanden.

Überraschenderweise wurde gefunden, dass sich Mischungen von Alkylsulfosuccinatmonoester mit einem Gehalt an Fettalkoholen von kleiner gleich 10 Gew.-% - bezogen auf die Alkylsulfosuccinatemonoester besonders gut sprühtrocknen lassen und es so technisch einfach möglich ist, Pulver zu erhalten.

Ein weiterer Gegenstand der Erfindung sind Mischungen von Alkylsulfosuccinatmonoestern der allgemeinen Formel (I) und/oder (II) 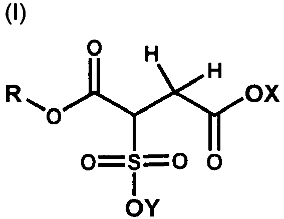 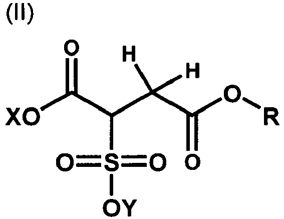
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18-**Alkylsulfosuccinatmonoester der Formel (I) und (II);
und wobei die Mischung mindestens 50 Gew.-% C16- und C18-Alkylsulfosuccinatmonoester bezogen auf die Summe der Alkylsulfosuccinatemonoester der Formel (I) und (II) enthält.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I) und (II).

Diese erfindungsgemäßen Mischungen können die Alkylsulfosuccinatmonoester der Formel (I) und (II) in beliebigen Anteilen enthalten. Die erfindungsgemäßen Mischungen können beispielsweise 0 bis 90%, 0 bis 80%, 0 bis 70%, 0 bis 60%, 0 bis 50%, 0 bis 40%, 0 bis 30%, 0 bis 20%, oder 0 bis 10% der Alkylsulfosuccinatmonoestern der allgemeinen Formel (I) enthalten. Die erfindungsgemäßen Mischungen können beispielsweise 10 bis 100%, 20 bis 100%, 30 bis 100%, 40 bis 100%, 50 bis 100%, 60 bis 100%, 70 bis 100%, 80 bis 100% oder 90 bis 100% der Alkylsulfosuccinatmonoestern der allgemeinen Formel (I) enthalten. Die erfindungsgemäßen Mischungen können beispielsweise 0 bis 90%, 0 bis 80%, 0 bis 70%, 0 bis 60%, 0 bis 50%, 0 bis 40%, 0 bis 30%, 0 bis 20%, oder 0 bis 10% der Alkylsulfosuccinatmonoestern der allgemeinen Formel (II) enthalten. Die erfindungsgemäßen Mischungen können beispielsweise 10 bis 100%, 20 bis 100%, 30 bis 100%, 40 bis 100%, 50 bis 100%, 60 bis 100%, 70 bis 100%, 80 bis 100% oder 90 bis 100% der Alkylsulfosuccinatmonoestern der allgemeinen Formel (II) enthalten.

Diese erfindungsgemäßen Mischungen können die Alkylsulfosuccinatmonoester der Formel (I) und (II) in beliebigen Anteilen enthalten. In einer bevorzugten Ausführungsform der Erfindung enthalten die erfindungsgemäßen Mischungen die Alkylsulfosuccinatmonoester der Formel (I) und die Alkylsulfosuccinatmonoester der Formel (II) im Gewichtsverhältnis [10 - 1] : 1, insbesondere [8 - 1] : 1, vorzugsweise [6 - 1,5] : 1, insbesondere [4 - 2] : 1, besonders bevorzugt [3,5 - 2,5] : 1, enthalten. In einer Ausführungsform der Erfindung enthalten die Mischungen die Alkylsulfosuccinate der Formel (I) und die Alkylsulfosuccinate der Formel (II) in einem Gewichtsverhältnis von 3 : 1.

Diese erfindungsgemäßen Mischungen können die Alkylsulfosuccinatmonoester der Formel (I) und (II) in beliebigen Anteilen enthalten. In einer weiteren Ausführungsform der Erfindung enthalten die erfindungsgemäßen Mischungen die Alkylsulfosuccinatmonoester der Formel **(II)** und die Alkylsulfosuccinatmonoester der Formel **(I)** im Gewichtsverhältnis [10 - 1] : 1, insbesondere [8 - 1] : 1, vorzugsweise [6 - 1,5] : 1, insbesondere [4 - 2] : 1, besonders bevorzugt [3,5 - 2,5] : 1, enthalten.

Entsprechend umfasst die vorliegende Erfindung als eine Ausführungsform Mischungen von Alkylsulfosuccinatmonoestern der allgemeinen Formel (I) 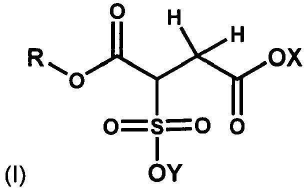
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18**-Alkylsulfosuccinatmonoester der Formel (I),
und wobei die Mischung mindestens 50 Gew.-% C16- und C18-Alkylsulfosuccinatmonoester bezogen auf die Summe der Alkylsulfosuccinatemonoester der Formel (I) enthält.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I).

Entsprechend umfasst die vorliegende Erfindung als eine Ausführungsform Mischungen von Alkylsulfosuccinatmonoestern der allgemeinen Formel (II) 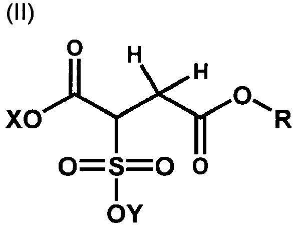
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18**-Alkylsulfosuccinatmonoester der Formel (II),
und wobei die Mischung mindestens 50 Gew.-% C16- und C18-Alkylsulfosuccinatmonoester bezogen auf die Summe der Alkylsulfosuccinatemonoester der Formel (II) enthält.

In einer bevorzugten Ausführungsform der Erfindung enthält diese Mischung mindestens 60 Gew.-%, vorzugsweise mindestens 70 Gew.-%, insbesondere mindestens 80 Gew.-% C16- und C18-Alkylsulfosuccinatmonoester bezogen auf die Summe der Alkylsulfosuccinatemonoester der Formel (II).

In einer bevorzugten Ausführungsform der Erfindung sind X und Y unabhängig voneinander ausgewählt aus der Gruppe bestehend aus Na, K, Mg, Ca und NH₄. Besonders bevorzugt sind Mischungen, in denen X und Y identisch sind, vorzugsweise sind X und Y Na.

R steht für einen linearen oder verzweigten, gesättigten oder ungesättigten Alkylrest mit 6 bis 22, vorzugsweise 8 bis 18 C-Atomen. Exemplarisch seien genannt n-Octyl, i-Octyl, n- Nonyl-, i-Nonyl, n-Decyl, i-Decyl, n-Undecyl, i-Undecyl, n-Dodecyl (Trivialname Lauryl), i-Dodecyl, n-Tridecyl-, i-Tridecyl, n-Tetradecyl-, i-Tetradecyl, n-Pentadecyl, i-Pentadecyl, n-Hexadecyl (Trivialname Cetyl-), i-Hexadecyl, n-Heptadecyl, i-Heptadecyl, n-Octadecyl (Trivialname Stearyl-), i-Octadecyl und n-Octadecenyl (Oleyl-).

**In dieser Ausführungsform** enthält die erfindungsgemäße Mischung 30 bis 70, vorzugsweise 40 bis 60 Gew.-%, insbesondere 45 bis 55 Gew.-% C16-Alkylsulfosuccinatmonoester **bezogen auf die Gesamtmenge der in der Mischung vorhandenen C16 und C18-Alkylsulfosuccinatmonoester.** Der Begriff C16-Alkylsulfosuccinatmonoester bezeichnet Verbindungen der allgemeinen Formel (I) und/oder (II) in der R einen Alkylrest mit 16 C Atomen darstellt. Vorzugsweise enthält die Mischung Verbindungen der allgemeinen Formel (I) und/oder (II), in der R einen linearen und gesättigten Alkylrest mit 16 C Atomen darstellt (= Hexadecylrest, Trivialname Cetylrest). **In dieser Ausführungsform** enthält die erfindungsgemäße Mischung 30 bis 70 Gew.-%, vorzugsweise 40 bis 60 Gew.-%, insbesondere 45 bis 55 Gew.-% C18-Alkylsulfosuccinatmonoester **bezogen auf die Gesamtmenge der in der Mischung vorhandenen C16 und C18-Alkylsulfosuccinatmonoester.** Der Begriff C18-Alkylsulfosuccinatmonoester bezeichnet Verbindungen der allgemeinen Formel (I) und/oder (II) in der R einen Alkylrest mit 18 C Atomen darstellt. Vorzugsweise enthält die Mischung Verbindungen der allgemeinen Formel (I) und/oder (II) in der R einen linearen und gesättigten Alkylrest mit 18 C Atomen darstellt (= Octadecylrest, Trivialname Stearylrest).

Die erfindungsgemäßen Alkylsulfosuccinat Mischungen können herstellungsbedingt bis zu 30 Gew.-% Nebenbestandteile enthalten. Dies sind beispielsweise nicht umgesetzte Edukte aus der Veresterungsreaktion (wie Fettalkohole oder Maleinsäureanhydrid) oder Nebenprodukte, die bei der Herstellung entstehen, beispielsweise Diester. Der Begriff "Mischungen von Alklysulfosuccinatmonoestern" umfasst somit Mischungen, welche mindestens 70 Gew.-% Alkylsulfosuccinatmonoester der allgemeinen Formel (I) und (II) enthalten und 0 bis 30 Gew.-% Nebenbestandteile.

In einer bevorzugten Ausführungsform der Erfindung ist der Gehalt an Fettalkoholen kleiner gleich 10 Gew.-%, vorzugsweise kleiner gleich 5 Gew.-% - bezogen auf Gesamtmenge an Alkylsulfosuccinatmonoestern in der Mischung.

Als Fettalkohole im Sinne der Erfindung werden lineare, gesättigte oder ungesättigte primäre Alkohole (Alkan-1-ole) mit 6 bis 22 Kohlenstoffatomen verstanden.

Überraschenderweise wurde gefunden, dass sich Mischungen von Alkylsulfosuccinatmonoester mit einem Gehalt an Fettalkoholen von kleiner gleich 10 Gew.-% - bezogen auf die Alkylsulfosuccinatemonoester besonders gut sprühtrocknen lassen und es so technisch einfach möglich ist, Pulver zu erhalten.

### Herstellung

Die erfindungsgemäßen Alkylsulfosuccinat Mischungen können beispielsweise durch Mischen von C16-Alkylsulfosuccinatmonoestern und C18-Alkylsulfosuccinatmonoestern in den beanspruchten Mengenverhältnissen erhalten werden. Die einzelnen Alkylsulfosuccinatmonoester sind beispielsweise erhältlich durch Reaktion von Maleinsäureanhydrid mit dem entsprechenden C16 bzw. C18 Fettalkohol nach dem Fachmann bekannten Methoden. Sie können aber beispielsweise auch durch Umsetzung von Maleinsäureanhydrid mit entsprechenden Fettalkoholmischungen erhalten werden. Als Fettalkohol Mischung eignet sich beispielsweise das C16/C18 Fettalkoholgemisch, welches unter dem Handelsnamen Lanette®O der Fa. Cognis GmbH erhältlich ist (technischer Name Cetearylalkohol). Die Abtrennung von freiem Fettalkohol aus dem Reaktionsgemisch kann durch dem Fachmann bekannte Methoden erfolgen, wie beispielsweise durch Destillation.

### Verwendung

Überraschenderweise wurde gefunden, dass mit den erfindungsgemäßen Alkylsulfosuccinat Mischungen stabile kosmetische und/oder pharmazeutische Zubereitungen erhalten werden. Die erfindungsgemäßen Alkylsulfosuccinat Mischungen haben sich insbesondere als vorteilhafte Emulgatoren erwiesen. Die erfindungsgemäßen Alkylsulfosuccinate Mischungen haben sich weiterhin als vorteilhaft für die Stabilisierung oder Dispergierung von festen Inhalts- oder Wirkstoffen in kosmetischen und/oder pharmazeutischen Zubereitungen erwiesen. Überraschenderweise werden auch Zubereitungen mit einem hohen Gehalt an Elektrolyten stabil erhalten. Weiterhin ermöglichen es die erfindungsgemäßen Alkylsulfosuccinat Mischungen Pigmente oder UV-Filter stabil in kosmetische und/oder pharmazeutische Zubereitungen einzuarbeiten bzw. zu emulgieren. Weiterhin zeigen die erfindungsgemäßen Alkylsulfosuccinat Mischungen eine überraschende Stabilität in alkalischen Formulierungen. Weiterhin ermöglichen sie es, kosmetische und/oder pharmazeutische Zubereitungen auch bei niedrigem pH-Wert stabil zu erhalten. Weiterhin ermöglichen die erfindungsgemäßen Alkylsulfosuccinat Mischungen die Herstellung von stabilen Emulsionen. Die erfindungsgemäßen Alkylsulfosuccinat Mischungen eignen sich daher als Bestandteile von kosmetischen und/oder pharmazeutischen Zubereitungen.

Ein Gegenstand der Erfindung betrifft die Verwendung einer Mischung von Alkylsulfosuccinatmonoestern der allgemeinen Formel (I) und/oder (II) 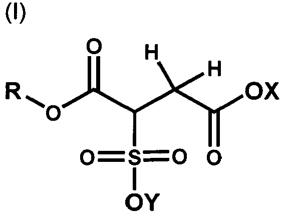 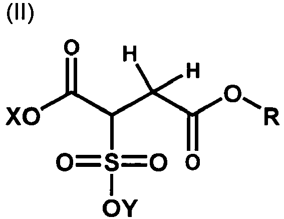
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II)
in kosmetischen und/oder pharmazeutischen Zubereitungen.

Diese erfindungsgemäßen Mischungen können die Alkylsulfosuccinatmonoester der Formel (I) und (II) in beliebigen Anteilen enthalten. Die erfindungsgemäßen Mischungen können beispielsweise 0 bis 90%, 0 bis 80%, 0 bis 70%, 0 bis 60%, 0 bis 50%, 0 bis 40%, 0 bis 30%, 0 bis 20%, oder 0 bis 10% der Alkylsulfosuccinatmonoestern der allgemeinen Formel (I) enthalten. Die erfindungsgemäßen Mischungen können beispielsweise 10 bis 100%, 20 bis 100%, 30 bis 100%, 40 bis 100%, 50 bis 100%, 60 bis 100%, 70 bis 100%, 80 bis 100% oder 90 bis 100% der Alkylsulfosuccinatmonoestern der allgemeinen Formel **(I)** enthalten. Die erfindungsgemäßen Mischungen können beispielsweise 0 bis 90%, 0 bis 80%, 0 bis 70%, 0 bis 60%, 0 bis 50%, 0 bis 40%, 0 bis 30%, 0 bis 20%, oder 0 bis 10% der Alkylsulfosuccinatmonoestern der allgemeinen Formel (II) enthalten. Die erfindungsgemäßen Mischungen können beispielsweise 10 bis 100%, 20 bis 100%, 30 bis 100%, 40 bis 100%, 50 bis 100%, 60 bis 100%, 70 bis 100%, 80 bis 100% oder 90 bis 100% der Alkylsulfosuccinatmonoestern der allgemeinen Formel (II) enthalten.

Diese erfindungsgemäßen Mischungen können die Alkylsulfosuccinatmonoester der Formel (I) und (II) in beliebigen Anteilen enthalten. In einer bevorzugten Ausführungsform der Erfindung enthalten die erfindungsgemäßen Mischungen die Alkylsulfosuccinatmonoester der Formel (I) und die Alkylsulfosuccinatmonoester der Formel (II) im Gewichtsverhältnis [10 - 1] : 1, insbesondere [8 - 1] : 1, vorzugsweise [6 - 1,5] : 1, insbesondere [4 - 2] : 1, besonders bevorzugt [3,5 - 2,5] : 1, enthalten. In einer Ausführungsform der Erfindung enthalten die Mischungen die Alkylsulfosuccinate der Formel (I) und die Alkylsulfosuccinate der Formel (II) in einem Gewichtsverhältnis von 3 : 1.

Diese erfindungsgemäßen Mischungen können die Alkylsulfosuccinatmonoester der Formel (I) und (II) in beliebigen Anteilen enthalten. In einer weiteren Ausführungsform der Erfindung enthalten die erfindungsgemäßen Mischungen die Alkylsulfosuccinatmonoester der Formel **(II)** und die Alkylsulfosuccinatmonoester der Formel **(I)** im Gewichtsverhältnis [10 - 1] : 1, insbesondere [8 - 1] : 1, vorzugsweise [6 - 1,5] : 1, insbesondere [4 - 2] : 1, besonders bevorzugt [3,5 - 2,5] : 1, enthalten.

Entsprechend umfasst die vorliegende Erfindung als eine Ausführungsform die Verwendung einer Mischung von Alkylsulfosuccinatmonoestern der allgemeinen Formel (I) 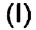 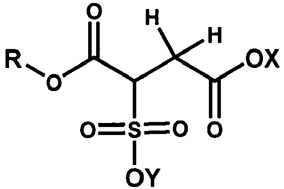
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I)
in kosmetischen und/oder pharmazeutischen Zubereitungen.

Entsprechend umfasst die vorliegende Erfindung als eine Ausführungsform die Verwendung einer Mischung von Alkylsulfosuccinatmonoestern der allgemeinen Formel (II) 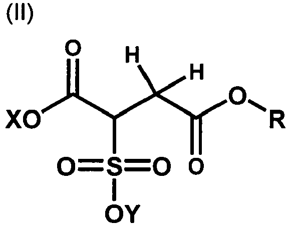
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (II)
in kosmetischen und/oder pharmazeutischen Zubereitungen.

Die erfindungsgemäßen Alkylsulfosuccinat Mischungen eignen sich insbesondere als grenzflächenaktive Substanz, wie z.B. als Emulgatoren und/oder Dispergatoren.

Ein weiterer Gegenstand der Erfindung betrifft kosmetische und/oder pharmazeutische Zubereitung enthalten 0,1 bis 50, vorzugsweise 0,5 bis 10 Gew.-% - bezogen auf die Zubereitung- einer Mischung von Alkylsulfosuccinatmonoestern der allgemeinen Formel (I) und/oder (II) 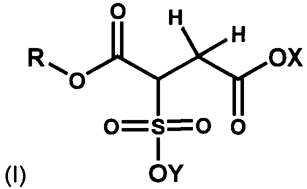 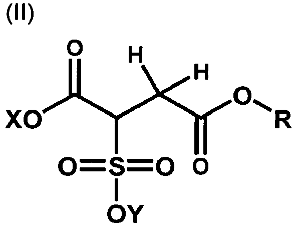
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II).

Ein besonders bevorzugter Gegenstand der Erfindung betrifft kosmetische und/oder pharmazeutische Zubereitung enthalten 0,1 bis 50, vorzugsweise 0,5 bis 10 Gew.-% - bezogen auf die Zubereitung- einer Mischung von Alkylsulfosuccinatmonoestern der allgemeinen Formel (I) und/oder (II) 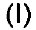 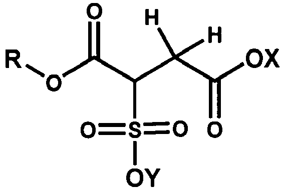 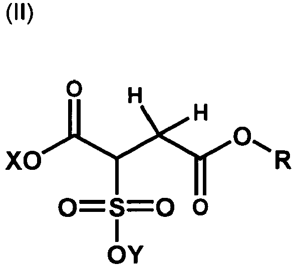
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II)
dadurch gekennzeichnet, dass die Zubereitung weniger als 10 Gew.-%, insbesondere weniger als 5 Gew.-% Fettalkohol - bezogen auf die Alkylsulfosuccinatmonoester der allgemeinen Formel (I) und (II) enthält.

Ein weiterer Gegenstand der Erfindung betrifft die Verwendung einer Mischung von Alkylsulfosuccinatmonoestern der allgemeinen Formel (I) und/oder (II) 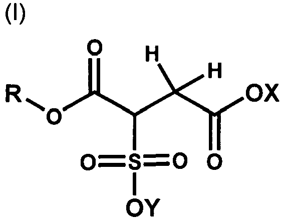 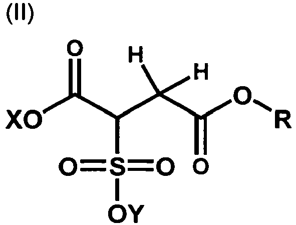
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18-**Alkylsulfosuccinatmonoester **C16- und C18-**Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinate-monoester der Formel (I) und (II) enthält,
in kosmetischen und/oder pharmazeutischen Zubereitungen.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I) und (II).

Diese erfindungsgemäßen Mischungen können die Alkylsulfosuccinatmonoester der Formel (I) und (II) in beliebigen Anteilen enthalten. Die erfindungsgemäßen Mischungen können beispielsweise 0 bis 90%, 0 bis 80%, 0 bis 70%, 0 bis 60%, 0 bis 50%, 0 bis 40%, 0 bis 30%, 0 bis 20%, oder 0 bis 10% der Alkylsulfosuccinatmonoestern der allgemeinen Formel (I) enthalten. Die erfindungsgemäßen Mischungen können beispielsweise 10 bis 100%, 20 bis 100%, 30 bis 100%, 40 bis 100%, 50 bis 100%, 60 bis 100%, 70 bis 100%, 80 bis 100% oder 90 bis 100% der Alkylsulfosuccinatmonoestern der allgemeinen Formel (I) enthalten. Die erfindungsgemäßen Mischungen können beispielsweise 0 bis 90%, 0 bis 80%, 0 bis 70%, 0 bis 60%, 0 bis 50%, 0 bis 40%, 0 bis 30%, 0 bis 20%, oder 0 bis 10% der Alkylsulfosuccinatmonoestern der allgemeinen Formel (II) enthalten. Die erfindungsgemäßen Mischungen können beispielsweise 10 bis 100%, 20 bis 100%, 30 bis 100%, 40 bis 100%, 50 bis 100%, 60 bis 100%, 70 bis 100%, 80 bis 100% oder 90 bis 100% der Alkylsulfosuccinatmonoestern der allgemeinen Formel (II) enthalten.

Diese erfindungsgemäßen Mischungen können die Alkylsulfosuccinatmonoester der Formel (I) und (II) in beliebigen Anteilen enthalten. In einer bevorzugten Ausführungsform der Erfindung enthalten die erfindungsgemäßen Mischungen die Alkylsulfosuccinatmonoester der Formel (I) und die Alkylsulfosuccinatmonoester der Formel (II) im Gewichtsverhältnis [10 - 1] : 1, insbesondere [8 - 1] : 1, vorzugsweise [6 - 1,5] : 1, insbesondere [4 - 2] : 1, besonders bevorzugt [3,5 - 2,5] : 1, enthalten. In einer Ausführungsform der Erfindung enthalten die Mischungen die Alkylsulfosuccinate der Formel (I) und die Alkylsulfosuccinate der Formel (II) in einem Gewichtsverhältnis von 3 : 1.

Diese erfindungsgemäßen Mischungen können die Alkylsulfosuccinatmonoester der Formel (I) und (II) in beliebigen Anteilen enthalten. In einer weiteren Ausführungsform der Erfindung enthalten die erfindungsgemäßen Mischungen die Alkylsulfosuccinatmonoester der Formel **(II)** und die Alkylsulfosuccinatmonoester der Formel **(I)** im Gewichtsverhältnis [10 - 1] : 1, insbesondere [8 - 1] : 1, vorzugsweise [6 - 1,5] : 1, insbesondere [4 - 2] : 1, besonders bevorzugt [3,5 - 2,5] : 1, enthalten.

Entsprechend umfasst die vorliegende Erfindung als eine Ausführungsform die Verwendung einer Mischung von Alkylsulfosuccinatmonoestern der allgemeinen Formel (I) 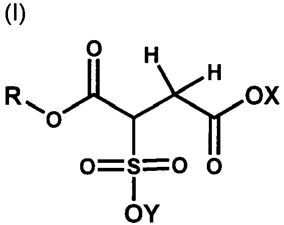
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die auf die Gesamtmenge der **in der Mischung vorhandenen C16- und C18-**Alkylsulfosuccinatmonoester der Formel (I)
und wobei die Mischung mindestens 50 Gew.-% C16- und C18-Alkylsulfosuccinatmonoester bezogen auf die Summe der Alkylsulfosuccinatemonoester der Formel (I) enthält.
in kosmetischen und/oder pharmazeutischen Zubereitungen.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I).

Entsprechend umfasst die vorliegende Erfindung als eine Ausführungsform die Verwendung einer Mischung von Alkylsulfosuccinatmonoestern der allgemeinen Formel (II) 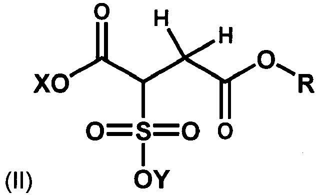
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18**-Alkylsulfosuccinatmonoester der Formel (II),
und wobei die Mischung mindestens 50 Gew.-% C16- und C18-Alkylsulfosuccinatmonoester bezogen auf die Summe der Alkylsulfosuccinatemonoester der Formel (II) enthält;
in kosmetischen und/oder pharmazeutischen Zubereitungen.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (II).

Die erfindungsgemäßen Alkylsulfosuccinat Mischungen eignen sich insbesondere als grenzflächenaktive Substanz, wie z.B. als Emulgatoren und/oder Dispergatoren.

Ein weiterer Gegenstand der Erfindung betrifft kosmetische und/oder pharmazeutische Zubereitung enthalten 0,1 bis 50, vorzugsweise 0,5 bis 10 Gew.-% - bezogen auf die Zubereitung- einer Mischung von Alkylsulfosuccinatmonoestern der allgemeinen Formel (I) und/oder (II) 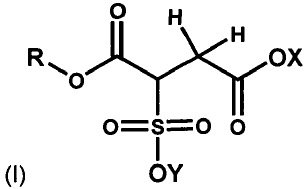 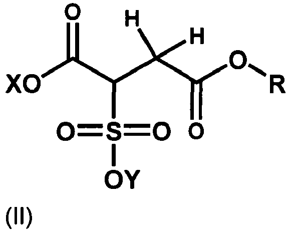
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18-**Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinatemonoester der Formel (I) und (II) enthält.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I) und (II).

Ein besonders bevorzugter Gegenstand der Erfindung betrifft kosmetische und/oder pharmazeutische Zubereitung enthalten 0,1 bis 50, vorzugsweise 0,5 bis 10 Gew.-% - bezogen auf die Zubereitung- einer Mischung von Alkylsulfosuccinatmonoestern der allgemeinen Formel (I) und/oder (II) 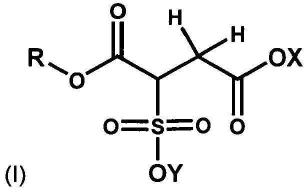 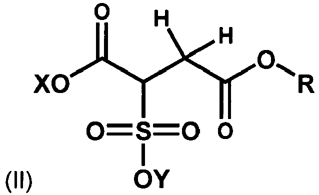
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18**-Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinatemonoester der Formel (I) und (II) enthält;
dadurch gekennzeichnet, dass die Zubereitung weniger als 10 Gew.-%, insbesondere weniger als 5 Gew.-% Fettalkohol - bezogen auf die Alkylsulfosuccinatmonoester der allgemeinen Formel (I) und (II) enthält.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I) und (II).

Die kosmetischen und/oder pharmazeutischen Zubereitungen können Formulierungen zur Körperpflege sein, z. B. eine Körpermilch, Cremes, Lotionen, sprühbare Emulsionen, Produkte zur Eliminierung des Körpergeruchs etc. Die erfindungsgemäßen Alkylsulfosuccinat Mischungen lassen sich auch in tensidhaltigen Formulierungen wie z. B. Schaum- und Duschbädern, Haarshampoos und Pflegespülungen einsetzen. Je nach Applikationszweck enthalten die kosmetischen und/oder pharmazeutischen Zubereitungen eine Reihe weiterer Hilfs- und Zusatzstoffe, wie beispielsweise Tenside, Ölkörper, (weitere) grenzflächenaktive Substanzen, wie Emulgatoren oder Tenside, Perlglanzwachse, Konsistenzgeber, Verdickungsmittel, Überfettungsmittel, Stabilisatoren, Polymere, Fette, Wachse, Lecithine, Phospholipide, biogene Wirkstoffe, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc.

Die erfindungsgemäßen kosmetischen und/oder pharmazeutischen Zubereitungen können beispielsweise als O/W oder W/O Pflegeemulsionen, als multiple Emulsionen, wie beispielsweise W/O/W oder O/W/O Emulsionen, Sonnenschutzformulierung, AP/Deo Konzepte, Formulierungen für die dekorative Kosmetik, ölige Pflegezubereitungen, Tränkflüssigkeiten für Substrate, wie beispielsweise Papier- und Vliessprodukte vorliegen. Exemplarisch seien genannt Wet Wipes, Taschentücher, Windeln oder Hygieneprodukte.

Die erfindungsgemäßen Alkylsulfosuccinat Mischungen sowie die erfindungsgemäßen kosmetischen und/oder pharmazeutischen Zubereitungen eignen sich insbesondere auch für leichte, sprühbare Anwendungen und/oder als Bestandteile von Pflegeemulsionen für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung. Sie lassen sich auf Tissues, Papiere, Wipes, Vlies-Produkte, Schwämme, Puffs, Pflaster und Bandagen applizieren, die ihren Einsatz im Bereich der Reinigung, Hygiene und/oder Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte, Selbstbräunungs Wipes, Toilettenpapier, Erfrischungstücher, After-shave Tücher). Sie lassen sich weiterhin in Zubereitungen zur Haarpflege, Haarreinigung oder Haarfärbung einsetzen. Die erfindungsgemäßen Alkylsulfosuccinat Mischungen eignen sich insbesondere als Bestandteile von Zubereitungen der dekorativen Kosmetik, wie beispielsweise Lippenstifte, Augen-Make up, wie beispielsweise Lidschatten, Mascara, Lidstifte, Kajal, Nagellack, etc. sowie Make-up Formulierungen.

Mit den erfindungsgemäßen Alkylsulfosuccinat Mischungen lassen sich insbesondere UV-Lichtschutzfilter stabil emulgieren bzw. dispergieren. Ein weiterer Gegenstand der Erfindung betrifft daher kosmetischen und/oder pharmazeutische Zubereitung enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 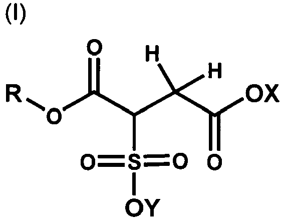 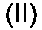 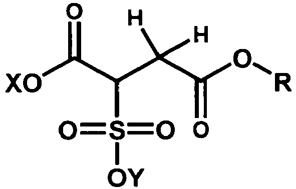
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II)
und **mindestens einen UV-Lichtschutzfilter.**

Ein weiterer Gegenstand der Erfindung betrifft kosmetischen und/oder pharmazeutische Zubereitung enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 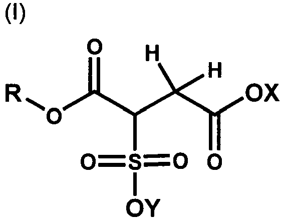 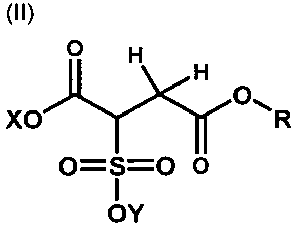
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18**-Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinatemonoester der Formel (I) und (II) enthält.
und **mindestens einen UV-Lichtschutzfilter.**

In einer bevorzugten Ausführungsform der Erfindung enthält diese Mischung mindestens 60 Gew.-%, vorzugsweise mindestens 70 Gew.-%, insbesondere mindestens 80 Gew.-% C16- und C18-Alkylsulfosuccinatmonoester bezogen auf die Summe der Alkylsulfosuccinatemonoester der Formel (I) und (II).

Erfindungsgemäß sind als UV-Lichtschutzfilter bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
➢ 3-(4'-Trimethylammonium) benzyliden- bornan-2-on-methylsulfat (Mexoryl SO)
➢ 3,3'-(1,4-Phenylendimethin)-bis (7,7- dimethyl-2-oxobicyclo-[2.2.1] heptan-1-methansulfonsäure) and salts (Mexoryl SX)
➢ 3-(4'-Sulfo)-benzyliden-bornan-2-on and salts (Mexoryl SL)
➢ Polymer von N-{(2und 4)- [2-oxoborn-3-yliden)methyl}benzyl]acrylamid (Mexoryl SW)
➢ 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl SL)
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenylamino]-1,3,5-triazin-2,4- diyl)diimino] bis(benzoesäure-2- ethylhexylester) (Uvasorb® HEB);
➢ 2,2(-Methylen-bis(6-(2H-benzotriazol-2-yl)-4-(1,1,3,3-tetramethyl-butyl)phenol) (Tinosorb M);
➢ 2,4-Bis[4-(2-ethylhexyloxy)-2-hydroxyphenyl]-6-(4-methoxyphenyl)-1,3,5-triazin (Tinosorb S);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX).

Als wasserlösliche UV - Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)-benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der **Fassung Commission Directive 2005/9/EC of 28 January 2005 amending Council Directive 76/768/EEC,** concerning cosmetic products, for the purposes of adapting Annexes VII thereof to technical progress) zugelassen Stoffe, auf die hier explizit Bezug genommen wird.

Neben den vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydro-guajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Ein Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 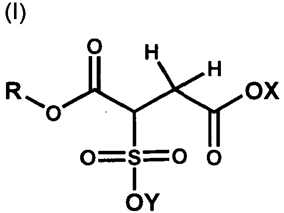 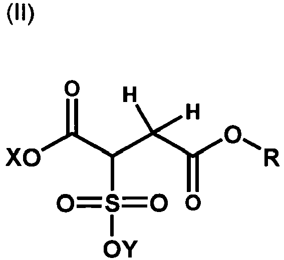
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II)
und mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden-bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzyliden-bornan-2-on und ihre Salze, Polymer von N-{(2und 4)- [2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy) disiloxanyl)propyl) phenol, Dimethicodiethylbenzalmalonate und ihren Mischungen.

Ein Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 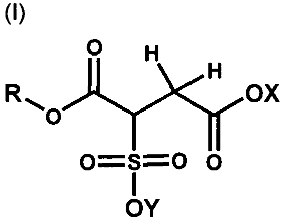 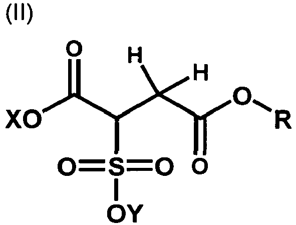
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18-** Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinatemonoester der Formel (I) und (II) enthält;
und mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden-bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzyliden-bornan-2-on und ihre Salze, Polymer von N-{(2und 4)- [2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy) disiloxanyl)propyl) phenol, Dimethicodiethylbenzalmalonate und ihren Mischungen.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I) und (II).

Diese UV-Lichtschutzfilter sind beispielsweise unter den folgenden Handelsnamen kommerziell erhältlich:
NeoHeliopan®MBC (INCI: 4-Methylbenzylidene Camphor; Hersteller: Symrise); NeoHeliopan® BB (INCI: Benzophenone-3, Hersteller: Symrise); Parsol®1789 (INCI: Butyl Methoxydibenzoylmethane, Hersteller: Hoffmann-La Roche (Givaudan); Tinosorb®S (INCI: Bis-Ethylhexyloxyphenol Methoxyphenyl Triazine); Tinosorb®M (INCI: Methylene Bis-Benzotriazolyl Tetramethylbutylphenol): Herstelller: Ciba Specialty Chemicals Corporation; Uvasorb®HEB (INCI: Diethylhexyl Butamido Triazone, Hersteller: 3V Inc.), Uvinul®T 150 (INCI: Ethylhexyl Triazone, Hersteller: BASF AG); Uvinul® A plus (INCI: Diethylamino Hydroxybenzoyl Hexyl Benzoate: Hersteller: BASF AG; Mexoryl® SO: 3-(4'-Trimethylammonium) benzyliden- bornan-2-on-methylsulfat, INCI: Camphor Benzalkonium Methosulfate; Mexoryl®SX: 3,3'-(1,4-Phenylendimethin)-bis (7,7-dimethyl-2-oxobicyclo-[2.2.1] heptan-1-methansulfonsäure), CTFA: INCI Terephthalylidene Dicamphor Sulfonic Acid; Mexory® SL: 3-(4'-Sulfo)-benzyliden-bornan-2-on, INCI Benzylidene Camphor Sulfonic Acid; Mexoryl®SW: Polymer von N-{(2und 4)-[2-oxoborn-3-yliden)methyl}benzyl]acrylamid, INCI Polyacrylamidomethyl Benzylidene Camphor; Mexoryl®SL: 2-(2H-Benzotriazol-2-yl)-4-methyl-6-(2-methyl-3-(1,3,3,3-tetramethyl-1-(trimethylsilyloxy) disiloxanyl)propyl) phenol; INCI: Benzylidene camphor sulfonic acid; Parsol® SLX: Dimethicodiethylbenzalmalonate, INCI Polysilicone-15, Mexoryl®XL: 2-(2H-Benzotriazol-2-yl)-4-methyl-6-(2-methyl-3-(1,3,3,3-tetramethyl-1-(trimethylsilyloxy) disiloxanyl)propyl) phenol INCI: Drometrizole trisiloxane.

Ein Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 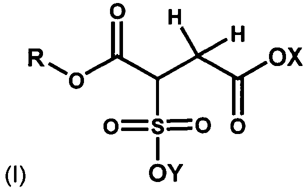 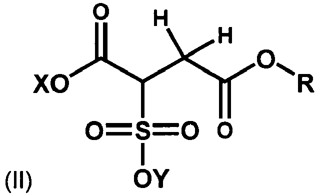
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält wobei die Gew.-% bezogen sind auf die Gesamtmenge der Alkylsulfosuccinatmonoester der Formel (I) und (II)
und mindestens einen UV-Lichtschutzfilter ausgewählt aus der Gruppe der Lichtschutzpigmente, insbesondere aus der Gruppe der Zinkoxide und Titanoxide.

Ein Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 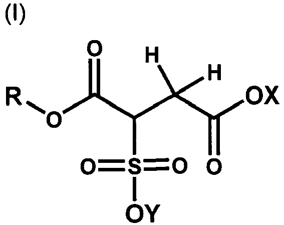 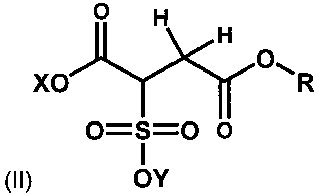
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18**-Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinatemonoester der Formel (I) und (II) enthält;
und mindestens einen UV-Lichtschutzfilter ausgewählt aus der Gruppe der Lichtschutzpigmente, insbesondere aus der Gruppe der Zinkoxide und Titanoxide.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I) und (II).

Überraschenderweise wurde gefunden, dass die erfindungsgemäßen Alkylsulfosuccinat Mischungen UV-Lichtschutzfilter, insbesondere Lichtschutzpigmente gut dispergieren bzw. stabilisieren und so eine gleichmäßige Verteilung des UV-Lichtschutzfilters in der kosmetischen und/pharmazeutischen Zubereitung gewährleisten.

Als Lichtschutzpigmente kommen in Frage feindisperse Metalloxide bzw. Salze. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 8/1996, S. 543-548 sowie Parf.Kosm. 80. Jahrgang, Nr. 3/1999, S. 10 bis 16 zu entnehmen.

Die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen auf das Gesamtgewicht der kosmetischen und/oder pharmazeutischen Zubereitung - enthalten.

Ein Gegenstand der Erfindung betrifft kosmetische Zubereitungen zum Färben von Haut und Haar, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 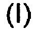 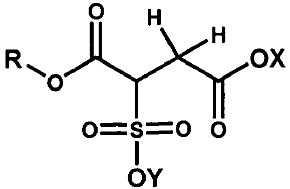 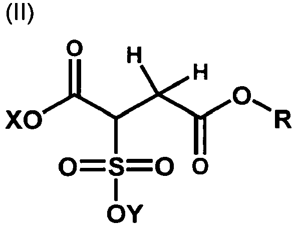
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II),
und mindestens einen direktziehenden Farbstoff oder ein Oxidationsfarbstoffvorprodukt.

Ein weiterer Gegenstand der Erfindung betrifft die Verwendung dieser kosmetischen Zubereitung zur Haarfärbung sowie ein Verfahren zur Haarfärbung oder der Auffrischung der Haarfärbung mit dieser Zubereitung.
Ein weiterer Gegenstand der Erfindung betrifft kosmetische Zubereitungen zum Färben von Haut und Haar, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 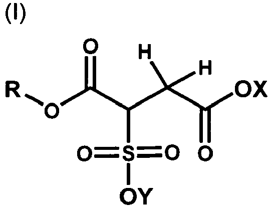 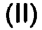 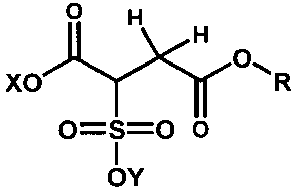
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18**-Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinatemonoester der Formel (I) und (II) enthält;
und mindestens einen direktziehenden Farbstoff oder ein Oxidationsfarbstoffvorprodukt.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I) und (II).

Ein weiterer Gegenstand der Erfindung betrifft die Verwendung dieser kosmetischen Zubereitung zur Haarfärbung sowie ein Verfahren zur Haarfärbung oder der Auffrischung der Haarfärbung mit dieser Zubereitung.

Für das Färben von Haaren kommen im Allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet. Als Entwicklerkomponenten werden üblicherweise primäre aromatische Amine mit einer weiteren, in para-oder ortho-Position befindlichen freien oder substituierten Hydroxy- oder Aminogruppe, Diaminopyridinderivate, heterocyclische Hydrazone, 4-Aminopyrazolonderivate sowie 2,4, 5,6-Tetraaminopyrimidin und dessen Derivate eingesetzt. Spezielle Vertreter sind beispielsweise p-Phenylendiamin, p-Toluylendiamin, 2,4, 5,6- Tetraaminopyrimidin, p-Aminophenol, N, N-Bis-(2-hydroxyethyl)-p-phenylendiamin, 2- (2, 5-Diaminophenyl)-ethanol, 2-(2, 5-Diaminophenoxy)-ethanol, 1-Phenyl-3- carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5, 6-diaminopyrimidin und 2,5, 6-Triamino-4-hydroxypyrimidin. Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere c-Naphtho" 1, 5-, 2,7-und 1,7-Dihydroxy-naphthalin, 5-Amino-2-methylphenol, m-Aminophenol, Resorcin, Resorcin- monomethylether, m-Phenylendiamin, 2, 4-diaminophenoxyethanol, 1-P henyl-3-methyl- pyrazolon-5, 2, 4-Dichlor-3-aminophenol, 1, 3-Bis- (2, 4-diaminophenoxy) -propan, 2- Chlorresorcin, 4-Chlorresorcin, 2-Chlor-6-methyl-3-aminophenol, 2-Methylresorcin und 5-Methylresorcin. Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7**,** Ch. Zviak, The Science of Hair Care, Kap. 7, Seiten 248-250 (Direktziehende Farbstoffe**), und** Kap. 8, Seiten 264-267 (Oxidationsfarbstoffe**),** sowie das **"**Europäische Inventar der Kosmetikrohstoffe", 1996, herausgegeben von der Europäischen Kommission [Hnhd-01938 (203-058-I**)],** erhältlich in Diskettenform vom Bundesverband der deutschen Industrie- und Handelsunternehmen für Arzneimittel, Reformwaren und Körperpflegemittel e. V., Mannheim, Bezug genommen.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino- 2-methyl-propanol, 2-Amino-2-methyl-1,3-propandiol, 2-Amino-2-ethyl-1,3-propandiol, 2- Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von omega-Aminosäuren wie omega-Aminocapronsäure als Alkalisierungsmittel ist möglich.

Erfolgt die Ausbildung der eigentlichen Haarfarben im Rahmen eines oxidativen Prozesses, so können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel, oder auch Enzyme verwendet werden, die Elektronen aus geeigneten Entwicklerkomponenten (Reduktionsmittel) auf Luftsauerstoff übertragen. Bevorzugt sind dabei Oxidasen wie Tyrosinase, Ascorbatoxidase und Laccase aber auch Glucoseoxidase, Uricase oder Pyruvatoxidase. Weiterhin sei das Vorgehen genannt, die Wirkung geringer Mengen (z. B. 1 % und weniger, bezogen auf die gesamte Zubereitung) Wasserstoffperoxid durch Peroxidasen zu verstärken.

Zweckmäßigerweise wird die Zubereitung des Oxidationsmittels dann unmittelbar vor dem Färben der Haare mit der Zubereitung mit dem Oxidationsfarbstoffvorprodukt vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40°C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit dem Oxidationsfarbstoffvorprodukt ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen des Oxidationsfarbstoffvorprodukts eine bessere Penetration in das Haar bewirken soll, die entsprechende Zubereitung auf einen pH-Wert von etwa 4 bis 7 eingestellt. Gemäß einer zweiten Variante wird zunächst eine Luftoxidation angestrebt, wobei die aufgebrachte Zubereitung bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Weiterhin kann die Ausbildung der Färbung dadurch unterstützt und gesteigert werden, dass dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Die direktziehenden Farbstoffe oder Oxidationsfarbstoffvorprodukte werden üblicherweise in Konzentrationen von 0,001 bis 0,1 Gew.-%, bezogen auf das Gesamtgewicht der Zubereitung, eingesetzt.

Der pH-Wert der erfindungsgemäßen Zubereitungen kann prinzipiell bei Werten von 2 - 1 1 liegen. Der pH - Wert wird je nach dem Zweck und der Verwendung der erfindungsgemäßen Zusammensetzung ganz gezielt ausgewählt und eingestellt. Für Färbemittel liegt er beispielsweise bevorzugt zwischen 5 und 11, wobei Werte von 6 bis 10 besonders bevorzugt sind. Zur Einstellung dieses pH-Wertes kann praktisch jede für kosmetische Zwecke verwendbare Säure oder Base verwendet werden. Bevorzugte Basen sind Ammoniak, Alkalihydroxide, Monoethanolamin, Triethanolamin sowie N,N,N',N'-Tetrakis-(2-hydroxypropyl)-ethylendiamin. Üblicherweise werden als Säuren Genusssäuren verwendet. Unter Genusssäuren werden solche Säuren verstanden, die im Rahmen der üblichen Nahrungsaufnahme aufgenommen werden und positive Auswirkungen auf den menschlichen Organismus haben. Genusssäuren sind beispielsweise Essigsäure, Milchsäure, Weinsäure, Zitronensäure, Äpfelsäure, Ascorbinsäure und Gluconsäure.

Ein Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 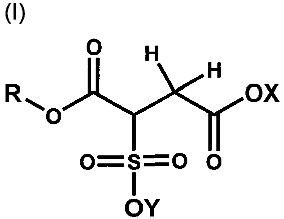 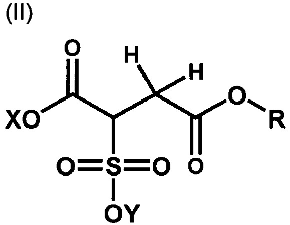
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II)
und mindestens einen Antiperspirant-/ Desodorant-Wirkstoff.

Ein weiterer Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 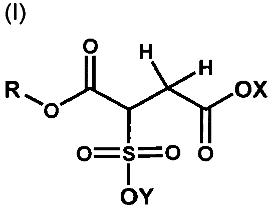 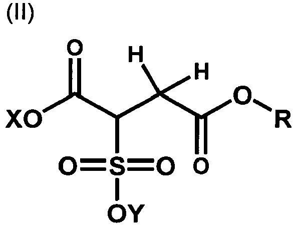
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18**-Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinatemonoester der Formel (I) und (II) enthält;
und mindestens einen Antiperspirant-/ Desodorant-Wirkstoff.

In einer bevorzugten Ausführungsform der Erfindung enthält diese Mischung mindestens 60 Gew.-%, vorzugsweise mindestens 70 Gew.-%, insbesondere mindestens 80 Gew.-% C16- und C18-Alkylsulfosuccinatmonoester bezogen auf die Summe der Alkylsulfosuccinatemonoester der Formel (I) und (II).

Erfindungsgemäß sind als Antiperspirant/Desodorant Wirkstoff alle Wirkstoffe geeignet, die Körpergerüchen entgegen wirken, diese überdecken oder beseitigen. Körpergerüche entstehen durch die Einwirkung von Hautbakterien auf apokrinen Schweiß, wobei unangenehm riechende Abbauprodukte gebildet werden. Als Antiperspirant /Desodorant Wirkstoffe eignen sich insbesondere Verbindungen ausgewählt aus der Gruppe bestehend aus Antiperspirantien, Esteraseinhibitoren, bakterizide bzw. bakteriostatische Wirkstoffe und/oder schweißabsorbierende Substanzen.

### Antiperspirantien

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkoniumtetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorohydrat und deren Komplexverbindungen eingesetzt.

Die erfindungsgemäßen Zubereitungen können die Antiperspirantien in Mengen von 1 bis 50, vorzugsweise 5 bis 30 und insbesondere 8 bis 25 Gew.-% - bezogen auf das Gesamtgewicht der kosmetischen und/oder pharmazeutischen Zubereitung - enthalten.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Triisopropylcitrat, Tributylcitrat und insbesondere Triethylcitrat (Hydagen® CAT, Cognis GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester sowie Zinkglycinat.
Die erfindungsgemäßen Zubereitungen können die Esteraseinhibitoren in Mengen von 0,01 bis 20, vorzugsweise 0,1 bis 10 und insbesondere 0,3 bis 5 Gew.-% - bezogen auf das Gesamtgewicht der kosmetischen und/oder pharmazeutischen Zubereitung - enthalten.

### Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Die erfindungsgemäßen Zubereitungen können die bakteriziden bzw. bakteriostatischen Wirkstoffe in Mengen von 0,01 bis 5 und vorzugsweise 0,1 bis 2 Gew.-% - bezogen auf das Gesamtgewicht der kosmetischen und/oder pharmazeutischen Zubereitung - enthalten.

### Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierende Substanzen in Mengen von 0,1 bis 30, vorzugsweise 1 bis 20 und insbesondere 2 bis 8 Gew.-% - bezogen auf das Gesamtgewicht der kosmetischen und/oder pharmazeutischen Zubereitung - enthalten.

Ein Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 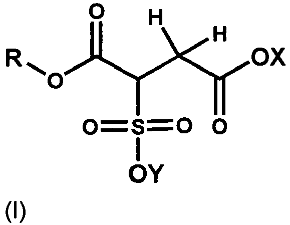 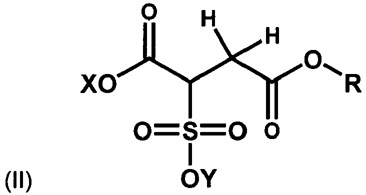
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II)
und mindestens einen Selbstbräuner.

Ein weiterer Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 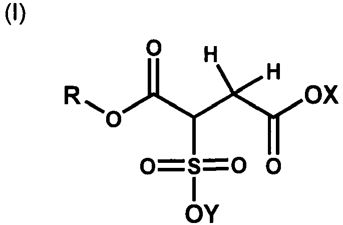 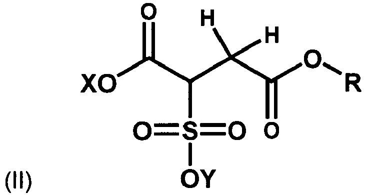
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18-**Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinatemonoester der Formel (I) und (II) enthält;
und mindestens einen Selbstbräuner.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I) und (II).

Als Selbstbräuner sind Substanzen zu verstehen, welche eine Bräunung der Haut verursachen. Beispielsweise seien alpha, beta-ungestättige Aldehyde genannt, welche mit den Aminosäuren der Haut im Sinne einer Maillard Reaktion zu gefärbten Verbindungen abreagieren. Als Wirkstoffe für Selbstbräuner kommen weiterhin in Frage natürliche oder synthetische Ketole oder Aldole. Als geeignete Wirkstoffe seien exemplarisch genannt Dihydroxyaceton, Erythrulose Glycerolaldehyd, Alloxan, Hydroxymethylglyoxal, gamma-Dialdehyd, 6-Aldo-D-Fructose, Ninhydrin und meso-Weinsäuredialdehyd. Als Selbstbräuner eignen sich insbesondere Dihydroxyaceton und/oder Erythrulose.

Als besonders vorteilhaft haben sich Mischungen der o.g. Wirkstoffe untereinander oder mit Mucondialdehyd oder/und Naphthochinone wie z. B. 5-Hydroxy-1,4-naphthochinon (Juglon) und 2-Hydroxy-1,4-Naphthochinon erwiesen.

Die erfindungsgemäßen Zusammensetzungen enthalten die Selbstbräuner üblicherweise in Konzentrationen von 1 bis 10, insbesondere von 2 bis 5 Gew.-% -bezogen auf das Gesamtgewicht der kosmetischen und/oder pharmazeutischen Zubereitung.

In einer bevorzugten Ausführungsform der Erfindung enthalten die erfindungsgemäßen kosmetischen und/oder pharmazeutischen Zubereitungen mindestens einen Selbstbräuner und mindestens einen UV-Lichtschutzfilter.
Ein Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 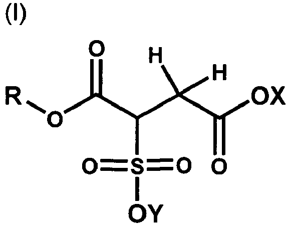 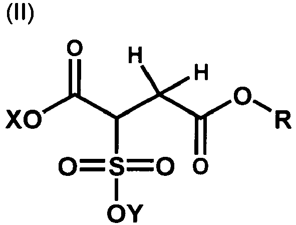
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II).
und mindestens ein Pigment und/oder einen Farbstoff.

Ein weiterer Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 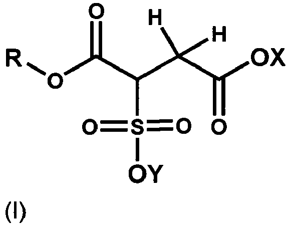 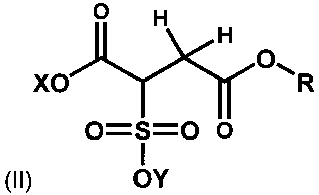
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18**-Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinatemonoester der Formel (I) und (II) enthält,
und mindestens ein Pigment und/oder einen Farbstoff.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I) und (II).

Der Begriff Pigment umfasst Partikel jeder Form, die weiß oder gefärbt, organisch oder anorganisch sind, in den Zubereitungen nicht löslich sind, und dem Zweck dienen die Zubereitung zu färben. In einer bevorzugten Ausführungsform werden anorganische Pigmente verwendet, besonders bevorzugt sind Metalloxide.

Als anorganische Pigmente seien exemplarisch genannt: Titandioxid, optional oberflächenbeschichtet, Zirkonium oder Ceriumoxide und Zink-, Eisen- (Schwarz, Gelb oder Rot) und Chrom-oxide, Manganviolett, Ultramarine Blau, Chromhydroate und Eisen(III)blau, Metallpulver wie Aluminiumpulver oder Kupferpulver.

In einer bevorzugten Ausführungsform der Erfindung ist das Pigment ausgewählt aus den anorganischen Pigmenten, vorzugsweise aus den Metalloxiden. In einer bevorzugten Ausführungsform ist das Pigment ausgewählt aus der Gruppe bestehend aus Titandioxid, Zinkoxid, Eisenoxid und Mischungen daraus.

Die Pigmente können sowohl einzeln als auch in Mischungen vorliegen.
Bevorzugt im Sinne der vorliegenden Erfindung sind Pigmentmischungen aus Weiss-Pigmenten (z. B. Kaolin, Titandioxid oder Zinkoxid) und anorganischen Farbpigmenten (z. B. Eisenoxid Pigmente, Chromoxide), wobei die Pigmente beschichtet ("gecoatet") oder unbeschichtet vorliegen koennen. Unter den Farbpigmenten sind Eisenoxide besonders bevorzugt.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäß vorteilhaft zusätzlich zu einem oder mehreren Weiss- und/oder Farbpigmenten eingesetzt.

Die bedeutendste Gruppe der Effektpigmente stellen die Glanzpigmente dar, zu denen nach **DIN 55944: 2003-11** die Metalleffektpigmente und die Perlglanzpigmente gehören. Einige spezielle Effektpigmente lassen sich diesen beiden Gruppen nicht zuordnen, z. B. plättchenfoermiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenfoermige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phänomen der Interferenz (Interferenzpigmente) (DIN 55944: 2003-11).

Beispiele für erfindungsgemäß bevorzugte handelsübliche Effektpigmente sind: Timiron and #174; von Merck, Iriodin and #174; von Merck (Perl- und Farbglanzpigmente für dekorative technische Anwendungen), Xirallic and #174; von Merck (farbintensive Kristalleffektpigmente).

Ferner können die erfindungsgemäßen Zubereitungen vorteilhaft auch organische Farbpigmente enthalten, d. h. organische Farbstoffe, welche in der Zubereitung praktisch unlöslich sind. Nach **DIN 55944: 1990-04** können organische Pigmente nach chemischen Gesichtspunkten in Azopigmente und polycyclische Pigmente sowie nach farblichen Gesichtspunkten in Bunt- oder Schwarzpigmente eingeteilt werden.

Die Pigmente können vorteilhaft im Sinne der vorliegenden Erfindung auch in Form kommerziell erhältlicher öliger oder wässriger Vordispersionen zur Anwendung kommen.

Die erfindungsgemäßen Zubereitungen enthalten üblicherweise 0,1 bis 40 Gew.-% Pigmente - bezogen auf das Gesamtgewicht der kosmetischen und/oder pharmazeutischen Zubereitung.
Es ist ferner vorteilhaft im Sinne der vorliegenden Erfindung, wenn die erfindungsgemäße Zubereitung einen oder mehrere Farbstoffe enthält.

Die Farbstoffe können sowohl synthetischen als auch natürlichen Ursprungs sein.
Eine Liste von geeigneten Farbstoffen findet sich in EP 1 371 359 A2**,** S.8, Z. 25-57, S.9 und S.10 sowie S.11, Z. 1 bis 54, auf welche hiermit explizit Bezug genommen wird.

Die erfindungsgemäßen Zubereitungen enthalten üblicherweise 0,01 bis 5, vorzugsweise 0,1 bis 1,0 Gew.-% Farbstoffe- bezogen auf das Gesamtgewicht der kosmetischen und/oder pharmazeutischen Zubereitung. Die erfindungsgemäßen Zubereitungen enthalten üblicherweise eine Gesamtmenge an Farbstoffen und Pigmenten im Bereich von 0,01 bis 30 Gew.-%, insbesonderen 0,1 bis 15 Gew.-%, vorzugsweise 1 bis 10 Gew.-%, bezogen auf das Gesamtgewicht der kosmetischen und/oder pharmazeutischen Zubereitung.

Als Farbstoffe und Pigmente eignen sich insbesondere die gemäß Annex IV der Kommissions Direktive zugelassenen Farbstoffe und Pigmente (in der Fassung: **Commission Directive 2007/22/EC of 17 April 2007 amending Council Directive 76/768/EEC,** concerning cosmetic products, for the purposes of adapting Annexes IV and VI thereto to technical progress) zugelassen Stoffe, auf die hiermit explizit Bezug genommen wird.

Ein Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 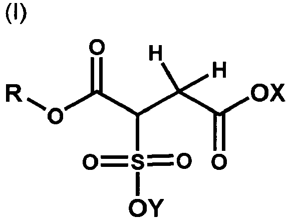 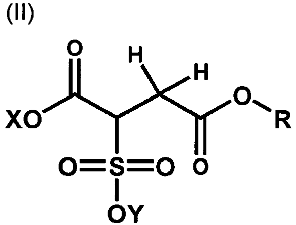
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II) und mindestens eine (weitere) grenzflächenaktive Substanz und/oder eine Wachskomponente und/oder ein Polymer und/oder einen Ölkörper.

Ein weiterer Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 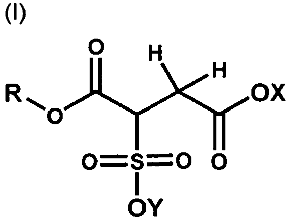 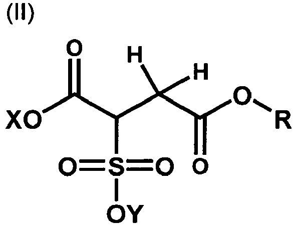
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18**-Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinatemonoester der Formel (I) und (II) enthält;
und mindestens eine (weitere) grenzflächenaktive Substanz und/oder eine Wachskomponente und/oder ein Polymer und/oder einen Ölkörper.

In einer bevorzugten Ausführungsform der Erfindung enthält diese Mischung mindestens 60 Gew.-%, vorzugsweise mindestens 70 Gew.-%, insbesondere mindestens 80 Gew.-% C16-und C18-Alkylsulfosuccinatmonoester bezogen auf die Summe der Alkylsulfosuccinatemonoester der Formel (I) und (II).

### Grenzflächen-aktive Substanz

In einer Ausführungsform der Erfindung enthält die erfindungsgemäße kosmetische und/oder pharmazeutische Zubereitungen mindestens eine grenzflächen-aktive Substanz. Die erfindungsgemäßen Zubereitungen enthalten den/die grenzflächen-aktiven Substanz(en) [ohne die erfindungsgemäßen Alkylsulfosuccinat Mischungen] in einer Menge von 0 bis 80 Gew.-% , insbesonderen 0 bis 40 Gew.-%, vorzugsweise 0,1 bis 20 Gew.-% vorzugsweise 0,1 bis 15 Gew.-% und insbesondere 0,1 bis 10 Gew.-% bezogen auf das Gesamtgewicht der Zubereitung.

Als grenzflächen-aktive Substanz eignet sich prinzipiell jede Substanz, welche die Oberflächenspannung zwischen der wässrigen und der nicht-wässrigen Phase erniedrigt. Grenzflächen-aktive Substanzen umfassen Emulgatoren und Tenside.

Enthält die erfindungsgemäße kosmetische und/oder pharmazeutische Zubereitung die erfindungsgemäße Alkylsulfosuccinat Mischung als grenzflächenaktive Substanz bezieht sich dieser Anspruch auf die Anwesenheit einer weiteren grenzflächenaktiven Substanz

In einer Ausführungsform der Erfindung enthält die erfindungsgemäße Zubereitung mehr als eine grenzflächenaktive Substanz. Der Fachmann setzt in Abhängigkeit der übrigen Komponenten übliche Systeme (wie z.B. Emulgator und Co-Emulgator) ein.

Ein geeigneter Emulgator ist prinzipiell jede grenzflächen-aktive Substanz, insbesondere jedoch Substanzen mit einem HLB-Wert von 1 bis 20 nach der Griffin Skala. Die Griffin Skala ist beschrieben in WC Griffin, J. Soc. Cosmet. Chem. 1 (1949) 311**;** WC Griffin, J. Soc. Cosmet. Chem. 5 (1954) 249**.** Jedem Emulgator wird ein so genannter HLB-Wert (eine dimensionslose Zahl zwischen 1 und 20, Griffin Skala) zugeschrieben, der angibt, ob eine bevorzugte Wasser- oder Öllöslichkeit vorliegt. Zahlen unter 9 kennzeichnen bevorzugt öllösliche, hydrophobe Emulgatoren, Zahlen über 11 wasserlösliche, hydrophile Emulgatoren. Der HLB-Wert sagt etwas über das Gleichgewicht der Größe und Stärke der hydrophilen und der lipophilen Gruppen eines Emulgators aus.

Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Öiphase entsteht unter sonst gleichen Herstellungsbedingungen eine W/O-Emulsion.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls^{®} PGPH" (W/O-Emulgator) oder "Eumulgin^{®} VL 75" (Abmischung mit Lauryl Glucosides im Gewichtsverhältnis 1:1, O/W-Emulgator) oder Dehymuls^{®} SBL (W/O-Emulgator) von der Cognis Deutschland GmbH vertrieben werden. In diesem Zusammenhang sei insbesondere auf das Europäische Patent EP 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Besonders bevorzugte Emulgatoren sind z. B. Cetyl Dimethicone Copolyol (z.B. Abil EM-90), Polyglyceryl-2 Dipolyhydroxystearate (z.B. Dehymuls PGPH), Polyglycerin-3-Diisostearate (z.B. Lameform TGI), Polyglyceryl-4 Isostearate (z.B. Isolan GI 34), Polyglyceryl-3 Oleate (z.B. Isolan GO 33), Diisostearoyl Polyglyceryl-3 Diisostearate (z.B. Isolan PDI), Polyglyceryl-3 Methylglucose Distearate (z.B. Tego Care 450), Polyglyceryl-3 Beeswax (z.B. Cera Bellina), Polyglyceryl-4 Caprate (z.B. Polyglycerol Caprate T2010/90), Polyglyceryl-3 Cetyl Ether (z.B. Chimexane NL), Polyglyceryl-3 Distearate (z. B. Cremophor GS 32) und Polyglyceryl Polyricinoleate (z.B. Admul WOL 1403), Glyceryl Oleate (z.B. Monomuls 90-O 18), Alkyl Glucoside (z.B. Plantacare 1200, Emulgade PL 68/50, Montanov 68, Tego Care CG 90, Tego Glucosid L 55), Methyl Glucose Isostearate (z.B. Tego Care IS), Methyl Glucose Sesquistearate (Tego Care PS), Sodium Cocoyl Hydrolyzed Wheat Protein (z.B. Gluadin WK), Potassium Cetyl Phosphate (z.B. Amphisol K, Crodafos CKP), Sodium Alkylsulfate (z.B. Lanette E), Sucrose Ester (z.B. Crodesta F-10, F-20, F-50, F-70, F-110, F-160, SL-40, Emulgade® Sucro), ethoxylierte und/oder propoxylierte Fettalkohole Fettsäuren, Rizinusöle bzw. hydrierte Rizinusöle (z.B. Eumulgin B2, B2, B3, L, HRE 40, HRE 60, RO 40, Cremophor HRE 40, HRE 60, L, WO 7, Dehymuls HRE 7, Arlacel 989), PEG-30 Dipolyhydroxystearate (z.B. Arlacel P 135, Dehymuls LE), Sorbitan Ester, Sorbitan Ester ethoxyliert und/oder propoxyliert sowie deren Gemische. Ein besonders effektives Gemisch besteht aus Polyglyceryl-2 Dipolyhydroxystearate und Lauryl Glucoside und Glycerin (z.B. Eumulgin VL 75). Geeignet sind weiterhin Polyglyceryl-4 Diisostearate/ Polyhydroxystearate/Sebacate (Isolan^{®} GPS), Diisostearoyl Polyglyceryl-3 Diisostearate (z. B. Isolan PDI), Alkalisalze Acylglutamate (z.B. Eumulgin SG).

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer,"Encyclopedia of Chemical Technology", 3. Aufl., 1979, Band 8, Seite 913**,** aufgelistet. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether), sowie Eumulgin^{®} SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und-oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade^{®} PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin^{®} VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Emulgatoren können beispielsweise Silikonemulgatoren enthalten sein. Diese können beispielsweise aus der Gruppe der Alkylmethicon-copolyole und/oder Alkyl-Dimethiconcopolyole gewählt werden, insbesondere aus der Gruppe der Verbindungen, welche gekennzeichnet sind durch die folgende chemische Struktur: 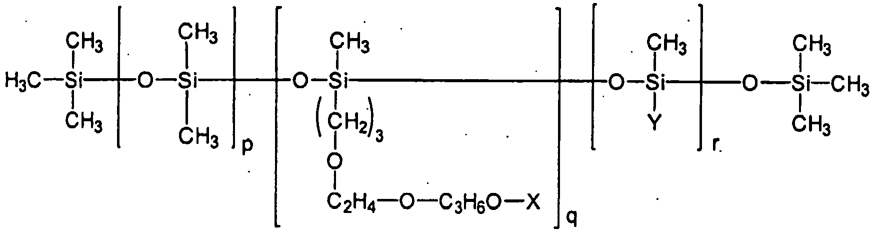 bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendende Silikonemulgatoren sind Dimethiconcopolyole, welche von Evonik Goldschmidt unter den Warenbezeichnungen AXIL® B 8842, ABIL® B 8843, ABIL® B 8847, ABIL® B 8851, ABIL® B 8852, ABIL® B 8863, ABILO B 8873 und ABILOB 88183 verkauft werden.

Ein weiteres Beispiel für besonders vorteilhaft im Sinne der vorliegenden Erfindung zu verwendende grenzflächenaktiven Substanzen ist das Cetyl PEG/PPG-10/1 Dimethicone (Cetyl Dimethiconcopolyol), welches von EvonikGoldschmidt unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von Evonik Goldschmidt unter der Warenbezeichnung ABIL®EM 97 und ABIL®WE 09 verkauft wird.

Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning® 5200 Formulation Aid von der Gesellschaft Dow Corning Ltd. erhältlich ist.
Ein weiterer vorteilhafter Silikonemulgator ist, Octyl Dimethicon Ethoxy Glucosid der Firma Wacker.

Für eine erfindungsgemäße Wasser-in-Silikonöl-Emulsion können alle bekannten für diesen Emulsionstyp verwendeten Emulgatoren eingesetzt werden. Erfindungsgemäß besonders bevorzugte Wasser-in-Silikon-Emulgatoren sind dabei Cetyl PEG/PPG- 10/1 Dimethicone und Lauryl PEG/PPG-18/18 Methicone [z. B. ABIL® EM 90 Evonik Goldschmidt), DC5200 Formulation Aid (Dow Corning)] sowie beliebige Mischungen aus beiden Emulgatoren.

In einer Ausführungsform der Erfindung enthält die erfindungsgemäße kosmetische und/oder pharmazeutische Zubereitung als grenzflächenaktive Substanz weiterhin mindestens ein Tensid.

Als grenzflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als zwitterionische Tenside werden solche oberflächenaktiven Verbindungen bezeichnet, die im Molekül mindestens eine quartäre Ammoniumgruppe und mindestens eine -COO⁽⁻⁾- oder-SO₃⁽⁻⁾-Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

Anionische Tenside sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Dialkylsulfosuccinate und Acylglutamate in Form ihrer Alkali- oder Ammoniumsalze.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid(ether)sulfate, Fettsäureamid(ether)sulfate, Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Ein geeignetes anionisches Tensid ist beispielsweise Glyceryl Stearate Citate.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyl-dimethylammoniumchlorid, Lauryldimethylammoniumchlorid, Lauryldimethylbenzyl-ammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zusammensetzungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

In einer Ausführungsform der Erfindung enthält die erfindungsgemäße kosmetische und/oder pharmazeutische Zubereitung weiterhin mindestens eine Wachskomponente.

Die erfindungsgemäßen Zubereitungen enthalten den/die Wachskomponente(n) üblicherweise in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zubereitung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30 °C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten TriglyceridGemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C12-C18-Mono-, Di- und Triglyceriden) sowie Cutina MD oder Cutina GMS (Glycerylstearat).
Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der Cognis Deutschland GmbH unter der Bezeichnung Lanette 18 (C18-Alkohol), Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zusammensetzungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat, - myristat oder -stearat kommen hierfür in Frage.

Als Wachse eignen sich weiterhin Perlglanzwachse. Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Polymere

In einer Ausführungsform der Erfindung enthält die erfindungsgemäße kosmetische und/oder pharmazeutische Zubereitung weiterhin mindestens ein Polymer.

Die erfindungsgemäßen Zubereitungen enthalten das/die Polymere(n) üblicherweise in einer Menge von 0 bis 20 Gew.-%, vorzugsweise 0,1 bis 15 Gew.-% und insbesondere 0,1 bis 10 Gew.-% bezogen auf das Gesamtgewicht der Zubereitung.

Geeignete kationische Polymere sind beispielsweise kationische Cellulosederivate, wie z. B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxy-propyl hydrolyzed collagen (Lamequat^{®}L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z. B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat Copolymere, Methylvinylether/Maleinsäureanhydrid-Copolymere und deren Ester, unvernetzte und mit Polyolen vernetzte Polyacrylsäuren, Acryl-amidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylamino-ethylmethacrylat/Vinylcaprolactam-Terpolymere sowie gegebenenfalls derivatisierte Celluloseether und Silicone in Frage.

Als Polymere eigen sich ebenso Polysaccharide, insbesondere Xanthan-Gum, Guar-Guar, Agar-Agar, Alginate und Tylosen sowie beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Ebenso geeignet sind sogenannte quaternäre Polymere, z.B. mit der INCI - Bezeichnung Polyquaternium-37, die der folgenden allgemeinen Formel entsprechen:

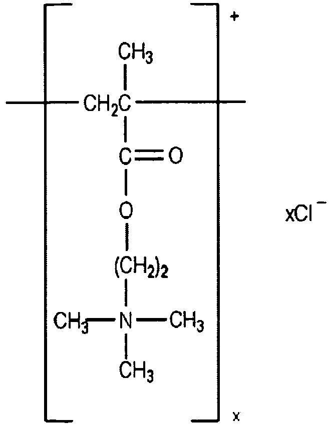

Alternativ können auch andere Dialkylaminoalkyl (meth)acrylate sowie deren durch Alkylierung oder Protonierung erhältliche Ammoniumsalze oder Dialkylaminoalkyl (meth)acrylamide sowie deren durch Alkylierung oder Protonierung erhältliche Ammoniumsalze eingesetzt werden. Besonders bevorzugt sind Polymere enthaltend MAPTAC, APTAC, MADAME, ADAME, DMAEMA und TMAEMAC. Darüberhinaus können auch Co-polymere mit anionischen, weiteren kationischen oder ungeladenen Monomeren erfindungsgemäß eingesetzt werden, insbesondere solche, die neben den genannten Alkylaminoalkyl (meth)acrylat oder -(meth)acrylamid Monomeren zusätzlich (Meth)acrylsäure und/oder 2-Acrylamido-2-methyl-propansulfonsäure und/oder Acrylamid und/oder Vinylpyrrolidon und/oder Alkyl(meth)acrylate enthalten.

Beispielhaft seien solche Polymere mit der INCI Bezeichnung Polyquaternium-11, Polyquaternium-13, Polyquaternium-14, Polyquaternium-15, Polyquaternium-28, Polyquaternium-32, Polyquaternium-43, Polyquaternium-47 genannt.

### Ölkörper

In einer Ausführungsform der Erfindung enthält die erfindungsgemäße kosmetische und/oder pharmazeutische Zubereitung weiterhin mindestens einen Ölkörper.

Die Ölkörper sind üblicherweise in einer Gesamtmenge von 0,1 - 90, insbesondere 0,1 - 80, insbesondere 0,5 bis 70, bevorzugt 1 bis 60, insbesondere 1 bis 50 Gew.-%, insbesondere 1 bis 40 Gew.-%, vorzugsweise 5 - 25 Gew.-% und insbesondere 5 - 15 Gew.-% enthalten. Die weiteren Ölkörper sind üblicherweise in einer Menge von 0,1 bis 40 Gew.-% -bezogen auf das Gesamtgewicht der Zubereitung- enthalten.

Als weitere Ölkörper kommen beispielsweise Guerbetalkohole auf Basis von Fettalkoholen mit 6 bis 18, vorzugsweise 8 bis 10 Kohlenstoffatomen, in Frage sowie weitere, zusätzliche Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen und Kohlenwasserstoffen oder deren Gemische. Weiterhin geeignet sind Ester von 2-Propylheptanol mit n-Octansäure, wie z.B. kommerziell erhältlich unter dem Handelsnamen Cetiol®SenSoft (Cognis GmbH). Weiterhin geeignet sind Kohlenwasserstoffe, wie zum Beispiel n-Undecan und n-Tridecan. Weiterhin geeignet sind Alkane, wie z.B. die Gemische mit der INCI Bezeichnung Conocnut/Palm/Palm Kernel Oil Alkanes (Handelsname Vegelight 1214 der Fa. Biosynthesis).

Als weitere Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel 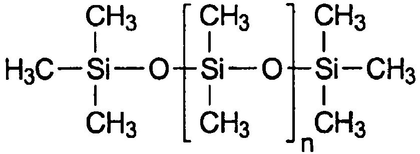 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handelsbezeichnungen Abil 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Goldschmidt erhältlich sind. Aber auch andere Silikonöle sind vorteilhaft im Sinne der vorliegenden Erfindung zu verwenden, beispielsweise Cetyidimethicon, Hexamethylcyclotrisiloxan, Polydimethylsiloxan, Poly(methylphenylsiloxan). Erfindungsgemäß besonders bevorzugte Silikone sind Dimethicon und Cyclomethicon.

Die erfindungsgemäßen Zubereitungen können weiterhin biogenen Wirkstoffe, Insekten-Repellentien, Tyrosinase Inhibitoren, Konservierungsmittel, Parfümöle, Überfettungsmittel, Stabilitsatoren und/oder Hydrotrope enthalten.

Ein Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 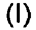 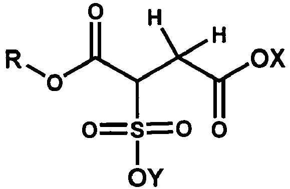 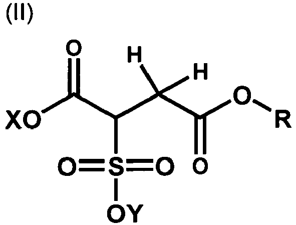
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II)
und mindestens eine Verbindung ausgewählt aus der Gruppe bestehend aus biogenen Wirkstoffen, Insekten-Repellents, Tyrosinase Inhibitoren, Konservierungsmitteln, Parfümölen, Stabilisatoren und/oder Hydrotropen.

Ein weiterer Gegenstand der Erfindung betrifft kosmetische und/oder pharmazeutische Zubereitungen, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 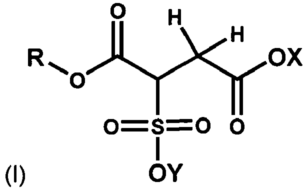 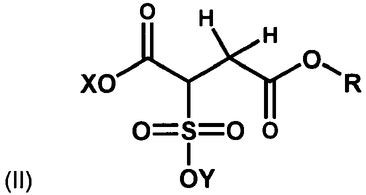
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, dadurch gekennzeichnet, dass die Mischung
   - 30 bis 70 Gew.-% C16-Alkylsulfosuccinatmonoester und
   - 30 bis 70 Gew.-% C18-Alkylsulfosuccinatmonoester enthält
wobei die Gew.-% bezogen sind auf die Gesamtmenge der **in der Mischung vorhandenen C16- und C18**-Alkylsulfosuccinatmonoester der Formel (I) und (II),
und wobei die Mischung mindestens 50 Gew.-% C16- und C18-Alkylsulfosuccinatmonoester bezogen auf die Summe der Alkylsulfosuccinatemonoester der Formel (I) und (II) enthält;
und mindestens eine Verbindung ausgewählt aus der Gruppe bestehend aus biogenen Wirkstoffen, Insekten-Repellents, Tyrosinase Inhibitoren, Konservierungsmitteln, Parfümölen, Stabilisatoren und/oder Hydrotropen.

In einer bevorzugten Ausführungsform der Erfindung enthält diese Mischung mindestens 60 Gew.-%, vorzugsweise mindestens 70 Gew.-%, insbesondere mindestens 80 Gew.-% C16- und C18-Alkylsulfosuccinatmonoester bezogen auf die Summe der Alkylsulfosuccinatemonoester der Formel (I) und (II).

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Aloe vera, Prunusextrakt, Bambaranussextrakt und Vitaminkomplexe zu verstehen. Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe. Weiterhin eignen sich als Konservierungsmittel die in WO07/048757 beschriebenen 1,2 Alkandiole mit 5 bis 8 C-Atomen.

Als Konservierungsmittel eignen sich insbesondere die gemäß Annex VI der Kommissions Direktive (in der **Fassung Commission Directive 2007/22/EC of 17 April 2007 amending Council Directive 76/768/EEC,** concerning cosmetic products, for the purposes of adapting Annexes IV and VI thereto to technical progress) zugelassen Stoffe, auf die hier explizit Bezug genommen wird.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als Stabilisatoren können Metallsalze von Fettsäuren, wie z. B. Magnesium-, Aluminium-und/oder Zinkstearat bzw. -ricinoleat eingesetzt werden.
Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

### Beispiele

### 1. Herstellungsbeispiel

Stufe 1: In einem 2 Liter Glaskolben wurden 382,5 g (1,5 Mol) eines C16/18 Fettalkohol Gemisches (INCI: Cetearyl Alkohol, Handelsname Lanette^{®} O, erhältlich von der Fa. Cognis GmbH), 149,3 g (1,52 Mol) Maleinsäureanhydrid und 1,9 Natriumcarbonat eingewogen und 3 Stunden unter Rühren auf 80°C erhitzt.

Stufe 2: In einem 4 Liter Glaskolben wurden 198,5 g (1,53 Mol) Natriumsulfit in 1336,0 g Wasser vorgelegt und unter Rühren auf 80°C aufgeheizt. Dazu wurden unter Rühren 518 g der Stufe 1 gegeben und 2 Stunden bei 80°C gerührt. Das weiße feste Produkt hatte die folgende Zusammensetzung:
Sulfosuccinat : 29, 3 Gew. % (davon 48 Gew.-% C16 Sulfosuccinatmonoester und 52 Gew.-% C18 Sulfosuccinatmonoester)
Natriumsulfat: 0,78 %; Trockenrückstand: 34,8 %; C16/18 Alkohol: 1,2 % (=3,4 % bezogen auf Trockenrückstand). Dies entspricht einem Anteil an Fettalkohol von 4,1 Gew.-% bezogen auf die Sulfosuccinate.

Stufe 3: Die Sprühtrocknung wurde in dem Sprühturm Typ Mobile Minor 2000, Fa. Niro mit 2-Stoffdüse und Taumelkolbenpumpe unter den folgenden Bedingungen durchgeführt:

| | |
|---|---|
| Sprühdruck: | 0,5 bar |
| Trocknungsgasmenge: | 100 m³/h |
| Trocknungsgastemperatur, Eintritt: | 170 °C |
| Trocknungsgastemperatur, Austritt: | 103 °C |
| Sprührate: | 900 g/h |

Das Produkt aus Stufe 1 wurde vor der Trocknung mit 40% Wasser verdünnt, um die Viskosität abzusenken und die Lösung pumpfähig zu machen. Die Trocknung erfolgte im Gegenstromverfahren, wobei die wässerige Lösung von unten nach oben und das Trocknungsgas von oben nach unten geführt wurden. Das Produkt ließ sich ohne Probleme auf einen Wassergehalt von 1,5 % sprühtrocknen. Es wurden keine Anbackungen an den Wänden beobachtet. Das so erhaltende Pulver hatte folgende Zusammensetzung:
Sulfosuccinate: 72 Gew.-% (davon 48 Gew.-% C16 Sulfosuccinatmonoester und 52 Gew.-% C18 Sulfosuccinatmonoester) Natriumsulfat 2,6 %, Wasser : 1,6 %, C16/18 Alkohol: 3,4 %. Dies entspricht einem Anteil an Fettalkohol von 4,7 Gew.-% bezogen auf die Sulfosuccinate.

### 2. Emulgiereigenschaften

Mit der gemäß Herstellbeispiel 1 hergestellten erfindungsgemäßen Alkysulfosuccinat Mischung (in Pulverform) wurde die nachstehende Emulsion zubereitet. Als Vergleichsbeispiel diente ein kommerziell erhältlicher Emulgator: Amphisol®K, INCI Potassium Cetyl Phosphate, erhältlich von DSM Nutritional Produkts. Wie aus der nachstehenden Tabelle ersichtlich, wird nur mit der erfindungsgemäßen Alkylsulfosuccinat Mischung eine stabile Emulsion erhalten. Alle Angaben in Gew.-%

| **Rezeptur** | **Erfindungsgemäßes Beispiel** | **Vergleichsbeispiel** |
|---|---|---|
| Phase I: | | |
| Alkylsulfosuccinat Mischung gemäß Beispiel 1 | 1,0 | - |
| Amphisol® K(Potassium Cetyl Phosphate) | - | 1,0 |
| Lanette®O (Cetearyl alkohol) | 5,0 | 5,0 |
| Cetiol®LC (Coco caprylat/caprate) | 16,0 | 16,0 |
| | | |
| Phase II | | |
| Wasser | 72,9 | 72,9 |
| Glycerin | 3,0 | 3,0 |
| Natriumchlorid | 1,0 | 1,0 |
| | | |
| Phase III: Phenonip® XB (Mischung aus Phenoxyethanol and Methylparaben and Propylparaben and Ethylparaben) | 1,0 | 1,0 |
| Phase IV: Euxyl® K100 (Mischung aus Benzyl Alcohol and Methylchloroisothiazolinone and Methylisothiazolinone) | 0,1 | 0,1 |
| | | |
| Optik nach Herstellung | weiße Emulsion | getrennte Emulsion, Wasser unten |
| Viskosität Tag 1, Brookfield RVF, Spindel TE, 4 UpM, mit Helipath | 125000 mPa*s | - |
| pH | 5,8 | - |

Die Beispiele wurde wie folgt hergestellt: Phase I wurde auf 85 °C erwärmt und homogen verrührt, Phase II wurde auf 85°C erwärmt und unter Rühren zu Phase I gegeben. Die Mischung wurde unter Rühren abgekühlt. Bei 45°C erfolgte die Zugabe der Phase III; bei 35°C die Zugabe der Phase IV. Bei Erreichen von 30 °C wurde das Rühren beendet.

Die nachfolgenden Rezepturen enthalten die Alkylsulfosuccinat Mischung gemäß Herstellbeispiel 1, die INCI Bezeichnung dieser Verbindung lautet Disodium Cetearyl Sulfosuccinate, sie ist unter dem Handelsnamen Eumulgin®Prisma von der Fa. Cognis GmbH, Düsseldorf, erhältlich. Alle Angaben sind Gew.-% bezogen auf die Gesamtzusammensetzung.

**Tabelle 1: O/W "Body Care" Emulsionen - Emulsionen zur Körperpflege**

| **Bestandteile: Handelsname** | **1** | **2** | **3** | **4** | **5** | **6** | **7** | **8** | **9** | **10** | **11** |
|---|---|---|---|---|---|---|---|---|---|---|---|
| **C - Creme, L - Lotion** | **C** | **C** | **C** | **L** | **C** | **L** | **L** | **C** | **L** | **C** | **C** |
| Eumulgin^{®} VL 75 | | | | | | | | 2,0 | | 1.5 | |
| Dehymuls^{®} PGPH | | 0.6 | | | | | | | | | |
| Generol^{®} R | | | 0.5 | | | | | | | | |
| Eumulgin^{®} B2 | | | 2,0 | | | | | | 2,0 | | |
| Tween^{®} 60 | | | | 0.2 | | | | | | | |
| Cutina^{®} E 24 | | | | 0.2 | | | | | | | |
| Hostaphat^{®} KL 340 N | | | | | | | | | 0.5 | | |
| Lanette^{®} E | | | | | | | | 0.6 | | | |
| Amphisol^{®} K | | | 0.2 | | | | | | | | |
| Sodium Stearate | | | | | 0.5 | | | | | | |
| Emulgade^{®} PL 68/50 | 3,0 | | | | | | 2,0 | | | | 1.2 |
| Eumulgin^{®} SG | 0,2 | | | | 0,2 | 0,3 | | | | | |
| Eumulgin^{®} Prisma | 0,1 | 0,2 | 0,1 | 0,1 | 0,1 | 0,5 | 0,2 | 0,1 | 0,1 | 0,2 | 0,5 |
| Imwitor 372 P | | 2,0 | 2,0 | | | 3,0 | | | | 3,0 | 3,0 |
| Tego^{®} Care CG | 0.7 | | | | | | | | | | |
| Tego^{®} Care 450 | | | | | 3 | | 1,0 | | | 1,0 | |
| Cutina^{®} PES | 2.5 | 2 | 3 | | | 2 | | 1.7 | 2.5 | | 1.2 |
| Cutina^{®} MD | | 1 | | 3 | 5 | | 2 | | | 3 | |
| Lanette^{®} 14 | | | | 1 | | | | 4 | | | 4 |
| Lanette^{®} O | 4.5 | | 4 | | 1 | | | | | | 2 |
| Novata^{®} AB | | 1 | | | | | | | | | 1 |
| Emery^{®} 1780 | | | | | 0.5 | 0.5 | | | | | |
| Lanolin, water-free, USP | | | | | | | 1.1 | | | | |
| Cosmedia^{®} DC | | 1.5 | 2 | | | 1.5 | 2 | | 1.5 | 1.5 | |
| Cetiol^{®} SB 45 | | | 1.5 | | | | 2 | | | | |
| Cegesoft^{®} C 17 | | | | | | | | | | | 2 |
| Myritol^{®} PC | | | | | 5 | | | | | | |
| Myritol^{®} 331 | 2 | 5 | 1 | | | 6 | | 6 | | | |
| Finsolv^{®} TN | | | 2 | | | 2 | 4 | | | | |
| Cetiol^{®} Sensoft | 4 | 3 | | 5 | 4 | 4 | | 6 | 8 | | 5 |
| Cetiol^{®} CC | 2,0 | 3 | | | | 2,0 | 4 | | | 3,0 | 5 |
| Cetiol^{®} OE | | | 2,0 | | | | | | 4 | | |
| Dow Corning DC^{®} 245 | | | 2 | | 1 | 1 | | | | | |
| Dow Corning DC^{®} 2502 | | | | | 2 | 1 | | | | 3 | |
| Prisorine^{®} 3758 | | | 4 | | | 1 | | | | | |
| Silicone Oil Wacker AK^{®} 350 | 0.5 | 0.5 | 0.5 | | | 1 | | | | | |
| Cetiol^{®} 868 | | | | | 2 | | 4 | | | | |
| Cetiol^{®} J 600 | 2 | | 3 | | 3 | 2 | | | | 5 | |
| Ceraphyl^{®} 45 | | | | | | | 3 | | | | |
| Mineral Oil | | | | 9 | | | | | | | |
| Cetiol^{®} SN | | | 5 | | | | | | | | |
| Cetiol^{®} B | | | | | | | | 4 | | 2 | |
| Eutanol^{®} G | | 2 | | 3 | | | | | | | |
| Cetiol^{®} PGL | | | | | | | | | 5 | 5 | |
| Dry Flo ^{®} Plus | 5 | | | | | | 1 | | | | |
| SFE 839 | 5 | | | | | | | | | | 2 |
| Almond Oil | | | | | | | 1 | | | | |
| Insect Repellent^{®} 3535 | | 2 | 4 | | | 2 | | | | 3 | |
| N,N-Diethyl-m-toluamide | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopheryl Acetate | 1 | | | | | | | | | | |
| Veegum^{®} Ultra | | | | | | | | | 1 | | |
| Keltrol^{®} T | | | 0.4 | | | | | | 0.5 | | |
| Cosmedia^{®} SP | | 0.2 | | 0.2 | 0,2 | | | | 0.2 | 0.3 | |
| Pemulen^{®} TR 2 | | | | | | | | 0.3 | | | |
| Carbopol^{®} Ultrez 10 | | | | | | 0.2 | | | | | |
| Rheocare® C Plus | 0.3 | 0,1 | 0.3 | | 0.2 | | | | | | |
| Ultragel™ 300 | | | | 0,3 | | | | | 0,2 | | |
| Ethanol | | | | | | | | | | 10 | |
| Butylene glycol | | | | 4 | 3 | | 2 | 5 | 2 | | |
| Glycerin | 2 | 5 | 5 | | 3 | 3 | 2 | | 4 | | 3 |
| Water, Preservatives, NaOH | ad 100, q.s., pH 6,5 - 7,5 | | | | | | | | | | |

**Tabelle 2: W/O Body Care Emulsions - W/O Emulsionen zur Körperpflege**

| **Bestandteile: Handelsnamen** | **1** | **2** | **3** | **4** | **5** | **6** | **7** | **8** | **9** | **10** | **11** |
|---|---|---|---|---|---|---|---|---|---|---|---|
| **L = Lotion, C = Creme** | C | L | C | L | C | L | L | L | C | C | C |
| Dehymuls^{®} PGPH | 1 | 2 | 1 | 2 | 3 | 1 | 1 | 2 | | | 1 |
| Monomuls^{®} 90-018 | 2 | | | | | | | | 2 | | 2 |
| Lameform^{®} TGI | 4 | 1 | | | 3 | | | 1 | 4 | 3 | |
| Abil^{®} EM 90 | | | | | | | 4 | | 1 | | |
| Isolan GPS | | | 2 | | 2 | | | | | 1 | |
| Isolan^{®} PDI | | | | | | 4 | | | | | 1 |
| Glucate^{®} DO | | | | 3 | | | | | | | |
| Arlacel^{®} 83 | | | 4 | | | | | | | | |
| Dehymuls^{®} LE | | 1 | 1 | 2 | | | | | | 1 | 1 |
| Dehymuls^{®} HRE 7 | | | | | | | | 4 | | 1 | |
| Zinc Stearate | 2 | 1 | | 1 | 1 | | | 1 | 1 | 1 | |
| Microcristalline Wax | | | 5 | | | 2 | | | | | 5 |
| Bees wax | 4 | | | 1 | | | | 1 | 4 | 7 | |
| Imwitor 372 P | | | | | 1 | | | | | | |
| Tego Care^{®} CG | | | | | 1 | | | | | | 0,5 |
| Eumulgin^{®} Prisma | 0,1 | 0,05 | 0,1 | 0,15 | 0,05 | 0,05 | 0,1 | 0,1 | 0,15 | 0,05 | 0,2 |
| Prisorine^{®} 3505 | | | 1 | 1 | | 1 | 1 | | | | 1 |
| SFE^{®} 839 | | | | | | | 3 | | | | |
| Emery^{®} 1780 | 1 | | | | | | | | | | 1 |
| Anhydrous Lanolin USP | | | 5 | | | | | | | 4 | |
| Cetiol^{®} Sensoft | 3 | 4 | | | 6 | | 2 | 6 | 3 | 8 | |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | 8 |
| Finsolv^{®} TN | | | | 5 | | 4 | 5 | | | | |
| Cetiol^{®} A | | 6 | | | | 4 | | | | | |
| Cetiol^{®} CC | | 8 | | 6 | 6 | 2 | 2 | | | 4 | 6 |
| Cetiol^{®} SN | | 5 | | | | | | 3 | | | |
| Cetiol^{®} OE | 3 | | 2 | | 4 | | 2 | | 4 | 2 | |
| Dow Corning DC^{®} 244 | | | | 6 | 1 | | 2 | | | | |
| Dow Corning DC^{®} 2502 | | | 1 | | 2 | | | | | | |
| Prisorine^{®} 3758 | | | | | 3 | | | | | | |
| Silicon Oil Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | | 6 | |
| Mineral oil | | | | | 4 | | | | | | |
| Cetiol^{®} B | | | 2 | 4 | | | | | | 3 | |
| Eutanol^{®} G 16 | | 1 | | | | | | | | 3 | |
| Eutanol^{®} G | | | 3 | | | | | 8 | | | |
| Cetiol^{®} PGL | | | | | | 4 | | | 9 | | |
| Almond Oil | | | | | 1 | | 5 | | | | |
| Insect Repellent^{®} 3535 | 2 | | | | | | | | | | |
| Unirep^{®} U-18 | | | | 3 | | | | 5 | | | |
| Photonyl^{®} LS | 2 | 2 | | | | | | | | | |
| Panthenol | 1,0 | | | | | | | | | | |
| Bisabolol | 0,2 | | | | | | | | | | |
| Copherol^{®} 1250 C | 1 | | | | | | | | | | |
| MgSO₄ x 7 H₂O | 1 | | | | | | | | | | |
| Bentone^{®} 38 | | | | | 1 | | | | | | |
| Propylene Carbonate | | | | | 0,5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Water, Preservative | ad 100, q.s. | | | | | | | | | | |

**Tabelle 3: O/W Sun Care Emulsions - O/W Emulsionen zum Sonnenschutz**

| **Bestandteile (Handelsnamen)** | **1** | **2** | **3** | **4** | **5** | **6** | **7** | **8** | **9** | **10** | **11** |
|---|---|---|---|---|---|---|---|---|---|---|---|
| **C - Creme, L - Lotion** | **L** | **C** | **S** | **L** | **C** | **L** | **L** | **C** | **L** | **C** | **L** |
| Dehymuls^{®} PGPH | | | | | | | 1,5 | | 1 | | |
| Eumulgin^{®} VL 75 | | | | | | | | 2 | | | 2 |
| Eumulgin^{®} B2 | | | | 0.5 | | | | | | | |
| Tween^{®} 60 | | | | 0.2 | | | | | | | |
| Myrj^{®} 51 | | | | 0.5 | | | | | | | |
| Cutina^{®} E 24 | | | | 0.1 | | | | | | | |
| Hostaphat^{®} KL 340 N | | | | | | | | | 1.6 | | |
| Lanette^{®} E | | | | | | | | | | | 0.1 |
| Amphisol^{®} K | | | | | | | | | | 1 | |
| Sodium Stearate | | | | | | | 1 | | | | |
| Emulgade^{®} PL 68/50 | | 2 | 1 | | | 2 | 2 | | | 2 | |
| Imwitor 372 P | | 2 | | | 2 | 1 | | 2 | | | |
| Eumulgin^{®} SG | | 0,5 | | | | 0,1 | | 0,2 | | | |
| Eumulgin^{®} Prisma | 0,5 | 0,2 | 0,2 | 0,2 | 0,75 | 0,2 | 0,2 | 0,1 | 0,2 | 0,4 | 0,3 |
| Tego^{®} Care 450 | | | | | | 2 | | | | 1 | 2.5 |
| Cutina^{®} PES | 2 | | 2.5 | 1 | 2.5 | | 2.5 | | 2.5 | 1.7 | 1.5 |
| Cutina^{®} MD | 2 | | 1 | 2 | | | 2 | | | 6 | |
| Lanette^{®} 14 | 1 | | | 1 | | | | 2 | | | 2 |
| Lanette^{®}O | 1 | 6 | | | 5 | 2 | | 2 | | | |
| Cosmedia^{®} DC | 1 | 1.5 | | 1 | 1 | | 2 | 2 | | | 2 |
| Antaron^{®}V 216 | | | 2 | | | 1.5 | | | 1 | 1 | |
| Emery 1780 | | | | | 0.5 | 0.5 | | | | | |
| Lanolin, water-free USP | | | | | | | 5 | | | | |
| Myritol^{®} PC | | | | | 5 | | | | | | |
| Myritol^{®} 331 | | | 8 | | | 6 | | 10 | | 2 | |
| Finsolv^{®} TN | 3 | | 1 | 5 | | | | 1 | | 2 | |
| Cetiol^{®} Sensoft | | 2 | 3 | | 3 | 4 | 3 | 2 | 5 | | 5 |
| Cetiol^{®} CC | 5 | 2,5 | 2 | | 2 | | 1 | | 3 | | |
| Cetiol^{®} OE | | | 3 | | | | | | 2 | 3 | |
| Dow Corning DC^{®} 244 | 4 | | 1 | | | | | 2 | | | 2 |
| Dow Corning DC^{®} 2502 | | 1 | | | 2 | | | | | | |
| Squatol^{®} S | | | | | | | 4 | | | | |
| Silicone Oil Wacker AK^{®} 350 | | 2 | | | | | | | | | |
| Cetiol^{®} 868 | | | | | 2 | | 4 | | | | 2 |
| Cetiol^{®} J 600 | | | | | 3 | 2 | | | | 5 | |
| Mineral Oil | | | | 4 | | | | | | | |
| Cetiol^{®} B | | | 1 | | | | | | | 2 | |
| Eutanol^{®} G | | | | 2 | | | | | 4 | | |
| Eutanol^{®} G 16 | 4 | | | | | 4 | | | | | |
| Cetiol^{®} PGL | | 5 | | | | | | | | 5 | |
| Almond Oil | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopheryl Acetate | 1 | | | | | | | | | | |
| Photonyl^{®} LS | | | | | | | | | | | |
| Neo Heliopan^{®} AP (Na-salt) | | 1 | | | | | | | 1 | | |
| Neo Heliopan^{®} Hydro (Na-salt) | | 1 | 2.2 | | | | | | 1 | | |
| Neo Heliopan^{®} 303 | 3 | 5 | 4 | 4 | | 2 | | | | | |
| Neo Heliopan^{®} BB | | | | | 1 | | | | | | 2 |
| Neo Heliopan^{®} MBC | | | | 3 | | 2 | 2 | 2 | | | 1 |
| Neo Heliopan^{®} HMS | | 2 | | | | | | | 5 | | |
| Neo Heliopan^{®} OS | | 2 | | | | | | | 5 | | |
| Neo Heliopan^{®} E 1000 | | | | 3 | | | | | | | 6 |
| Neo Heliopan^{®} AV | | | 7.5 | 3 | | 4 | 4 | 5 | | | |
| Uvinul^{®} A Plus | | 2 | | 2 | 1 | | | | 2 | | 1 |
| Uvinul^{®} T 150 | | 2 | | | 2.5 | | | 1 | | 2 | 2.5 |
| Tinosorb^{®} M | | | 3 | | 2 | | 2 | 2 | | | 2 |
| Tinosorb^{®} S | | | 1 | | 1 | | 1,5 | 1 | | | 1 |
| Uvasorb^{®} HEB | | 1 | | | 1 | | | | | | |
| Parsol^{®} 1789 | 3 | | 1 | | 1 | 1 | 2 | | 2 | 2 | 1 |
| Mexoryl SX (Na-salt) | 1 | | | | | 2 | 1 | | | 2 | |
| Mexoryl XL | 2 | | | | | 2 | 2 | | | 3 | |
| Zinkoxid NDM | | | 5 | | | 5 | | 3 | | 5 | 4 |
| Eusolex^{®} T 2000 | 5 | | | | 5 | 5 | 3 | 3 | | | 4 |
| Veegum^{®} Ultra | 1.5 | | 0.75 | | | | | 1 | 1 | | |
| Keltrol^{®} T | 0.5 | | 0.25 | | | | | 0.5 | 0.5 | | |
| Cosmedia^{®} SP | | 0.2 | | | 0.2 | | 0.2 | 0.2 | | 0.2 | 0.2 |
| Ultragel™ 300 | | | | 0.4 | | 0.2 | | | 0.1 | | |
| Rheocare® C Plus | 0,1 | 0,3 | | | 0,3 | | 0,1 | | | 0.3 | 0.2 |
| Ethanol | | | | | | | | | | 10 | |
| Butylene glycol | | 2 | | 4 | 3 | | 2 | 5 | 2 | | 2 |
| Glycerin | 5 | 5 | 5 | | 3 | 3 | 2 | | 4 | | 3 |
| Preservatives, NaOH, Water | q.s.ad 100 | | | | | | | | | | |

**Tabelle 4: Dekorative Kosmetik - O/W Foundations**

| **Bestandteile (Handelsnamen)** | **1** | **2** | **3** | **4** | **5** | **6** | **7** | **8** |
|---|---|---|---|---|---|---|---|---|
| Cutina^{®} GMS-SE | 5,5 | | | | | | | |
| Emulgade^{®} PL 68/50 | | 5,0 | | | | 2,0 | | |
| Eumulgin^{®} VL 75 | | | | 3,0 | | | 5,0 | |
| Tego Care^{®} 450 | | | | | | 2,0 | 2,0 | |
| Crodesta^{®} F-50 | | | | | 6,0 | | | |
| Amphisol^{®} K | | | | 2,0 | | | | |
| Lanette^{®} E | | 0,25 | | | | | | |
| Eumulgin^{®} SG | | | | | 0,5 | | 0,2 | |
| Eumulgin^{®} Prisma | 0,1 | 0,3 | 0,1 | 0,5 | 0,5 | 1,0 | 1,0 | 0,75 |
| Imwitor 372 P | | 2 | | 2 | | | 1 | |
| Cutina^{®} FS 45 | 1,5 | | | | | | 1 | |
| Eumulgin^{®} B 2 | | | 2,0 | | | | | |
| Cutina^{®} PES | 2,0 | 1,0 | 2,0 | | 2,0 | 1,0 | 2,5 | 2,0 |
| Lanette^{®} O | | | 2,0 | 4,0 | | | | 1,0 |
| Cutina^{®} MD | | 0,5 | 3,0 | 3,0 | | | | 3,0 |
| Cetiol^{®} LC | 4,0 | | | | 3,0 | | | |
| Cosmedia^{®} DC | 0,5 | | | 1,0 | | | | 1,0 |
| Cetiol^{®} Sensoft | 4,0 | 5,0 | | 2,0 | | | 5 | 4,0 |
| Tegosoft^{®} DEC | | | | 2,0 | | 2,0 | | 2,0 |
| Cetiol^{®} CC | 2,0 | | 2,0 | | 2,0 | | | 2,0 |
| Dow Corning^{®} 1503 Fluid | | 5,0 | 8,0 | | | 5,0 | | |
| Dow Corning^{®} 245 | | 2,0 | | 2,0 | 7,0 | | 5 | |
| Eutanol^{®} G 16 | 4,0 | | | | | 3,0 | | |
| Myritol^{®} 331 | | 5,0 | | | 2,0 | 2,0 | 5,0 | |
| Uvinul^{®} T 150 | | | | 0,5 | | | | 0,5 |
| Uvasorb^{®} HEB | 2,0 | | | | | | 1,0 | 1,0 |
| Tinosorb^{®} M | | | 2,0 | | | | | 2,0 |
| Tinosorb^{®} S | | | | 3,0 | | | | 2,0 |
| Neo Heliopan^{®} AV | | | | 2,0 | | | 2,0 | |
| Heo Heliopan^{®} AP | | | | 1,0 | | | 1,0 | |
| Uvinul^{®} A Plus | | | 1 | | | | 2,0 | 2,0 |
| Microna^{®} Matte White | 5,0 | 5,0 | | 5,0 | 5,0 | 5,0 | | 5,0 |
| Microna^{®} Matte Black | 0,3 | 0,3 | 0,1 | 0,3 | 0,3 | 0,3 | 0,4 | 0,3 |
| Microna^{®} Matte Yellow | 3,0 | 3,0 | | 3,0 | 3,0 | 3,0 | 2,0 | 3,0 |
| Microna^{®} Matte Red | 0,6 | 0,6 | 1,0 | 0,6 | 0,6 | 0,6 | 1,0 | 0,6 |
| Ronasphere^{®} LDP | 1,0 | 1,0 | | 1,0 | 1,0 | 1,0 | | 1,0 |
| Pigment White 6 | | | 6,0 | | | | 6,0 | |
| Dry Flow PC | | | | | | | 2,0 | 2,0 |
| Glycerin | 5,0 | 5,0 | 3,0 | 5,0 | 5,0 | 5,0 | 3,0 | |
| Cosmedia^{®} SP | | | 0,3 | | 0,2 | | | |
| Ultragel™ 300 | | 0,4 | | | | | | 0,2 |
| Rheocare^{®} C Plus | 0,3 | | 0,1 | 0,4 | | 0,3 | | |
| Water, de-ionized, Preservative | ad 100 | | | | | | | |

**Tabelle 5: AP/Deo Konzepte**

| **Bestandteile: INCI (Handelsnamen)** | **1** | **2** | **3** | **4** | **5** | **6** | **7** |
|---|---|---|---|---|---|---|---|
| Glyceryl Stearate, Ceteareth-20, Ceteareth-12, Cetearyl Alcohol, Cetyl Palmitate (Emulgade^{®} SE) | 6 | | | 4,5 | | 6 | |
| Ceteareth-20 (Eumulgin^{®}B2) | | | | 1 | | | |
| Glyceryl Stearate Citrate (Imwitor 372 P) | | 4,0 | | | | | |
| Polyglyceryl-3 Diisostearate (Lameform^{®} TGI) | | | 3 | | | | |
| Cocoglycerides (Novata^{®}) AB) | | | | | | | 4 |
| Stearyl alcohol (Lanette^{®} 18) | | | | | 10 | | |
| Hydrogenated Castor Oil (Cutina^{®} HR) | | | | | 3,7 | | 6,5 |
| Polyglyceryl-2 Dipolyhydroxystearate (Dehymuls^{®} PGPH) | | | 1 | | | | |
| Sodium Stearoyl Glutamate (Eumulgin^{®} SG) | | 0,2 | | | | | |
| Disodium Cetearyl Sulfosuccinate (Eumulgin^{®} Prisma) | 0,3 | 0,1 | 0,05 | 0,05 | 0,2 | 0,2 | 0,1 |
| Sodium Cetearyl Sulfate (Lanette^{®} E) | | | | | | 0,3 | |
| Pentaerythrityl Distearate (Cutina^{®} PES) | 5 | 1 | 2 | 1 | 4,7 | 5 | 4 |
| Behenyl Alcohol (Lanette^{®} 22) | 2 | 1 | | | | 4 | |
| 2-Hexyldecyl-hexansäureester | 4 | 4 | 5 | 3 | 4 | 3 | 5 |
| Propylheptyl Caprylate (Cetiol^{®} Sensoft) | | 2 | | | 20 | | 10 |
| Dicaprylyl Carbonate (Cetiol^{®} CC) | | | 2 | | | | |
| Dicaprylyl Ether (Cetiol^{®} OE) | 2 | | | 2 | 5 | 3 | 4 |
| Cocoglycerides (Myritol^{®} 331) | | | | | | | |
| Diethylhexylcyclohexane (Cetiol^{®} S) | | | | 5 | 14,7 | | 25 |
| Cyclopentasiloxane | 3 | | 5 | | 14 | 3 | 14 |
| Cyclopentasiloxane and Dimethicone / Vinyldimethicone Crosspolymer SFE 839 (GE Bayer) | | | 3 | | | | |
| Dimethicone AK 350 | 1 | 2 | | | | | |
| Hydrogenated Dimer Dilinoleyl / Dimethylcarbonate Copolymer (Cosmedia^{®} DC) | 0,5 | | 1 | 1,5 | 1 | 2 | 1 |
| Triethyl Citrate (Hydagen^{®} C.A.T) | | | | 2 | | | |
| Tocopheryl Acetate | | | | | 1 | | |
| Aluminium Zirconium Tetrachlorohydrex GLY (Rezal36) | 30 | | 40 | | 22,9 | 30 | 25 |
| Aluminum Chlorhydrate (Locron® L) | | 20 | | 10 | | | |
| Chitosan (Hydagen^{®} DCMF) | 0,05 | | | | | | |
| Glycolic Acid | 0,02 | | | | | | |
| Glycerin | | | 5 | 5 | | | |
| Propylene Carbonate | | | | | | | 0,5 |
| Quaternium-18 Hectorite | | | | | | | 1 |
| Polyquaternium- 37 (Ultragel^{®} 300) | | 5 | | | | | |
| Talcum | | | | | | 5 | 5 |
| MgSO₄x 7H₂O | | | 1 | | | | |
| Water, Perfume, Preservatives | q.s. | q.s. | q.s. | q.s. | q.s. | q.s. | q.s. |

| | | | | | | | |
|---|---|---|---|---|---|---|---|
| 1/2 - Antiperspirant / Deo Creme, 3 - Antiperspirant Creme ( W/0 ), 4 - Antiperspirant / deo Spray, 5 - Antiperspirant Stift mit Vitamin E, 6 - Antiperspirant Creme, 7 - Antiperspirant Creme 'Soft Solid' | | | | | | | |

### Feuchtigkeitsspendende Körpermilch

| Phase | Komponente Handelsname | INCI | Gew.-% |
|---|---|---|---|
| | EMULGADE® CM | Cetearyl Isononanoate (and) Ceteareth-20 (and) Cetearyl Alcohol (and)Glyceryl Stearate (and) Glycerin (and) Ceteareth | 5.0 |
| | EUMULGIN® VL 75 | Lauryl Glucoside (and) Polyglyceryl-2 Dipolyhydroxystearate (and) Glycerin | 2.0 |
| | Eumulgin^{®}Prisma | Disodium Cetearyl Sulfosuccinate | 0,5 |
| | CETIOL® OE | Dicaprylyl Ether | 4.0 |
| | CETIOL® J 600 | Oleyl Erucate | 1.0 |
| | ISOPROPYLMYRISTATE | Isopropyl Myristate | 7.0 |
| II. | Wasser, deionisiert | | ad 100 |
| III. | Cosmedia SP | Sodium Polyacrylate | 0.4 |
| IV. | HISPAGEL® 200 | Glycerin (and) Glyceryl Polyacrylate | 20.0 |
| V. | Konservierungsmittel, Parfum | | q.s. |

Die Herstellung erfolgte durch Mischen von Phase I und Wasser bei Raumtemperatur unter Rühren. Dann wurde Phase III zugefügt und solange gerührt bis eine homogene, gequollene Mischung vorlag. Dann wurde Phase IV zugefügt, gefolgt von Phase V, danach wurde der pH Wert auf 5,5 eingestellt.

### O/W Soft Creme

| Phase | Komponente Handelsname | INCI | Gew.-% |
|---|---|---|---|
| I. | EMULGADE® SE-PF | Glyceryl Stearate (and) Ceteareth-20 (and) Ceteareth-12 (and) Stearyl Alcohol (and) Ceteareth-20 (and) Distearyl Ether | 6.0 |
| | Eumulgin^{®}Prisma | Disodium Cetearyl Sulfosuccinate | 0,2 |
| | LANETTE® O | Cetearyl Alcohol | 1.0 |
| | CUTINA® MD | Glyceryl Stearate | 2.0 |
| | CETIOL® MM | Myristyl Myristate | 2.0 |
| | Jojoba Oil | Simmondsia Chinensis (jojoba) Seed Oil | 2.0 |
| | Cetiol^{®} Sensoft | Propylheptyl Caprylate | 5,0 |
| | COPHEROL® 1250 | Tocopheryl Acetate | 0.5 |
| | | Dimethicone | 0.5 |
| | | Cyclomethicone | 3.0 |
| II. | Wasser | Aqua | ad 100 |
| | | Propylene Glycol | 3.0 |
| III. | HISPAGEL® 200 | Glycerin (and) Glyceryl Polyacrylate | 15.0 |
| IV. | Konservierungsmittel | | q.s. |
| | pH 5.5-6.5 | | |

Diese Creme wurde hergestellt, indem Phase I auf 80°C erhitzt wurde, Phase II wurde ebenfalls auf 80°C erhitzt und zu Phase I unter Rühren hinzugefügt. Diese Mischung wurde unter Rühren abgekühlt und bei ca. 55°C mit einem geeigneten Dispersionsgerät (z.B. Ultra Turrax) homogenisiert. Danach wurde Phase III unter kontinuierlichem Rühren hinzugefügt, Phase IV wurde zugegeben und der pH-Wert eingestellt.

### O/W-Sonnenschutzemulsionen

| **Komponente** | **1** | **2** | **3** | **4** | **5** | **6** | **7** | **8** | **9** | **10** | **11** |
|---|---|---|---|---|---|---|---|---|---|---|---|
| **L = Lotion, C = Creme,** | **L** | **C** | **S** | **L** | **C** | **L** | **L** | **C** | **L** | **C** | **L** |
| Eumulgin^{®}Prisma | 0,5 | 0,5 | 0.1 | 0,6 | 0,2 | 0,1 | 0,1 | 0.5 | 1,0 | 0,2 | 0,9 |
| Amphisol^{®} K | | | 1 | | | 1 | | | | | |
| Natriumstearat | | | | | | | 1 | | | | |
| Emulgade^{®} PL 68/50 | | | 1 | | 5 | | | | | 4 | |
| Tego^{®} Care 450 | | | | | | | | | | 3 | |
| Cutina^{®} MD | 2 | | | 6 | | | 4 | | | 6 | |
| Lanette^{®} 14 | 1 | | | 1 | | | | 2 | | | 4 |
| Lanette^{®}O | 1 | 6 | | | 5 | 2 | | 2 | | | |
| Antaron V 216 | | | 1 | | 2 | 2 | | | | 1 | |
| Lanolin, wasserfrei USP | | | | | | | 5 | | | | |
| Cetiol^{®} Sensoft | 5 | | | | 5 | | | 5 | | | |
| Myritol^{®} 331 | | | 8 | | | 6 | | 5 | | 2 | |
| Finsolv^{®} TN | | | 1 | | | | | 1 | 8 | | |
| Cetiol^{®} CC | | 2 | 5 | | | 4 | 4 | 2 | | 2 | |
| Cetiol^{®} OE | | | 3 | | | | | | 2 | 3 | |
| Dow Corning DC^{®} 244 | 4 | | 1 | | 5 | | | 2 | | | 2 |
| Dow Corning DC^{®} 2502 | | 1 | | | 2 | | | | | | |
| Silikonöl Wacker AK^{®} 350 | | 2 | | | | | | | | | |
| Cetiol^{®} 868 | | | | | 2 | | 8 | | | | 7 |
| Cetiol^{®} J 600 | | | | | 3 | 2 | | | | 5 | |
| Mineralöl | | | | 9 | | | | | | | |
| Cetiol^{®} B | | | 1 | | | | | | | 2 | |
| Eutanol^{®} G | | | | | | | | | | | |
| Eutanol^{®} G 16 | | | | | | | | | | | |
| Cetiol^{®} PGL | | 5 | | | | | | | | 5 | |
| Mandelöl | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl^{®} LS | 1 | | | | | | | | | | |
| Neo Heliopan^{®} Hydro (Na-Salz) | 2 | | 2.2 | | 3 | 3 | | | | | 2 |
| Neo Heliopan AP (Na-Salz) | 2 | | | | 1,5 | 2 | 2 | | 1 | | 1 |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} T 150 | 2 | | | | 2.5 | | | 1 | | | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex^{®} T 2000 | | | | | 5 | | 3 | 3 | | | 4 |
| Veegum^{®} Ultra | | | 0.7 | | | | | 1 | 1 | | |
| Keltrol^{®} T | | | 0.2 | | | | | 0.5 | 0.5 | | |
| Carbopol^{®} 980 | | 0.5 | | 0.2 | 0.2 | 0.2 | | 0.5 | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |
| Konservierungsmittel, NaOH | q.s. | | | | | | | | | | |
| Wasser | Ad 100 | | | | | | | | | | |

### O/W-Sonnenschutzemulsionen

| **Komponente** | **12** | **13** | **14** | **15** | **16** | **17** | **18** | **19** | **20** | **21** | **22** |
|---|---|---|---|---|---|---|---|---|---|---|---|
| **L = Lotion, C = Creme,** | **L** | **L** | **L** | **C** | **L** | **C** | **S** | **C** | **C** | **L** | **L** |
| Eumulgin^{®} VL 75 | 4 | | | | | | | | | | |
| Eumulgin^{®} B2 | | | | | | | | | | | 1 |
| Tween^{®} 60 | | | | | | | | | | 1 | |
| Cutina^{®} E 24 | | | | 2 | | | | | | | |
| Eumulgin^{®}Prisma | 0,3 | 0,5 | 0,6 | 0,1 | 0,4 | 0,2 | 0,2 | 0,1 | 0,4 | 0,6 | 0,4 |
| Emulgade^{®} PL 68/50 | | 2 | | | | 4.5 | 1 | 5 | | | |
| Tego^{®} Care 450 | 1 | | | | | | 2 | | 4 | | |
| Cutina^{®} MD | 1 | | | 8 | 6 | 1 | | | | 4 | 1 |
| Lanette^{®} 14 | | 2 | | | | | | 2 | | 1 | |
| Lanette^{®} O | | | | 2 | | | | | 1 | 1 | |
| Antaron V 220 | 1 | | | 2 | | | 0.5 | | | 2 | 0.5 |
| Myritol^{®} PC | | | | | | | | | 5 | | |
| Myritol^{®} 331 | 12 | | 12 | | | 8 | 8 | | | 10 | 4 |
| Finsolv^{®} TN | | | | | 5 | | | 3 | | | |
| Cetiol^{®} Sensoft | 6 | | | | | | 5 | | 3 | | 4 |
| Cetiol^{®} CC | | | 6 | | | 5 | | | | | |
| Cetiol^{®} OE | | | | | 2 | | | | | | 2 |
| Dow Corning DC^{®} 244 | | 2 | | | 1 | | | | | | |
| Dow Corning DC^{®} 2502 | | 1 | | | 1 | | | | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Cetiol^{®} B | 4 | | 4 | | | | | 4 | | | |
| Eutanol^{®} G | | 3 | | 5 | | 3 | | | | | |
| Eutanol^{®} G 16 S | 10 | | | | | | | | | | |
| Cetiol^{®} PGL | | | | 5 | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Neo Heliopan AP (Na-Salz) | | 2 | | 2 | | | 2 | | | | 1 |
| Eusolex^{®} OCR | 6 | | 9 | | 5 | 7 | 9 | | 4 | | 7 |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} T 150 | 1 | | | | | | | | 1.3 | 1 | 1 |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Pemulen^{®} TR 2 | | 0.3 | | 0.3 | | | 0.1 | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser ad 100, Konservierungsmittel q.s., NaOH q.s. | | | | | | | | | | | |

### O/W-Pflegeemulsionen

| **Komponente** | **67** | **68** | **69** | **70** | **71** | **72** | **73** | **74** | **75** | **76** | **77** |
|---|---|---|---|---|---|---|---|---|---|---|---|
| **L = Lotion, C = Creme** | **C** | **C** | **C** | **L** | **C** | **L** | **L** | **C** | **L** | **C** | **C** |
| Dehymuls^{®} PGPH | | 2 | | | | | | | | | |
| Generol^{®} R | | | 1 | | | | | | | | |
| Hostaphat^{®} KL 340 N | | | | | | | | | 2 | | |
| Eumulgin^{®}Prisma | 0,4 | 0,6 | 0,5 | 0,5 | 0,4 | 0,4 | 1 | 0,3 | 0,1 | 0,1 | 0,1 |
| Emulgade^{®} PL 68/50 | | 2.5 | | | | | | | | 4 | |
| Tego^{®} Care CG | | | | | | | | | | | 2 |
| Tego^{®} Care 450 | | | | | | | | 5 | | | |
| Cutina^{®} MD | | 1 | | 6 | 5 | | 4 | | | 6 | |
| Lanette^{®} 14 | | | | 1 | | | | 2 | | | 4 |
| Lanette^{®} O | 4.5 | | 4 | | 1 | 2 | | | | | 2 |
| Novata^{®} AB | | 1 | | | | | | | | | 1 |
| Lanolin, wasserfrei, USP | | | | | | | 5 | | | | |
| Cetiol^{®} SB 45 | | | 1.5 | | | | 2 | | | | |
| Myritol^{®} PC | | | | | 5 | | | | | | |
| Myritol^{®} 331 | 2 | 5 | | | | 6 | | 12 | | | |
| Finsolv^{®} TN | | | | | | 2 | | | 8 | | |
| Cetiol^{®} CC | 4 | | | | | 4 | | | | | 5 |
| Cetiol^{®} Sensoft | | 6 | | | 5 | | 4 | | 4 | | |
| Cetiol^{®} OE | | | 7 | | | | | | | 3 | |
| Dow Corning DC^{®} 245 | | | 2 | | | 1 | | | | | |
| Dow Corning DC^{®} 2502 | | | | | 2 | 1 | | | | | |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol^{®} 868 | | | | | 2 | | 4 | | | | |
| Cetiol^{®} J 600 | 2 | | 3 | | 3 | 2 | | | | 5 | |
| Ceraphyl^{®} 45 | | | | | | | 3 | | | | |
| Cetiol^{®} SN | | | 5 | | | | | | | | |
| Cetiol^{®} B | | | | | | | | | | 2 | |
| Eutanol^{®} G | | 2 | | 5 | | | | | | | |
| Cetiol^{®} PGL | | | | 7 | | | | | 5 | 5 | |
| Dry Flo ^{®} Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N,N-Diethyl-m-toluamid | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} ultra | | | | | | | | | 1 | | |
| Keltrol^{®} T | | | 0.4 | | | | | | 0.5 | | |
| Pemulen^{®} TR 2 | 0.3 | | | | | | | 0.3 | | | |
| Carbopol^{®} Ultrez 10 | | | 0.3 | 0.2 | | 0.2 | | | 0.1 | 0.3 | |
| Cosmedia SP | | 0,3 | | | 0.2 | | | | | | 0.2 |
| Aluminum Chlorohydrate | | | 7 | | | | | | | | |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel, NaOH | ad 100, q.s.,pH 6,5 - 7,5 | | | | | | | | | | |

### O/W-Pflegeemulsionen

| **Komponente** | **78** | **79** | **80** | **81** | **82** | **83** | **84** | **85** | **86** | **87** | **88** |
|---|---|---|---|---|---|---|---|---|---|---|---|
| **L = Lotion, C = Creme** | **C** | **C** | **L** | **C** | **L** | **C** | **L** | **L** | **L** | **L** | **C** |
| Eumulgin^{®} VL 75 | | 3 | | | | | 1 | | | | 2 |
| Generol^{®} R | | | | | | 2 | | | | | |
| Eumulgin^{®} B2 | | | | | | | | | | 1 | |
| Tween^{®} 60 | | | | | | | | | | 1 | |
| Cutina^{®} E 24 | | | | 2 | | | | | | | |
| Eumulgin^{®} Prisma | 0.5 | 0,2 | 0,3 | 0,1 | 1 | 0,5 | 0,2 | 1 | 0,7 | 0,5 | 1 |
| Lanette^{®} E | | | | | | | | | | | 0,1 |
| Amphisol^{®} K | | 1 | | | | | | 0,1 | | | |
| Natriumstearat | | | | | | | | | | | |
| Emulgade^{®} PL 68/50 | | 6 | | | | | | 5 | | | 4 |
| Tego^{®} Care 450 | | | | | | | | | 4 | | |
| Cutina^{®} MD | 3 | | 3 | 8 | 6 | 8 | | | | 4 | |
| Lanette^{®} 14 | | 2 | | | | | | 2 | | 1 | |
| Lanette^{®} O | 2 | | | 2 | | 3 | 1 | | 1 | 1 | 6 |
| Lanolin, wasserfrei, USP | | | | | | 4 | | | | | |
| Cetiol^{®} SB 45 | | | | | | | 2 | | | | |
| Cetiol^{®} Sensoft | 6 | | | 2 | | | | | 3 | 5 | 3 |
| Cegesoft^{®} C 17 | 4 | | | | | | | | | | |
| Myritol^{®} PC | | | | | | 5 | | | 5 | | |
| Myritol^{®} 331 | 5 | | 5 | | | | 7 | | | 5 | |
| Finsolv^{®} TN | | 5 | | | 5 | | | 3 | | | 1 |
| Cetiol^{®} CC | | | | 2 | | | | | | | 2 |
| Cetiol^{®} OE | | | | | 2 | | 2 | | 5 | | |
| Dow Corning DC^{®} 245 | | 2 | | | 1 | | | | | 8 | 2 |
| Dow Corning DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | | | | | | | | |
| Ceraphyl^{®} 45 | | | | | | | 3 | | | | |
| Cetiol^{®} SN | | | | | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Carbopol^{®} ETD 2001 | | 0.3 | | 0.3 | | 0.5 | 0.2 | 0.2 | | | |
| Pemulen^{®} TR 2 | | | 0.3 | | | 0.3 | | | | | 0.5 |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser ad 100, Konservierungsmittel q.s., NaOH (pH 6,5 - 7,5) | | | | | | | | | | | |

### Sprayformulierungen

| **Komponente** | **97** | **98** | **99** |
|---|---|---|---|
| **S Sonnenschutzspray** | **S** | **S** | **S** |
| Eumulgin® Prisma | 1 | 1 | 1 |
| Eumulgin^{®} VL 75 | | | 2 |
| Emulgade^{®} PL 68/50 | 2.5 | 1 | |
| Antaron V 220 | | 1 | 1 |
| Cetiol^{®} Sensoft | 4 | 4 | 5 |
| Myritol^{®} 331 | 3 | 3 | 3 |
| Finsolv^{®} TN | | | 8 |
| Cetiol^{®} CC | 2 | 2 | 4 |
| Cetiol^{®} OE | 2 | | |
| Photonyl^{®} LS | | 2 | 2 |
| Panthenol | | 1 | 1 |
| Bisabolol | | 0,2 | 0,2 |
| Tocopherol / Tocopherylacetat | | 1 | 1 |
| Neo Heliopan^{®} Hydro (Na-Salz) | | 3 | |
| Neo Heliopan AP (Na-Salz) | | | 1 |
| Eusolex^{®} OCR | | | 3 |
| Neo Heliopan^{®} BB | | 1 | |
| Neo Heliopan^{®} MBC | | 1 | 1 |
| Neo Heliopan^{®} AV | | 7.5 | 2 |
| Uvinul^{®} T 150 | | 1 | |
| Parsol^{®} 1789 | | 1 | |
| Z-Cote^{®} HP 1 | | 2 | 2 |
| Eusolex^{®} T 2000 | | 2 | 2 |
| Veegum^{®} Ultra | | | 1.5 |
| Laponite^{®}XLG (synthetic layered silicate) | | 1.5 | |
| Keltrol^{®} T | | | 0.5 |
| Pemulen^{®} TR 2 | 0.2 | | |
| Insect Repellent^{®} 3535 | 1 | | |
| N,N-Diethyl-m-toluamid | 1 | | |
| Ethanol | | 5 | |
| Butylenglykol | | 2 | 1 |
| Glycerin | 2 | | 3 |
| Wasser/ Konservierungsmittel/ NaOH | ad100 | | |

### Rezepturen 1 bis 13

| **Komponenten INCI (Handelsname)** | **1** | **2** | **3** | **4** | **5** | **6** | **7** | **8** | **9** | **10** | **11** | **12** | **13** |
|---|---|---|---|---|---|---|---|---|---|---|---|---|---|
| Glyceryl Stearate, Ceteareth-20, Ceteareth-12, Cetearyl Alcohol, Cetyl Palmitate (Emulgade^{®} SE) | | | | | | | | | | | | 10.7 | 5.1 |
| Ceteareth-20 (Eumulgin^{®}B2) | | | | | | | | | | | | 5.8 | 3.4 |
| Cetearyl Glucoside, Cetearyl Alcohol (Emulgade^{®} PL 68/50) | 1 | | 1 | 1 | | 2 | 2 | | 2 | | 2 | | |
| Polyglyceryl-2 Dipolyhydroxystearate, Lauryl Glucoside, Glycerin (Eumulgin^{®} VL 75) | | 1 | | | 1 | | | 3 | | 2.5 | | | |
| Disodium Cetearyl Sulfosuccinate (Eumulgin® Prisma) | 1 | 1 | 1 | 0,5 | 1 | 1 | 0,5 | 1 | 1 | 1 | 1 | 1 | 0,5 |
| Dicaprylyl Carbonate (Cetiol^{®} CC) | 5 | 5 | | | | | | 4 | | 5 | 3 | 4 | |
| Cocoglycerides(Myritol^{®}331) | 3 | 4 | | 4 | 4 | | | | 5 | | | 3 | 3 |
| Dicaprylyl Ether (Cetiol^{®} OE) | | | | | 5 | | 3 | 2 | 2 | | | | |
| Dibutyl Adipate (Cetiol^{®} B) | | | | 4 | | | | 4 | | 4 | | | |
| Hydrogenated Dimer Dilinoleyl/Dimethylcarbonate Copolymer (Cosmedia^{®} DC) | 1 | 1 | 1 | 1 | 1 | 1.5 | 1.5 | 2 | 3 | 2 | 1.5 | 2 | 2 |
| Propylheptyl Caprylate (Cetiol^{®} Sensoft) | | | 5 | | | 5 | | | | | | 5 | |
| Tocopherol | 1 | 1 | 1 | 1 | 1 | 1 | 1 | 1 | 1 | 1 | 1 | 1 | 1 |
| Zinc oxide, nanonisiert, qecoated | 5 | 5 | 5 | 5 | 5 | | 2 | | 5 | 3 | | | |
| Titaniumdioxide, nanonisiert, Gecoated | | | | 5 | 5 | | 2 | 3 | 5 | 2 | | | |
| Ethylhexyl Methoxycinnamate | 7.5 | 7.5 | 7.5 | | | | 3 | 1 | 3 | 5 | | 5 | 5 |
| Octocrylene | 9 | 9 | 9 | | | 2 | 1 | | | | 2 | | 1.5 |
| Butyl Methoxydibenzoylmethane | | | | | | 2 | 2 | | | 1 | 2 | 2 | |
| 4-Methylbenzylidene Camphor | | | | | | | 2 | | | | | | 2 |
| Ethylhexyl Triazone | | | | | | 1 | 1 | 2 | | | 1 | | |
| Diethylhexyl Butamido Triazone | | | | | | 1 | 1 | 2 | | | 1 | 2 | |
| Phenylbenzimidazole Sulfonic Acid als Na-Salz, 15% wäßrige Lsg. | | | | | | | | | | | | | 13.3 |
| Glycerin | 3 | 3 | 3 | 3 | 3 | 3 | 3 | 3 | 3 | 3 | 3 | 5 | 5 |
| Magnesium aluminium silicate (and) cellulose Gum | 0.75 | 0.75 | | 0.5 | 0.5 | | 0.5 | | | 0.35 | | | |
| Xanthan Gum | 0.25 | 0.25 | | 0.5 | 0.5 | | 0.5 | | | 0.35 | | | |
| Sodium Polyacrylate (Cosmedia^{®} SP) | | | 0.1 | | | 0.1 | 0.2 | | | | 0.1 | | |
| Acrylates/C10-30 Alkyl Acrylate Crosspolymer | | | | | | | | 0.2 | 0.1 | | | | |
| Wasser, Perfume, Preservatives | q.s. | q.s. | q.s. | q.s. | q.s. | q.s. | q.s. | q.s. | q.s. | q.s. | q.s. | q.s. | q.s. |

### Rezepturen 14 bis 26

| **Komponenten INCI (Handelsname)** | **14** | **15** | **16** | **17** | **18** | **19** | **20** | **21** | **22** | **23** | **24** | **25** | **26** |
|---|---|---|---|---|---|---|---|---|---|---|---|---|---|
| Glyceryl Stearate, Ceteareth-20, Ceteareth-12, Cetearyl Alcohol, Cetyl Palmitate (Emugade^{®} SE) | 3.7 | 3.7 | | | | | | | | | | 4.9 | 4.1 |
| Ceteareth-12 (Eumulgin^{®}B1) | 1.3 | 1.3 | | | | | | | | | | | |
| Ceteareth-20 (Eumulgin^{®}B2) | | | | | | | | | | | | 1.1 | 0.9 |
| Cetearyl Glucoside, Cetearyl Alcohol (Emulgade^{®} PL 68/50) | | | 5 | 1 | 1 | 1 | 1 | 3 | | | | | |
| Polyglyceryl-2 Dipolyhydroxystearate, Lauryl Glucoside, Glycerin (Eumulgin^{®} VL 75) | | | | | | | | | 3 | 5 | 5 | | |
| (Disodium Cetearyl Sulfosuccinate) Eumulgin® Prisma | 0,1 | 0,1 | 0,1 | 0.25 | 0.25 | 0.25 | 0.25 | 0.25 | 0,2 | 0,2 | 0,3 | 0,3 | 0,3 |
| Potassium Cetyl Phosphate | | | 0.5 | | | | | | | | | | |
| Dicaprylyl Carbonate (Cetiol^{®} CC) | 5 | | 5 | | | | | | 2.5 | 4 | 4 | 5 | 5 |
| Coco-Caprylate/Caprate (Cetiol^{®} LC) | | | | 1 | 1 | 1 | 1 | 1 | | | | | |
| Caprylic/Capric Triglyceride (Myritol^{®} 312) | | | | 1 | 1 | 1 | 1 | 1 | | | | | |
| Cocoglycerides (Myritol^{®} 331) | | | | | | | | | | 4 | 4 | | |
| Cetearyl Isononanoate (Cetiol^{®} SN) | 3 | 3 | 3.5 | | | | | | | | | | |
| Octyldodecanol (Eutanol^{®} G) | | | | | | | | | 3.5 | 2 | 2 | | |
| Propylheptyl Caprylate (Cetiol^{®} Sensoft) | | | | 1 | 1 | 1 | 1 | 1 | | | | | |
| Olus (Cegesoft^{®} PS6) | | 1.5 | 1.5 | | | | | | | | | | |
| Passiflora Incarnata (Cegesoft^{®} PFO) | 1.5 | | | | | | | | | | | | |
| Dimethicone | | | | 0.5 | 0.5 | 0.5 | 0.5 | 0.5 | | | | | |
| Dimer Distearyltricarbonate (Cosmedia^{®} DC) | 1 | | 1.5 | | | | | 1.5 | | 2,5 | 2,5 | | 0.5 |
| Triethyl Citrate (Hydagen^{®} C.A.T) | | | | | | | | | | | | 1.5 | |
| Tocopherol | | | | | | | | | 0.5 | 0.5 | 0.5 | | |
| Tocopheryl Acetate | 0.5 | 0.5 | | | | | | | | | | | |
| Ethanol | | | | | | | | | | | 5 | | |
| Aluminum Chlorhydrate (Locron L) | | | | | | | | | | | | | 40 |
| Chitosan (Hydagen^{®} DCMF) | | | | | | | | | | | | 0.1 | |
| Glycolic Acid | | | | | | | | | | | | 0.04 | |
| Glycerin | 2 | 2 | 2 | 3 | 3 | 3 | 3 | 3 | 2 | 2 | 2 | 3 | 2 |
| Potassium Hydroxyde, 20% wäßrige Lsg. | | | | | | 0.3 | 0.2 | 0.1 | 0.4 | 0.3 | 0.5 | | |
| Glycerin, Glyceryl Polyacrylate (Hispagel^{®} 50) | | | | | | | | | | 10 | | | |
| Carbomer | | | | | | | 0.1 | | 0.2 | | 0.2 | | |
| Sodium Polyacrylate (Cosmedia^{®} SP) | | | | | 0.15 | | | | | | | | |
| Acrylates/C10-30 Alkyl Acrylate Crosspolymer | | | | | | 0.15 | | 0.05 | | | | | |
| Wasser, Perfume, Preservatives | q.s | q.s. | q.s. | q.s. | q.s. | q.s. | q.s. | q.s. | q.s. | q.s. | q.s. | q.s. | |

### Rezepturen 27 bis 33 (Formulierungen für AP/Deo)

| **Komponenten INCI (Handelsname)** | **27** | **28** | **29** | **30** | **31** | **32** | **33** |
|---|---|---|---|---|---|---|---|
| Glyceryl Stearate, Ceteareth-20, Ceteareth-12, Cetearyl Alcohol, Cetyl Palmitate (Emulgade^{®} SE) | 6 | | 4,5 | | | 6 | |
| Ceteareth-20 (Eumulgin^{®}B2) | | | 1 | | | | |
| Cetearyl Isononanoate, Glyceryl Stearate, Ceteareth-20, Ceteareth-12, Cetearyl Alcohol, Cetyl Palmitate (Emulgade^{®} CM) | | | | | 20 | | |
| Polyglyceryl-3 Diisostearate Lameform TGI | | 3 | | | | | |
| Cocoglycerides (Novata^{®} AB) | | | | | | | 4 |
| Stearyl alcohol (Lanette 18) | | | | 14,7 | | | |
| Hydrogenated Castor Oil (Cutina^{®} HR) | | | | 3,7 | | | 6,5 |
| Polyglyceryl-2 Dipolyhydroxystearate, (Dehymuls^{®} PGPH) | | 1 | | | | | |
| Disodium Cetearyl Sulfosuccinate (Eumulgin^{®} Prisma) | 0,3 | 0,05 | 0,2 | 0,4 | 0,1 | 0,3 | 0,2 |
| Behenyl Alcohol (Lanette^{®} 22) | 2 | | | | | 4 | |
| Dicaprylyl Carbonate (Cetiol^{®} CC) | | 3 | | | | | |
| Dicaprylylether (Cetiol^{®} OE) | 2 | | | 4 | | 3 | 9 |
| Propylheptyl Caprylate (Cetiol^{®} Sensoft) | | 5 | | 14 | | | 10 |
| Cyclopentasiloxane | 3 | | | 30 | | 2 | 4 |
| Cyclopentasiloxane and Dimethicone/Vinyldimethicone Crosspolymer SFE 839 (GE Bayer) | | 3 | | | | | |
| Dimethicone | 1 | | | | | | |
| Dimer Distearyltricarbonate (Cosmedia^{®} DC) | 1 | 1 | 1 | 1 | 1 | 1 | 1 |
| Triethyl Citrate (Hydagen^{®} C.A.T) | | | 2 | | | | |
| PEG-40 Hydrogenated Castor Oil | | | | | 1 | | |
| Tocopheryl Acetate | | | | 1 | | | |
| Aluminium Zirconium Tetrachlorohydrex GLY (Rezal 36) | 30 | 40 | | 22,9 | | 30 | 25 |
| Aluminum Chlorhydrate (Locron L) | | | 10 | | | | |
| Chitosan (Hydagen^{®} DCMF) | 0,05 | | | | | | |
| Glycolic Acid | 0,02 | | | | | | |
| Glycerin | | 5 | 5 | | | | |
| Propylene Carbonate (Fluka) | | | | | | | 0,5 |
| Quaternium-18 Hectorite (Bentone 18) | | | | | | | 1 |
| Talc (Merck) | | | | | | 5 | 5 |
| MgSO4x 7H2O | | 1 | | | | | |
| Wasser Phase II | 46,7 | | 35 | | | | |
| Wasser, Perfume, Preservatives | q.s. | q.s. | q.s. | q.s. | q.s. | q.s. | q.s. |

| | | | | | | | |
|---|---|---|---|---|---|---|---|
| 27 - Antiperspirant / Deo Creme / 28 - Antiperspirant Creme (W/0) / 29 - Antiperspirant / Deo Spray ; 30 - Antiperspirant Stift mit Vitamin E / 31 - Deodorant Wipe - Formulierung / 32 - Antiperspirant Creme; 33 - Antiperspirant Creme «Soft Solid » | | | | | | | |

### O/W-Sonnenschutzemulsionen

| **Komponente** | **12** | **13** | **14** | **15** | **16** | **17** | **18** | **19** | **20** | **21** | **22** |
|---|---|---|---|---|---|---|---|---|---|---|---|
| **L = Lotion, C = Creme, S = Spray** | L | C | L | C | L | C | S | C | C | L | L |
| Eumulgin^{®} VL 75 | 2 | | | | 3 | | 4 | | 1 | | |
| Dehymuls^{®} PGPH | | 1 | | | | | 0,5 | | | | |
| Disodium Cetearyl Sulfosuccinate (Eumulgin® Prisma) | 0,5 | 0,6 | 0,5 | 0,7 | 0,5 | 0,5 | 0,1 | 0,2 | 0,1 | 0,5 | 0,5 |
| Eumulgin^{®} SG | | | | | | | | 0,1 | | | |
| Emulgade^{®} PL 68/50 | | 2 | 1 | | | 3 | | | | | |
| Tego^{®} Care 450 | | 2 | | | | | | | 2 | | |
| Cutina^{®} MD | | | | 2 | 1 | 3 | | | | | 1 |
| Lanette^{®} 14 | | 1 | | | | | | | | | |
| Lanette^{®} O | | | | 2 | | | | 2 | 1 | 1 | |
| Cutina^{®} PES | 1 | 1 | | 2 | | | | | | 1 | |
| Allianz^{®} OPT | 1 | | | 1 | 1 | | | 2 | | | 2 |
| Cosmedia^{®} DC | | 1.5 | 2 | | | 1.5 | 2 | | 1.5 | 1.5 | |
| Lanolin, wasserfrei, USP | | | | | | 1 | 1 | | | | |
| Propylheptyl Caprylate (Cetiol^{®} Sensoft) | 6 | 2 | 4 | 7 | 3 | 7 | 6 | 6 | 4 | 4 | 5 |
| Myritol^{®} PC | | | | | | | | | 5 | | |
| Myritol^{®} 331 | 6 | | 4 | | | 5 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Corning DC^{®} 244 | | 2 | | | 1 | | | | | | |
| Dow Corning DC^{®} 2502 | | 1 | | | 1 | | | 3 | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Cetiol^{®} B | 4 | | 4 | | | | | 4 | | | |
| Eutanol^{®} G | | 3 | | | | 3 | | | | | |
| Eutanol^{®} G 16 S | 3 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Eusolex^{®} OCR | 6 | | 9 | | 5 | 7 | 9 | | 4 | | 7 |
| Neo Heliopan^{®} AP (Na-Salz) | | | | 0.5 | | 1 | | | | | |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} A PLUS | | | | | 1 | | 2 | | | | |
| Uvinul^{®} T 150 | 1 | 1 | | | | | | | 1.3 | 1 | 1 |
| Tinosorb^{®} M | | 2 | | | 2 | | 2 | | | | |
| Tinosorb^{®} S | | 1 | | | 2 | | 2 | | | | |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Cosmedia^{®} SP | | | 0.2 | 0.3 | | | 0.1 | | | 0.2 | |
| Pemulen^{®} TR 2 | | 0.3 | | 0.3 | | | | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser ad 100 / Konservierungsmittel q.s. / NaOH q.s. | | | | | | | | | | | |

### O/W-Pflegeemulsionen

| **Komponente** | **34** | **35** | **36** | **37** | **38** | **39** | **40** | **41** | **42** | **43** | **44** |
|---|---|---|---|---|---|---|---|---|---|---|---|
| **L = Lotion, C = Creme** | C | C | L | C | L | C | L | L | L | L | C |
| Eumulgin^{®} VL 75 | | | 5 | | 4 | | | | | | 2 |
| Generol^{®} R | | | | | | 2 | | | | | |
| Eumulgin® Prisma | 0.5 | 0.5 | 0,1 | 0,5 | 1 | 0,4 | 0,5 | 0,2 | 0,1 | 1 | 1 |
| Emulgade^{®} PL 68/50 | | 2 | | 2 | | | | 3 | 4 | | |
| Tego^{®} Care 450 | | 1 | | | | | | | 1 | | |
| Cutina^{®} MD | 2 | 1 | 1 | 1 | | 5 | | | | 2 | |
| Lanette^{®} 14 | | | | | 1 | | | 2 | | 1 | |
| Lanette^{®} O | 2 | | | 2 | 1 | 3 | 1 | | 1 | 1 | 3 |
| Cutina^{®} PES | 1 | 2 | | 3 | 1 | | | | | | 3 |
| Novata^{®} AB | | | | | | | | | 1 | 1 | |
| Lanolin, wasserfrei, USP | | | | | | 4 | | | | | |
| Cosmedia^{®} DC | | | 2 | | | 1.5 | | | 1 | 1 | |
| Cetiol^{®} SB 45 | | | | | | | 2 | | | | |
| Cegesoft^{®} C 17 | 2 | | | | | | | | | | |
| Cetiol^{®} Sensoft | 5 | 5 | 4 | 4 | 3 | 4 | 5 | 4 | 5 | 10 | 2 |
| Myritol^{®} PC | 6 | | | | | 5 | | | | | |
| Myritol^{®} 331 | 2 | | 5 | | | | 2 | | | | 3 |
| Finsolv^{®} TN | | | | 3 | 5 | | | 3 | 3 | | 1 |
| Cetiol^{®} CC | | | | 3 | | | 4 | 3 | | | |
| Cetiol^{®} OE | | | | | 2 | | 2 | | 5 | | |
| Dow Corninq DC^{®} 245 | | 2 | | | 1 | 4 | | | | 8 | 2 |
| Dow Corning DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | 2 | | | | | | | |
| Ceraphyl^{®} 45 | | | | | | | 3 | | | | |
| Cetiol^{®} SN | | | | 5 | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Cosmedia^{®} SP | 0.5 | | | | | 0.5 | 0.5 | 0.2 | | | 0.5 |
| Carbopol^{®} ETD 2001 | | 0.3 | | 0.3 | | | | | | | |
| Pemulen^{®} TR 2 | | | 0.3 | | | 0.3 | | | | | |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. | | | | | | | | | | |
| | (pH 6,5 - 7,5) | | | | | | | | | | |

| **Komponente** | **1** | **2** | **3** | **4** | **5** | **6** | **7** |
|---|---|---|---|---|---|---|---|
| Ethanol | | 4,00 | | 3,00 | | 4,50 | |
| Wasser dem. (add to) | 100 | 100 | 100 | 100 | 100 | 100 | 100 |
| Phenonip | 1,00 | 1,00 | 1,00 | 1,00 | 1,00 | 1.00 | 1,00 |
| Glycerin 99% | 3,00 | 3,00 | 3,00 | 3,00 | 3,00 | 3,00 | 3,00 |
| Ultragel^{®} 300 (Polyquaternium-37) | 0,8 | 1,00 | 1,50 | 0,90 | 2,00 | 1,50 | 0,75 |
| Eumulgin^{®} VL 75 (Lauryl Glucoside (and) Polyglyceryl-2 Dipolyhydroxystearate (and) Glycerin) | 1,80 | - | 0,80 | - | - | - | - |
| Emulgade^{®} PL 68/50 (Cetearyl Glucoside (and Cetearyl Alcohol) | - | - | - | - | - | 3,00 | - |
| Eumulgin® Prisma (Disodium Cetearyl Sulfosuccinate) | 0,10 | 0,12 | 0,30 | 0,10 | 0,30 | 0,10 | 0,08 |
| Cutina^{®} PES (Pentaerythrityl Distearate) | 2,50 | 3,5 0 | 1,70 | 4,00 | 2,00 | 4,00 | 5,00 |
| Cetiol^{®} J600 (Oleyl Erucate) | 2,00 | - | 5,00 | - | - | 1,00 | 3,00 |
| Cetiol^{®} CC (Dicaprylyl Carbonate) | 3,50 | - | 4,00 | 4,00 | 2,50 | 3,00 | 4,00 |
| Cetiol^{®} PGL (Hexyldecanol (and) Hexyldecyl Laurate) | - | 2,00 | - | 4,00 | 5, 00 | 5,00 | 1,00 |
| Tegosoft^{®} DEC (Diethylhexyl Carbonate) | - | - | - | 2,50 | - | - | 3,00 |
| DC^{®} 345 (Cyclopentasiloxane) | 4,00 | - | - | - | 2,00 | - | - |
| Cetiol^{®} B (Dibutyl Adipate) | 5,00 | 3,5 | 5,00 | | | 2,00 | |
| Myritol^{®} 331 (Cocoglycerides) | 3,00 | - | - | - | - | 4,00 | 4,50 |
| Cetiol^{®} Sensoft (Propylheptyl Caprylate) | 3,00 | 3,00 | 5,00 | 2,00 | 7,00 | 5,00 | 3,00 |
| DHA (Dihydroxyaceton) | 2,00 | 1,00 | 2,50 | 1,00 | 2,00 | 2,00 | 3,50 |
| Uvinul^{®} T 150 (Ethylhexyl Triazone) | - | | 1,00 | - | - | - | - |
| Tinosorb^{®} M (Methylene Bis-Benzotriazoyl Tetramethylbutylphenol) | - | 1,00 | 1,00 | - | - | - | - |
| Tinosorb^{®} S (Bis-Ethylhexyloxyphenol Methoxyphenyl Triazine) | - | 2,00 | - | - | 0,50 | - | - |
| Uvasorb^{®} HEB (Diethylhexyl Butamino Triazone) | - | - | - | - | 1,00 | - | - |
| Neo Heliopan^{®} AV (Ethylhexyl Methoxycinnamate | - | 5.00 | 5,00 | - | 3,00 | - | - |
| Parsol^{®} 1789 (Butyl Methoxydibenzoylmethane) | - | 3.00 | 3,00 | - | 2,00 | - | - |
| Neo Heliopan^{®} 303 (Octocrylene) | - | 3,50 | 2,00 | - | - | - | - |

### Sonnenschutz Spray

| Phase | Bestandteil | INCI | Gew.-% |
|---|---|---|---|
| I | Deionized Water | Aqua | qs 100 |
| | Butylene Glycol | Butylene Glycol | 3.00 |
| II | Veegum Ultra | Magnesium Aluminum Silicate | 0.50 |
| | Keltrol T | Xanthan Gum | 0.30 |
| III | Eumulgin ® VL 75 | Lauryl Glucoside (and) Polyglyceryl-2-Dipolyhydroxystearate (and) Glycerin | 6.00 |
| | Eumulgin® Prisma | Disodium Cetearyl Sulfosuccinate | 0,2 |
| | Cetiol® CC | Dicaprylyl Carbonate | 3,50 |
| | Cetiol® Sensoft | Propylheptyl Caprylate | 3,50 |
| | Cetiol® AB | C12-15 Alkyl Benzoate | 7.00 |
| | Cetiol® Sun | Titanium Dioxide Dispersion | 10.00 |
| | Preservative | | q.s. |
| | Parfum | | q.s. |
| Viskosität: Brookfield, RVT, 20°C, Sp 4, 5 rpm 3500 mPas pH= 6.5 | | | |

### Sonnenlotion

| | Bestandteil | INCI | Gew.-% |
|---|---|---|---|
| I | Myritol® 331 | Cocoglycerides | 2.00 |
| | Cetiol® SB 45 | Shea Butter | 1.00 |
| | Cosmedia Gel CC | Dicaprylyl Carbonate (and) Stearalkonium Hectorite | 2.00 |
| | Uvasorb® HEB | Dioctyl Butamido Triazone | 3.00 |
| | Cetiol® Sensoft | Propylheptyl Caprylate | 3,50 |
| | Neo Heliopan® AV | Ethylhexyl Methoxycinnamate | 4.00 |
| | Neo Heliopan® E1000 | Isoamyl p-Methoxycinnamate | 4.00 |
| | Neo Heliopan® 303 | Octocrylene | 3.00 |
| | Copherol® 1250 C | Tocopheryl Acetate | 0.25 |
| | Cosmedia® DC | Hydrogenated Dimer Dilinoleyl/Dimethylcarbonate Copolymer | 2.00 |
| II | Cetiol® Sun | Titanium Dioxide Dispersion | 6.00 |
| | Eumulgin® Prisma | Disodium Cetearyl Sulfosuccinate | 0,15 |
| | Satiaxane CX 91 | Xanthan qum | 0,30 |
| | Eumulgin® VL75 | Lauryl Glucoside (and) Polyglyceryl-2-Dipolyhydroxystearate (and) Glycerin | 3.0 |
| III | Deionized Water | Water | 58.22 |
| | 2 NaEDTA | Disodium EDTA | 0.10 |
| | Liponic EG-1 | Glycereth-26 | 3.00 |
| IV | Tinosorb M | Methylene bis-benzotriazolyltetramethylbutylphenol | 3.00 |
| | Water | Water | 3.00 |
| | Preservative | | q.s. |
| | Parfum | | q.s |
| Viscosität Brook. RVT, 20°C, spindle 5, 10 rpm 2560 mPaspH= 6.70 | | | |

### Sonnenlotion

| | Bestandteil | INCI | Gew.-% |
|---|---|---|---|
| I | Cetiol® B | Bibutyl Adipate | 2.00 |
| | Cosmedia® DC | Hydrogenated Dimer Dilinoleyl/Dimethylcarbonate Copolymer | 1.00 |
| | Cetiol® Sensoft | Propylheptyl Caprylate | 2.00 |
| II | Neo Heliopan® OS | Ethylhexyl Salicylate | 3.00 |
| | Tinosorb® S | Bis-ethylhexyloxyphenol Methoxyphenyl Triazine | 2.50 |
| | Neo Heliopan® AV | Ethylhexyl Methoxycinnamate | 6.00 |
| | Neo Heliopan® 303 | Octocrylene | 8.00 |
| | Neo Heliopan® 357 | Butyl Methoxydibenzoylmethane | 2.50 |
| III | Cosmedia® Gel CC | Dicaprylyl Carbonate (and) Steralkonium Hectorite (and) Propylene Carbonate | 2.00 |
| IV | Eumulgin® Prisma | Disodium Cetearyl Sulfosuccinate | 050 |
| | Eumulgin ® VL 75 | Lauryl Glucoside (and) Polyglyceryl-2-Dipolyhydroxystearate (and) Glycerin | 3.00 |
| V | Cetiol® Sun | Titanium Dioxide Dispersion | 10.00 |
| VI | Deionized Water | Aqua | qs 100 |
| | 2 NaEDTA | Disodium EDTA | 0.10 |
| | Preservative | | q.s. |
| | Liponic EG-1 | Glycereth-26 | 3.00 |
| | Keltrol® T | Xanthan gum | 0.60 |
| | Veegum Ultra | Magnesium Aluminum Silicate | 2.00 |
| VII | Dry Flo Plus | Aluminum Starch Octenylsuccinate | 2.00 |
| VIII | Tinosorb® M | Methylene Bis-Benzotriazolyl Tetramethylbutylphenol | 5.00 |
| IX | Parfum | | q.s. |
| Viskosität Brookfield Helipath 20°C, TB, 10 rpm 16200 mPas pH= 6.7 | | | |

### Anti-Ageing Sonnenschutz

| | Bestandteil | INCI | Gew.-% |
|---|---|---|---|
| I | Myritol® 331 | Cocoglycerides | 2.00 |
| | Cetiol® B | Bibutyl Adipate | 3.00 |
| | Cetiol® Sensoft | Propylheptyl Caprylate | 2.00 |
| | Uvinul A plus | Diethylamino Hydroxybenzoyl Hexyl Benzoate | 5.00 |
| | Uvinul T 150 | Ethylhexyl Triazone | 2.50 |
| | Cosmedia® Gel CC | Dicaprylyl Carbonate (and) Stearalkonium Hectorite | 2.00 |
| | Cosmedia® DC | Hydrogenated Dimer Dilinoleyl/Dimethylcarbonate Copolymer | 2.00 |
| II | Cetiol® Sun | Titanium Dioxide Dispersion | 10.00 |
| | Satiaxane CX 91 | Xanthan qum | 0.40 |
| | Eumulgin® Prisma | Disodium Cetearyl Sulfosuccinate | 0,0 |
| III | Deionized Water | Aqua | 49.60 |
| | 2NaEDTA | Disodium EDTA | 0.10 |
| | Glycerin | Glycerin | 3.00 |
| | Preservative | | q.s. |
| IV | Photonyl® LS | Arginine (and) Disodium Adenosine Triphosphate (and) Mannitol (and) Pyridoxine HCl (and) RNA (and) Histidine HCl (and) Phenylalanine (and) Tyrosine | 2.00 |
| | Water | | 12.00 |
| V | Copherol® 1250 C | Tocopheryl Acetate | 0.50 |
| | Parfum | | q.s. |
| Viskosität: Brook. RVT, 20°C, Spindel 5, 10 rpm 3560 mPaspH= 6.50 | | | |

### Anti-Falten Creme

| | Bestandteil | INCI | Gew.-% |
|---|---|---|---|
| I | Eumulgin ® BA 25 | Beheneth-25 | 3.30 |
| | Lameform® TGI | Polyglyceryl-3 Diisostearate | 0.70 |
| | Eumulgin® Prisma | Disodium Cetearyl Sulfosuccinate | 0,1 |
| | Cetiol® Sensoft | Propylheptyl Caprylate | 2.00 |
| | Cutina ® PES | Pentaerythrityl Distearate | 2.00 |
| | Cetiol ®CC | Dicaprylyl Carbonate | 1.30 |
| | Cetiol ® AB | C12-C15 Alkyl Benzoate | 5.00 |
| | Cetiol ® LC | Coco-Caprylate/Caprate | 3.00 |
| | Cetiol® Sun | Titanium Dioxide Dispersion | 6.00 |
| | Phenonip | Phenoxyethanol (and) parabens | 0.80 |
| II | Deionized Water | Aqua | 61.85 |
| | 4NaEDTA | Tetrasodium EDTA | 0.05 |
| | Glycerin | Glycerin | 3.00 |
| | Butylene Glycol | Butylene Glycol | 2.00 |
| III | Veegum Ultra | Magnessium Aluminum Silicate | 3.00 |
| | Keltrol T | Xanthan Gum | 0.30 |
| IV | | Perfume Waterfresh | 0.50 |
| | Coviox ® T70 C | Tocopherol | 0.10 |
| V | Deionized Water | Aqua | 1.00 |
| | Plantactiv ® Aesculus | Escin | 0.10 |
| Viskosität, Brookfield, RVT, 20°C, Spindel 5, 5 rpm 11500 mPas pH= 6.2-6.7 | | | |

### Sonnenschutzlotion

| | Bestandteil | INCI | Gew.-% |
|---|---|---|---|
| I | Myritol® 331 | Cocoglycerides | 2.00 |
| | Cetiol® CC | Dicaprylyl Carbonate | 1.00 |
| | Cetiol® Sensoft | Propylheptyl Caprylate | 300 |
| | Cetiol® B | Bibutyl Adipate | 2.00 |
| | Neo Heliopan® MBC | 4-Methylbenzilidene Camphor | 3.50 |
| | Neo Heliopan® AV | Ethylhexyl Methoxycinnamate | 6.00 |
| | Neo Heliopan® E1000 | Isoamyl p-Methoxycinnamate | 6.00 |
| | Neo Heliopan® 357 | Butyl Methoxydibenzoylmethane | 1.00 |
| | Cosmedia ® DC | Hydrogenated Dimer Dilinoleyl/Dimethylcarbonate Copolymer | 1.00 |
| II | Cetiol® Sun | Titanium Dioxide Dispersion | 14.00 |
| | Satiaxane CX 91 | Xanthan gum | 0.10 |
| | Cosmedia ® SP | Sodium Polyacrylate | 0.40 |
| | Eumulgin® Prisma | Disodium Cetearyl Sulfosuccinate | 0,75 |
| III | Deionized Water | Aqua | qs 100.00 |
| | Neo Heliopan® Hydro | Phenylbenzimidazole Sulfonic acid | 2.00 |
| | Trometamine | Trometamine | 1.60 |
| | Phenonip | | qs |
| Viscosität: Brookfield, RVT, 20°C, Spindel 5, 10 rpm 2240 mPas pH= 7.35 | | | |

### Sonnencreme

| | Bestandteil | INCI | Gew.-% |
|---|---|---|---|
| I | Myritol® 331 | Cocoglycerides | 2.00 |
| | Cetiol® CC | Dicaprylyl Carbonate | 1.00 |
| | Cetiol® Sensoft | Propylheptyl Caprylate | 1.00 |
| | Cetiol® B | Bibutyl Adipate | 2.00 |
| | Neo Heliopan® MBC | 4-Methylbenzilidene Camphor | 3.50 |
| | Neo Heliopan® AV | Ethylhexyl Methoxycinnamate | 6.00 |
| | Neo Heliopan® E1000 | Isoamyl p-Methoxycinnamate | 6.00 |
| | Neo Heliopan® 357 | Butyl Methoxydibenzoylmethane | 1.00 |
| | Cosmedia® DC | Hydrogenated Dimer Dilinoleyl/Dimethylcarbonate Copolymer | 2.00 |
| | Emulgade® PL 6850 | Cetearyl Glucoside (and) Cetearyl Alcohol | 4.50 |
| | Eumulgin® Prisma | Disodium Cetearyl Sulfosuccinate | 1.0 |
| II | Cetiol® Sun | Titanium Dioxide Dispersion | 14.00 |
| III | Deionized Water | Aqua | qs 100.00 |
| | Neo Heliopan® Hydro | Phenylbenzimidazole Sulfonic Acid | 2.00 |
| | NaOH 50% | Sodium Hydroxide | 0.6 |
| | | Preservative | q.s. |
| | | Parfum | q.s. |
| Viskosität: Brookfield, Helipath 20°C, TE, 4 rpm, 195000 mPas pH= 7.5 | | | |

### Sonnenschutz Spray (Gew.-%)

| | Ingredient | INCI | A | B |
|---|---|---|---|---|
| I | Eumulgin ® VL 75 | Lauryl Glucoside (and) Polyglyceryl-2 Dipolyhydroxystearate (and) Glycerin | 6.0 | 6.0 |
| | Generol ® R | Brassica Campestris (Rapeseed) | 1.0 | 1.0 |
| | Cegesoft ® SH | Sterols Shorea Stenoptera | 1.0 | 1.0 |
| | Cosmedia ® Gel CC | Dicaprylyl Carbonate(and) Stearalkonium Hectorite (and) Propylene Carbonate | 2.0 | |
| | Eumulgin® Prisma | Disodium Cetearyl Sulfosuccinate | 0,5 | 0,5 |
| | Eusolex 2292 | Ethylhexylmethoxycinnamate | 7.5 | 7.5 |
| | Tinosorb® S | Bis-ethylhexyloxyphenol methoxyphenyltriazine | 2.0 | 2.0 |
| | Cetiol ® CC | Dicaprylyl carbonate | 1.5 | |
| | Cetiol® Sensoft | Propylheptyl Caprylate | 1.5 | 1.5 |
| | Cetiol ® AB | C12-15 Alkyl Benzoate | 4.0 | 4.0 |
| | Cosmedia ® DC | Hydrogenated Dimer Dilinoleyl / Dimethylcarbonate Copolymer | 1.00 | 1.00 |
| II | Water | | 61.45 | 63.45 |
| | EDTA 4Na (Prolabo) | Tetrasodium EDTA | 0,05 | 0.05 |
| | Elestab 50J | Chlorphenesin & Methylparaben | 0.60 | |
| III | Cosmedia®SP | Sodium Polyacrylate | 0.40 | 0.40 |
| IV | Tinsorb® M | Methylenebis benzotriazolyl tetramethylbutylphenol | 10.0 | 10.0 |
| Viskosität, Brookfield. RVT, 23° C, spindle 2,5 rpm, ph:6 3500mPas 700mPas | | | | |

### Spray

| | Bestandteil | INCI | A | B |
|---|---|---|---|---|
| I | Eumulgin® Pisma | Disodium Cetearyl Sulfosuccinate | 0,6 | 0,6 |
| | Generol® | Brassica Campestris (Rapeseed) Sterols | 1.0 | 1.0 |
| | Cegesoft® SH | Shorea Stenoptera | 1.0 | 1.0 |
| | Cosmedia® Gel | Dicaprylyl Carbonate(and) Stearalkonium | 3.0 | - |
| | CC | Hectorite (and) Propylene Carbonate | | |
| | Cetiol® Sensoft | Propylheptyl Caprylate | 2.0 | 2.0 |
| | Eusolex 2292 | Ethylhexylmethoxycinnamate | 7.5 | 7.5 |
| | Tinosorb S | Bis-ethylhexyloxyphenol methoxyphenyltriazine | 2.13 | 4.74 |
| | Cetiol® CC | Dicaprylyl Carbonate | 2.0 | 2.0 |
| | Cetiol® AB | C12-15 Alkyl Benzoate | 4.0 | 4.0 |
| II | Water | | 62.32 | 62.71 |
| | EDTA 4Na | Tetrasodium EDTA | 0.05 | 0.05 |
| | Elestab 50J | Chlorphenesin & Methylparaben | 0.60 | 0.60 |
| III | Cosmedia® SP | Sodium Polyacrylate | 0.40 | 0.40 |
| IV | Tinosorb M | Methylenebis benzotriazolyl tetramethylbutylphenol | 10.00 | 10.00 |
| Viskosität Book. RVT, 23° C, spindle 2,5 rpm, ph:6 A: 4020 mPas B: 730 mPas | | | | |

### Spray (Angaben in Gew.-%)

| | Bestandteil | INCI | A | B |
|---|---|---|---|---|
| I | Eumulgin® VL 75 | Lauryl Glucoside (and) Polyglyceryl-2 Dipolyhydroxystearate (and) Glycerin | 6.0 | 6.0 |
| | Eumulgin® Prisma | Disodium Cetearyl Sulfosuccinate | 0,2 | 0,2 |
| | Generol® R | Brassica Campestris (Rapeseed) Sterols | 1.0 | 1.0 |
| | Cegesoft® SH | Shorea Stenoptera | 1.0 | 1.0 |
| | Cosmedia® Gel CC | Dicaprylyl Carbonate(and) Stearalkonium Hectorite (and) Propylene Carbonate | 2.0 | |
| | Eusolex 2292 | Ethylhexylmethoxycinnamate | 7.5 | 7.5 |
| | Tinosorb S | Bis-ethylhexyloxyphenol methoxyphenyltriazine | 2.0 | 2.0 |
| | Cetiol® CC | Dicaprylyl carbonate | 1.5 | 1.5 |
| | Cetiol® Sensoft | Propylheptyl Caprylate | 1.5 | 1.5 |
| | Cetiol® AB | C12-15 Alkyl Benzoate | 4.0 | 4.0 |
| | Cosmedia ® DC | Hydrogenated Dimer Dilinoleyl/Dimethylcarbonate Copolymer | 1.0 | 1.00 |
| II | Water | | 61.45 | 63.45 |
| | EDTA 4Na | Tetrasodium EDTA | 0.05 | 0.05 |
| | Elestab 50J | Chlorphenesin & Methylparaben | 0.60 | 0.60 |
| III | Cosmedia® SP | Sodium Polyacrylate | 0.40 | 0.40 |
| IV | Tinosorb M | Methylenebis benzotriazolyl tetramethylbutylphenol | 10.0 | 10.0 |
| Viskosität, Brook. RVT, 23° C, spindle 2, 5 rpm, pH: 6 [mPas] A: 3500, B: 700 | | | | |

### Mat finish fluid foundation

| | Bestandteil | INCI | Gew.-% |
|---|---|---|---|
| I | Lameform® TGI | Polyglyceryl-3 Diisostearate | 1.50 |
| | Eumulgin® BA25 | Beheneth-25 | 3.50 |
| | Eumulgin® Prisma | Disodium Cetearyl Sulfosuccinate | 0,05 |
| | Cetiol® CC | Dicaprylyl Carbonate | 4.00 |
| | Myritol® 331 | Cocoglycerides | 2.00 |
| | Cetiol® Sensoft | Propylheptyl Caprylate | 2.00 |
| | Fitoderm® | Squalane | 3.00 |
| | Cutina® PES | Pentaerythiryl Distearate | 1.00 |
| | Cegesoft® SH | Shorea Stenoptera | 1.00 |
| | Phenonip | Phenoxyethanol (and) Parabens | 0.80 |
| | KF 96, 100cs | Dimethicone | 3.00 |
| | Cetiol® OE | Dicaprylyl Ether | 3.00 |
| II | Sun Croma C47-051 | Titanium Dioxide | 6.00 |
| | SunPuro Yellow Iron Oxide C33-9001 | Iron Oxide Cl 77492 | 1.30 |
| | SunPuro Red Iron Oxide C33-8001 | Iron Oxide Cl 77491 | 0.60 |
| | SunPuro Black Iron Oxide C33-7001 | Iron Oxide Cl 77499 | 0.20 |
| III | Deionized Water | Aqua | 52.50 |
| | Glycerine | Glycerin | 3.00 |
| | Butylene Glycol | Butylene Glycol | 2.00 |
| IV | Veequm Ultra | Magnesium Aluminium Silicate | 1.00 |
| | Keltrol T | Xanthan Gum | 0.40 |
| V | Cosmedia® Gel CC | Dicaprylyl Carbonate (and) Stearalkonium Hectorite (and) Propylene Carbonate | 3.00 |
| VI | Cosmedia® PMMA V12 | Polymethylmethacrylate | 2.00 |
| VII | Mirasil CDPDM | Cyclomethicone (and) Diphenyldimethicone | 3.00 |
| VIII | Perfume Caresse | | 0.20 |
| Viskosiät, Brook. RVT, spindle 3, speed 5, 8400mPas pH:6.7 | | | |

### Ultra protective spray

| | Bestandteil | INCI | Gew.-% |
|---|---|---|---|
| I | Eumulgin® VL 75 | Lauryl Glucoside (and) Polyglyceryl-2 Dipolyhydroxystearate (and) Glycerin | 7.00 |
| | Eumulgin® Prisma | Disodium Cetearyl Sulfosuccinate | 0,5 |
| | Generol® R | Brassica Campestris (Rapeseed) Sterols | 1.00 |
| | Cosmedia® DC | Hydrogenated Dimer Dilinoleyl / Dimethylcarbonate Copolymer | 1.00 |
| | Eusolex 2292 | Ethylhexylmethoxycinnamate | 7.50 |
| | Tinosorb S | Bis-ethylhexyloxyphenol methoxyphenyltriazine | 2.00 |
| | Cetiol® CC | Dicaprylyl carbonate | 2.00 |
| | Cetiol® AB | C12-15 Alkyl Benzoate | 4.00 |
| | Cetiol® Sensoft | Propylheptyl Caprylate | 2.00 |
| | Cegesoft® VP | Olus & Hydrogenated Vegetable Oil & Candelilla Cera | 2.50 |
| | Myritol® 331 | Cocoglycerides | 2.00 |
| | Cosmedia® Gel CC | Sodium polyacrylate Carbonate(and) Stearalkonium Hectorite (and) Propylene Carbonate | 4.00 |
| II | Water | qsp100 | 55.15 |
| | EDTA 4Na | Tetrasodium EDTA 0.05 | 0.05 |
| | Elestab 50J | Chlorphenesin & Methylparaben | 0.60 |
| III | Keltrol T | Xanthan Gum | 0.20 |
| IV | Tinosorb M | Methylenebis benzotriazolyl tetramethylbutylphenol | 10.00 |
| Viscosity, Brook. RVT, 23° C, spindle 2,5 rpm, 2500mPas pH:6.4 | | | |

### Soft moisture Gel Creme

| | Bestandteil | INCI | Gew.-% |
|---|---|---|---|
| I | Deionized Water | Aqua | 65.35 |
| | Eumulgin® Prisma | Disodium Cetearyl Sulfosuccinate | 0,2 |
| | Glycerine | Glycerin | 3.00 |
| | Phenonip | Phenoxyethanol (and) Parabens | 0.60 |
| II | Cosmedia® SP | Sodium Polyacrylate | 1.20 |
| III | Cegesoft® PFO | Passiflora Incarnata | 2.00 |
| | Eusolex® 2292 | Ethylhexyl Methoxycinnamate | 2.00 |
| | KF 96,100cs | Dimethicone 2.00 | 2.00 |
| IV | Cetiol® CC | Dicaprylyl Carbonate | 2.50 |
| | Eutanol® G | Octyldodecanol | 4.0 |
| | Cetiol® Sensoft | Propylheptyl Caprylate | 2.50 |
| | Eusolex® 4360 | Benzophenone-3 | 0.10 |
| | Cegesoft® SBE | Butyrospermum Parkii | 1.50 |
| V | Cosmedia® Gel CC | Dicaprylyl Carbonate (and) Stearalkonium Hectorite (and) Propylene Carbonate | 5.00 |
| VI | Deionized Water | Aqua | 5.00 |
| | Osmhydran® LS 8453 | Mannitol (and) Arginine (and) Serine (and) Sucrose (and) PCA (and) Citrulline (and) Glycogen (and) Histidine HCl (and) Alanine (and) Threonine (and) Glutamic Acid (and) Lysine HCl | 2.00 |
| VII | Vegeseryl® LP LS 9058 | Hydrolyzed Soy Protein | 1.00 |
| VIII | Coviox® T70C | Tocopherol | 0.10 |
| | Perfume Raphaelle | | 0.15 |
| Viscosity, Brook. RVT, Helipath TE, speed 4, 101 000mP pH:5.5 | | | |

### Samtige Reinigungsmaske

| | Bestandteil | INCI | Gew.-% |
|---|---|---|---|
| I | Emulgade® SE-PF | Glyceryl Stearate (and) Ceteareth-20 (and) Ceteareth-12 (and) Cetearyl Alcohol (and) Cetyl Palmitate | 8.00 |
| | Eumulgin® Prisma | Disodium Cetearyl Sulfosuccinate | 1 |
| | Lanette® O | Cetearyl Alcohol | 2.00 |
| | Cegesoft® PS6 | Olus | 5.00 |
| | Cetiol® CC | Dicaprylyl Carbonate | 2.00 |
| | Cutina® PES | Pentaerythrityl Distearate | 3.00 |
| | Cetiol® Sensoft | Propylheptyl Caprylate | 2.00 |
| II | Cosmedia® Gel CC | Dicaprylyl Carbonate (and) Stearalkonium Hectorite (and) Propylene Carbonate | 5.00 |
| III | Deionized Water | Aqua | 45.00 |
| | Glycerine | Glycerin | 10.00 |
| | EDTA, 4Na | Tetrasodium EDTA | 0.10 |
| | Elestab® 50J | Chlorphenesin (and) Methylparaben | 0.40 |
| IV | Dry Flo AF | Corn Starch Modified 0 | 1.00 |
| | Propylene Glycol | Propylene Glycol | 3.00 |
| V | Kaolin | Kaolin | 10.00 |
| VI | Perfume Concombre | | 0.20 |
| | Coviox® T70C | Tocopherol | 0.10 |
| VII | Purisoft ® LS 9602 | Glycerin (and) Moringa Pterygosperma | 2.00 |
| VIII | Green Covasol W7035 (a.s.0.1%) | Cl 42090 (and) Cl19140 | 3.20 |
| Viscosity, Brook. RVT, Helipath TE, Speed 4, 254 000mPas pH:6.3 | | | |

### Luxuriöse Körper Butter

| | Bestandteil | INCI | Gew.-% |
|---|---|---|---|
| I | Deionized water | Aqua | 61.50 |
| | Glycerine | Glycerin | 5.00 |
| | Butylene Glycol | Butylene Glycol | 2.00 |
| | EDTA, 4 Na | Tetrasodium EDTA | 0.10 |
| | Phenonip | Phenoxyethanol (and) Parabens | 0.80 |
| II | Cosmedia® SP | Sodium Polyacrylate | 1.50 |
| III | Eumulgin® BA 25 | Beheneth-25 | 3.00 |
| | Lanette® O | Cetearyl Alcohol | 4.00 |
| | Eumulgin® Prisma | Disodium Cetearyl Sulfosuccinate | 0,6 |
| | Cetiol® SN | Cetearyl Isononanoate | 3.00 |
| | Cetiol® SB45 | Butyrospermum Parkii | 5.00 |
| | Cetiol® CC | Dicaprylyl Carbonate | 0.50 |
| | Cetiol® Sensoft | Propylheptyl Caprylate | 0.50 |
| | KF 96, 100cs | Dimethicone | 1.00 |
| | Generol® R | Brassica Campestris (Rapeseed) Sterols | 0.50 |
| IV | Cosmedia® Gel CC | Dicaprylyl Carbonate (and) Stearalkonium Hectorite (and) Propylene Carbonate | 5.00 |
| V | Cosmedia®PMMAV12 | Polymethylmethacrylate | 2.00 |
| | Deionized water | Aqua | 4.00 |
| VI | Coviox® T70C | Tocopherol | 0.10 |
| | Perfume Pétale 139012E | | 0.50 |
| Viscosity, Brook. RVT Helipath TE, speed4, 800 000mPas pH:7 | | | |

### Makeup foundation

| Komponente INCI Bezeichung (Handelsname, Hersteller) | Gew.-% |
|---|---|
| Wasser | 78,2 |
| Glycerin | 1,89 |
| Triethanolamine 99% | 0,75 |
| Methylparaben (Nipagin M. Nipa) | 0,19 |
| Xanthan gum (Keltrol SP, CP Kelco) | 0,28 |
| Titanium dioxid (Softex Titanium dioxide, C47-7756, Sun Chemical) | 8,75 |
| Brown iron oxid (SunChroma Brown iron oxide, C33-315 Sun Chroma, Sun Chemical) | 1,08 |
| Red iron oxide (SunChroma red iron oxide, C33-124 Sun Chroma, Sun Chemical) | 0,17 |
| Phenyl trimethicone (DC 556 cosmetic Fluid, Dow Corning) | 3,41 |
| Cetiol® Sensoft (Propylheptyl Caprylate) | 1 |
| Stearic acid, 94% | 1,33 |
| Eumulgin® Prisma (Disodium Cetearyl Sulfosuccinate) | 0,5 |
| Cetyl stearyl alcohol (Lanette O, Cognis) | 2,84 |
| Propylparaben (Nipasol, M. Nipa) | 0,1 |

### O/W Flüssig Foundation

| | Inhaltsstoff | INCI Bezeichnung | Gew.-% |
|---|---|---|---|
| I | Deionized Water | Aqua | 50.50 |
| | Butylene Glycol | Butylene Glycol | 2.00 |
| | Glycerine | Glycerin | 3.00 |
| II | Veegum Ultra | Magnesium Aluminium Silicate | 1.00 |
| | Keltrol T | Xanthan Gum | 0.40 |
| III | Lameform® TGI | Polyglyceryl-3 Diisostearate | 1.50 |
| | Eumulgin® B2 | Ceteareth-20 | 3.50 |
| | Eumulgin® Prisma | Disodium Cetearyl Sulfosuccinate | 0,5 |
| | Cutina® PES | Pentaerythrityl Distearate | 1.00 |
| | Cegesoft® SH | Shorea Stenoptera | 1.00 |
| | Cetiol® CC | Dicaprylyl Carbonate | 4.00 |
| | Cetiol® Sensoft | Propylheptyl Caprylate | 2.00 |
| | Myritol® 331 | Cocoglycerides | 4.00 |
| | Fitoderm® | Squalane | 3.00 |
| | Cetiol® Sensoft | Propylheptyl Caprylate | 5.00 |
| | Phenonip | Phenoxyethanol (and) Parabens | 0.80 |
| IV | Sun Croma C47-051 | Titanium Dioxide | 6.00 |
| | SunPuro Yellow Iron Oxide C33-9001 | Iron Oxide Cl 77492 | 1.30 |
| | SunPuro Red Iron Oxide C33-8001 | Iron Oxide Cl 77491 | 0.60 |
| | SunPuro Black Iron Oxide C33-7001 | Iron Oxide Cl 77499 | 0.20 |
| V | Cosmedia® Gel CC | Dicaprylyl Carbonate (and) Stearalkonium Hectorite (and) PropyleneCarbonate | 3.00 |
| VI | Micropearl M100 | Polymethylmethacrylate | 2.00 |
| VII | Mirasil CDPDM | Cyclomethicone (and) Diphenyldimethicone | 3.00 |
| VIII | Perfume Caresse | | 0.20 |
| Viskosität (mPa.s): Brk. RVT spindle 5, speed 10 5 500, pH 7.3 | | | |

**Tabelle 1: Beispiele für getönte Tagespflege vom Typ O/W**

| **Inhaltstoff** | **1** | **2** | **3** | **4** | **5** | **6** | **7** | **8** |
|---|---|---|---|---|---|---|---|---|
| Eumulgin^{®} PRISMA | 0,5 | 1,0 | 2,0 | 0,5 | 0,5 | 2,0 | 2,0 | 1,0 |
| Cutina^{®} GMS-SE | 5,5 | | | | | | | 3,0 |
| Emulgade^{®} PL 68/50 | | 5,0 | | | | 2,0 | | |
| Eumulgin^{®} VL 75 | | | | 3,0 | | | 5,0 | |
| Tego Care^{®} 450 | | | | | | 2,0 | 2,0 | |
| Crodesta^{®} F-50 | | | | | 6,0 | | | |
| Amphisol^{®} K | | | | 2,0 | | | | |
| Lanette^{®} E | | 0,25 | | | 1,0 | 1,0 | | |
| Cutina^{®} FS 45 | 1,5 | | | | | | | |
| Eumulgin^{®} B 2 | | | 2,0 | | | | | 2,0 |
| Cutina^{®} PES | 2,0 | 1,0 | 2,0 | 4,0 | 2,0 | 2,0 | 2,5 | 2,0 |
| Lanette^{®} O | | | 2,0 | | | | | 1,0 |
| Cutina^{®} MD | | 0,5 | 3,0 | 3,0 | | | | |
| Cetiol^{®} LC | 4,0 | 5,0 | | | | | | |
| Cosmedia^{®} DC | 0,5 | | | 1,0 | | | | 1,0 |
| Cetiol^{®} CC | 4,0 | | 4,0 | | 7,0 | 5,0 | 10,0 | |
| Tegosoft^{®} DEC | | 5,0 | 2,0 | 4,0 | 2,0 | 2,0 | | 6,0 |
| Dow Corning^{®} 245 | | 2,0 | | 2,0 | | | | 2,0 |
| Eutanol^{®} G 16 | 4,0 | | | | 3,0 | 3,0 | | |
| Myritol^{®} 331 | | 5,0 | | | 2,0 | 2,0 | 5,0 | |
| Uvinul^{®} T 150 | | | | 0,5 | | | | 0,5 |
| Uvasorb^{®} HEB | 2,0 | | | | | | 1,0 | 1,0 |
| Tinosorb^{®} M | | | 2,0 | | | | | 2,0 |
| Tinosorb^{®} S | | | | 3,0 | | | | 2,0 |
| Neo Heliopan^{®} AV | | | | 2,0 | | | 2,0 | |
| Heo Heliopan^{®} AP | | | | 1,0 | | | 1,0 | |
| Uvinul^{®} A plus | | | 1 | | | | 2,0 | 2,0 |
| Microna^{®} Matte White | 5,0 | 5,0 | | 5,0 | 5,0 | 5,0 | | 5,0 |
| Microna^{®} Matte Black | 0,3 | 0,3 | 0,1 | 0,3 | 0,3 | 0,3 | 0,4 | 0,3 |
| Microna^{®} Matte Yellow | 3,0 | 3,0 | | 3,0 | 3,0 | 3,0 | 2,0 | 3,0 |
| Microna^{®} Matte Red | 0,6 | 0,6 | 1,0 | 0,6 | 0,6 | 0,6 | 1,0 | 0,6 |
| Ronasphere^{®}LDP | 1,0 | 1,0 | | 1,0 | 1,0 | 1,0 | | 1,0 |
| Pigment White 6 | | | 6,0 | | | | 6,0 | |
| Dry Flow PC | | | | | | | 2,0 | 2,0 |
| Glycerin | 5,0 | 5,0 | 3,0 | 5,0 | 5,0 | 5,0 | 3,0 | |
| Cosmedia^{®} SP | | | 0,3 | | 0,2 | | | |
| Water, de-ionized: ad 100, Preservative | | | | | | | | |

**Tabelle 2: Beispiele für getönte Tagespflege vom Typ W/O**

| **Inhaltstoff** | **1** | **2** | **3** | **4** | **5** | **6** | **7** | **8** |
|---|---|---|---|---|---|---|---|---|
| Eumulgin^{®} SG | 0,1 | 0,2 | 0,15 | 0.3 | 0,1 | 0,05 | 0,1 | 0,01 |
| Dehymuls^{®} PGPH | 5,5 | | 4,0 | | | | | 3,0 |
| Lameform^{®} TGI | | 5,0 | | | | 2,0 | | |
| Abil^{®} EM 90 | | | | 3,0 | | | 5,0 | |
| Isolan^{®} GI 34 | | | | | | 2,0 | 2,0 | |
| Isolan^{®} PDI | | | | | 6,0 | | | |
| Admul^{®} WOL 1403 | | | | 2,0 | | | | |
| Dehymuls^{®} HRE 7 | | 1,0 | | | 1,0 | 1,0 | | |
| Monomuls^{®} 90-O18 | 1,5 | | | | | | | 2,0 |
| Cutina^{®} PES | 2,0 | 1,0 | | 4,0 | | 1,0 | 2,5 | 2,0 |
| Cera Bellina | | | 2,0 | | | | | 2,0 |
| Beeswax | | | 2,0 | | | 2,0 | | 1,0 |
| Microcristalline Wax | | 1,5 | 3,0 | 3,0 | | | | |
| Cetiol^{®} LC | 4,0 | 5,0 | | | | | | |
| Cosmedia^{®} DC | 1,0 | | | | 0,5 | | 1,0 | |
| Cetiol^{®} CC | 4,0 | | | | 7,0 | 5,0 | 10,0 | |
| Tegosoft^{®} DEC | | 5,0 | 2,0 | 4,0 | 2,0 | 2,0 | | 6,0 |
| Dow Corning^{®} 245 | | 2,0 | | 2,0 | | | | 2,0 |
| Eutanol^{®} G 16 | 4,0 | | | | 3,0 | 3,0 | | |
| Myritol^{®} 331 | | 5,0 | | | 2,0 | 2,0 | 5,0 | |
| Uvinul^{®} T 150 | | | | 0,5 | | | | 0,5 |
| Uvasorb^{®} HEB | | | 2,0 | | | | 1,0 | 1,0 |
| Tinosorb^{®} M | | | 2,0 | | | | | 2,0 |
| Tinosorb S | | | | 3,0 | | | | 2,0 |
| Neo Heliopan^{®} AV | | | | 2,0 | | | 2,0 | |
| Heo Heliopan^{®} AP | | | | 1,0 | | | 1,0 | |
| Uvinul^{®} A plus | | | 1,0 | | | | 2,0 | 2,0 |
| Microna^{®} Matte White | 5,0 | 5,0 | | 5,0 | 5,0 | 5,0 | | 5,0 |
| Microna^{®} Matte Black | 0,3 | 0,3 | 0,1 | 0,3 | 0,3 | 0,3 | 0,4 | 0,3 |
| Microna^{®} Matte Yellow | 3,0 | 3,0 | 3,5 | 3,0 | 3,0 | 3,0 | 2,0 | 3,0 |
| Microna^{®} Matte Red | 0,6 | 0,6 | 1,0 | 0,6 | 0,6 | 0,6 | 1,0 | 0,6 |
| Ronasphere® LDP | 1,0 | 1,0 | | 1,0 | 1,0 | 1,0 | | 1,0 |
| Pigment White 6 | | | 6,0 | | | | 6,0 | |
| Dry Flow PC | | | | | | | 2,0 | 2,0 |
| Glycerin | 5,0 | 5,0 | 3,0 | 5,0 | 5,0 | 5,0 | 3,0 | |
| Water, de-ionized: ad 100, Preservative | | | | | | | | |

**Tabelle 3: Beispiele für Linpenstifte**

| **Inhaltstoff** | **1** | **2** | **3** | **4** |
|---|---|---|---|---|
| Eumulgin^{®} SG | 2,0 | 1,0 | 0,5 | 2,0 |
| Cutina^{®} LM conc | | | 10,0 | 36,0 |
| Candelilla Wax | 9,39 | 5,0 | 10,0 | |
| Carnauba wax | 2,85 | 7,0 | 5,0 | |
| Beeswax | 1,86 | 5,0 | 4,0 | |
| Cutina^{®} PES | 3,2 | 5,0 | 6,4 | 4,5 |
| Cetiol^{®} MM | | | 5,0 | |
| Cosmedia^{®} DC | 5,0 | 4,0 | 2,0 | 6,0 |
| Cetiol^{®} CC | 7,0 | 6,0 | 3,0 | 5,0 |
| Tegosoft^{®} DEC | 3,0 | 3,0 | 3,0 | 5,0 |
| Eutanol^{®} G | 10,97 | 12,0 | 12,0 | |
| Fitoderm^{®} | | | 4,0 | |
| Monomuls^{®} 90L 12 | | 3,0 | | |
| Dehymuls^{®} PGPH | | 4,0 | | |
| Castor Oill | 11,0 | 15,5 | 14,5 | 30,0 |
| Copherol^{®} F 1300 | 1,0 | 1,0 | 1,0 | 1,0 |
| Cosmetic white C47056 | 5,0 | 2,0 | 5,0 | |
| FDC Yellow 6 Al Lake C705270 | 7,0 | 7,0 | 8,0 | |
| Pigment White 6 | | | | |
| DC Red 7 Ca Lake C 19003 | 6,0 | 4,5 | 1,1 | 2,9 |
| Irodin 100 Silverpearl | | | | 9,6 |
| Hydagen^{®} CMF | | 10,0 | | |
| Irwinol^{®} LS 9319 | 1,0 | | 3,0 | |
| Mineral Oil | 12,8 | | | |
| Petrolatum | 6,84 | 3,0 | | |
| Ceresin | 2,75 | | | |
| Mycrocristalline Wax | 2,45 | | | |
| Colophane Claire type Y | 1,89 | | | |

**Tabelle 4: O/W-Sonnenschutzemulsionen**

| **Komponente** | **1** | **2** | **3** | **4** | **5** | **6** | **7** | **8** | **9** | **10** | **11** |
|---|---|---|---|---|---|---|---|---|---|---|---|
| **C - Creme, L - Lotion** | **L** | **C** | **S** | **L** | **C** | **L** | **L** | **C** | **L** | **C** | **L** |
| Eumulgin^{®} PRISMA | 0,5 | 1,0 | 0,2 | 0,1 | 1,0 | 0,5 | 0,5 | 2,0 | 0,5 | 0,1 | 0,5 |
| Eumulgin^{®} VL 75 | | | | | | | | | | | 2 |
| Eumulgin^{®} B2 | 0.5 | | | | | | | | | | |
| Tween^{®} 60 | | | | 0.2 | | | | | | | |
| Myrj^{®} 51 | | | | 0.5 | | | | | | | |
| Cutina^{®} E 24 | | | | 0.1 | | | | | | | |
| Hostaphat^{®} KL 340 N | | | | | | | | | 0.6 | | |
| Lanette^{®} E | | | 0.2 | | | | | | | | |
| Amphisol^{®} K | | | | | | | | | | 1 | |
| Natriumstearat | | | | | | | 1 | | | | |
| Emulgade^{®} PL 68/50 | | | 1 | | | | | | | | |
| Tego^{®} Care 450 | | | | | | | | | | 1 | |
| Cutina^{®} PES | 2 | 2.5 | 2.5 | | 2.5 | 2.5 | 2.5 | 2 | 2.5 | 1.7 | 1.5 |
| Cutina^{®} MD | 2 | | | 2 | | | 2 | | | 6 | |
| Lanette^{®} 14 | 1 | | | 1 | | | | 2 | | | 2 |
| Lanette^{®}O | 1 | 6 | | | 5 | 2 | | 2 | | | |
| Cosmedia^{®} DC | 1 | 1.5 | | 1 | 1 | | 2 | 2 | | | 2 |
| Antaron^{®}V 216 | | | 2 | | | 1.5 | | | 1 | 1 | |
| Emery 1780 | | | | | 0.5 | 0.5 | | | | | |
| Lanolin, wasserfrei USP | | | | | | | 5 | | | | |
| Myritol^{®} PC | | | | | 5 | | | | | | |
| Myritol^{®} 331 | 5 | | 8 | | | 6 | | 10 | | 2 | |
| Finsolv^{®} TN | | | 1 | | | | | 1 | 8 | | |
| Cetiol^{®} CC | | 2 | 5 | | | 4 | 4 | 2 | | 2 | |
| Cetiol^{®} OE | | | 3 | | | | | | 2 | 3 | |
| Dow Corning DC^{®} 244 | 4 | | 1 | | 5 | | | 2 | | | 2 |
| Dow Corning DC^{®} 2502 | | 1 | | | 2 | | | | | | |
| Squatol^{®} S | | | | | | | 4 | | | | |
| Silikonöl Wacker AK^{®} 350 | | 2 | | | | | | | | | |
| Cetiol^{®} 868 | | | | | 2 | | 4 | | | | 7 |
| Cetiol^{®} J 600 | | | | | 3 | 2 | | | | 5 | |
| Mineralöl | | | | 9 | | | | | | | |
| Cetiol^{®} B | | | 1 | | | | | | | 2 | |
| Eutanol^{®} G | | | | 2 | | | | | 4 | | |
| Eutanol^{®} G 16 | 4 | | | | | 4 | | | | | |
| Cetiol^{®} PGL | | 5 | | | | | | | | 5 | |
| Mandelöl | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl^{®} LS | | | | | | | | | | | |
| Neo Heliopan^{®} AP (Na-Salz) | | 1 | | | | | | | 1 | | |
| Neo Heliopan^{®} Hydro (Na-Salz) | 2 | | 2.2 | | | | | | 1 | | |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} A PLUS | | | | 2 | 1 | | | | | | |
| Uvinul^{®} T 150 | 2 | | | | 2.5 | | | 1 | | | |
| Tinosorb^{®} M | | | 3 | | | | 2 | | | | 3 |
| Tinosorb^{®} S | | | 1 | | | | 1,5 | | | | |
| Uvasorb^{®} HEB | | 1 | | | 1 | | | | | | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex^{®} T 2000 | | | | | 5 | | 3 | 3 | | | 4 |
| Veegum^{®} Ultra | 1.5 | | 0.75 | | | | | 1 | 1 | | |
| Keltrol^{®} T | 0.5 | | 0.25 | | | | | 0.5 | 0.5 | | |
| Cosmedia^{®} SP | | 0.5 | | | 0.5 | | 0.2 | 0.2 | | 0.2 | 0.2 |
| Carbopol^{®} 980 | | | | 0.2 | | 0.2 | | | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |
| Konservierungsmittel, NaOH, Wasser | q.s.ad 100 | | | | | | | | | | |

**Tabelle 5: O/W-Sonnenschutzemulsionen**

| **Komponente** | **12** | **13** | **14** | **15** | **16** | **17** | **18** | **19** | **20** | **21** | **22** |
|---|---|---|---|---|---|---|---|---|---|---|---|
| **C - Creme, L - Lotion** | L | C | L | C | L | C | S | C | C | L | L |
| Eumulgin^{®} PRISMA | 0,4 | 1,0 | 0,3 | 1,5 | 0,6 | 1,2 | 0,3 | 0,6 | 2,0 | 0,4 | 0,4 |
| Eumulgin^{®} VL 75 | | | | | 1.8 | | | | | | |
| Eumulgin^{®} B2 | | | | | | | | | | 0.2 | |
| Tween^{®} 60 | | | | | | | | | | 0.3 | |
| Cutina^{®} E 24 | | | | 0.5 | | | | | | | |
| Lanette^{®} E | | | | | | | 0.1 | | | | |
| Amphisol^{®} K | 0.5 | | | | | | 1 | | | | |
| Natriumstearat | | | | | 1 | | | | | | |
| Emulgade^{®} PL 68/50 | | 1.5 | | | | | | | | | |
| Tego^{®} Care 450 | | | | | | | | | 0.3 | | |
| Cutina^{®} PES | 2 | 1.8 | 2.5 | 1.5 | 1 | 2 | 2.5 | 3 | 2.0 | 1.5 | 1.5 |
| Cutina^{®} MD | 1 | | | 4 | 1 | 3 | | | | | 1 |
| Lanette^{®} 14 | | 2 | | | | | | | | 1 | |
| Lanette^{®} O | | | | 2 | | | | 2 | 1 | 1 | |
| Allianz^{®} OPT | 1 | | | 1 | 1 | | | 2 | | | 2 |
| Cosmedia^{®} DC | | 1.5 | 2 | | | 1.5 | 2 | | 1.5 | 1.5 | |
| Emery^{®} 1780 | | | | 1 | 1 | | | | | | |
| Lanolin, wasserfrei, USP | | | | | | 1 | 1 | | | | |
| Myritol^{®} PC | | | | | | | | | 5 | | |
| Myritol^{®} 331 | 12 | | 12 | | | 8 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Corning DC^{®} 244 | | 2 | | | 1 | | | | | | |
| Dow Corning DC^{®} 2502 | | 1 | | | 1 | | | 3 | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Mineralöl | | | | 10 | | | | | | | |
| Cetiol^{®} B | 4 | | 4 | | | | | 4 | | | |
| Eutanol^{®} G | | 3 | | | | 3 | | | | | |
| Eutanol^{®} G 16 S | 10 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol /Tocopherylacetat | 1 | | | | | | | | | | |
| Neo Heliopan^{®} Hydro (Na-Salz) | | | | | | | | | | 3 | |
| Eusolex^{®} OCR | 6 | | 9 | | 5 | 7 | 9 | | 4 | | 7 |
| Neo Heliopan^{®} AP (Na-Salz) | | | | 0.5 | | 1 | | | | | |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} A PLUS | | | | | 1 | | 2 | | | | |
| Uvinul^{®} T 150 | 1 | | | | | | | | 1.3 | 1 | 1 |
| Tinosorb^{®} M | | | 6,5 | | | | | | | 4 | |
| Tinosorb^{®} S | | | 1 | | 2 | | | | | | |
| Uvasorb^{®} HEB | 1 | | | | | | | | | | 2 |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Cosmedia^{®} SP | | | 0.2 | 0.3 | | | 0.1 | | | 0.2 | |
| Pemulen^{®} TR 2 | | 0.3 | | 0.3 | | | | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 6: W/O-Sonnenschutzemulsionen**

| Komponente | 23 | 24 | 25 | 26 | 27 | 28 | 29 | 30 | 31 | 32 | 34 |
|---|---|---|---|---|---|---|---|---|---|---|---|
| C - Creme, L - Lotion | C | L | C | L | C | L | L | L | L | C | C |
| Eumulgin^{®} PRISMA | 0,05 | 0,1 | 0,2 | 0,1 | 0,2 | 0,1 | 0,3 | 0,1 | 0,4 | 0,1 | 0,2 |
| Dehymuls^{®} PGPH | 4 | 2 | 1 | 3 | 3 | 1 | 1 | 2 | 2 | 4 | 1 |
| Monomuls^{®} 90-O18 | | | 2 | | | | | | | | |
| Lameform^{®} TGI | 2 | | 4 | | 3 | | | | | 1 | 3 |
| Abil^{®} EM 90 | | | | | | | 4 | | | | |
| Isolan^{®} PDI | | | | | | 4 | | 2 | | | |
| Zinc Stearate | 1 | | | 1 | 1 | | | 1 | | 1 | |
| Beeswax | 1 | | 5 | 1 | | | | 5 | | 7 | 5 |
| Tego^{®} Care CG | | | | | 1 | | | | | | 0.5 |
| Cutina^{®} PES | 2 | 3 | 2 | 1 | 2 | 1 | 1 | 1 | 5 | 1 | 4 |
| Prisorine^{®} 3505 | | | 1 | | | 1 | 1 | | | | 1 |
| Cosmedia DC | 3 | 4 | 2 | 1 | 1 | 2 | 2 | 2 | 3 | 1 | 1 |
| Myritol^{®} 331 | 2 | | | | 3 | 6 | | | | | 8 |
| Finsolv^{®} TN | | | | 5 | | | 5 | | | | |
| Cetiol^{®} CC | 10 | 4 | 2 | | 4 | 2 | | | 2 | 3 | 5 |
| Tegosoft DEC | | 4 | | 3 | 2 | | 5 | 5 | | 3 | |
| Cetiol^{®} OE | | | | | 4 | | 5 | | 4 | 2 | |
| Dow Corning^{®} DC 244 | | | 3 | | | | 2 | | 4 | 4 | |
| Dow Corning^{®} DC 2502 | 1 | | 1 | | 2 | 1 | | | | | 1 |
| Silikonöl Wacker AK 350 | | 1 | | 4 | | | | 3 | | | |
| Cetiol^{®} PGL | | 3 | | | | 4 | | | 4 | | |
| Copherol^{®} F 1300 | 1 | | | | | | | | | | |
| Magnesiumsulfat x 7 Wasser | 1 | | | | | | | | | | |
| Neo Heliopan^{®} Hydro (Na-Salz) | 2 | | 2.2 | | 3 | 3 | | | 1 | | 2 |
| Neo Heliopan^{®} 303 | | 5 | | | | | | | 4 | | 4 |
| Uvasorb^{®} HEB | 1 | | | 1 | 1 | | | | | | 2 |
| Neo Heliopan^{®} MBC | 2 | | | | | 2 | 2 | 2 | | | |
| Uvinul^{®} A plus | | | | | 2 | | | | 3 | 3 | |
| Neo Heliopan^{®} AP (Na-Salz) | | 2 | 2 | | 1 | | | | 1 | | 6 |
| Neo Heliopan^{®} AV | 3 | | 4 | 6 | 4 | 7.5 | 4 | 5 | | | 1 |
| Uvinul^{®} T 150 | 1 | 1 | | | 2.5 | | | 1 | | | |
| Parsol^{®} 1789 | 2 | 1 | | | | | 2 | | 2 | 2 | |
| Zinkoxid NDM | | | | | | 10 | | 3 | | | 4 |
| Tinosorb^{®} M | | 3 | | 3 | | | | 2 | | 2 | |
| Tinosorb^{®} S | | 3 | | 3 | | | | 2 | | 2 | |
| Eusolex^{®} T Aqua | | | 8 | | | | | 5 | | | |
| Eusolex^{®} T 2000 | | | | | 5 | | 3 | 3 | | | 4 |
| Ethanol | | | | | | | | | | 8 | |
| Glycerin | 5 | 3 | 3 | 3 | 5 | 3 | 2 | 3 | 10 | 4 | 3 |
| Wasser, Konservierungsmittel | ad 100, q.s. | | | | | | | | | | |

**Tabelle 7: Sprühbare Emulsionen**

| **Komponente** | **1** | **2** | **3** | **4** | **5** | **6** | **7** | **8** | **9** | **10** | **11** | **12** |
|---|---|---|---|---|---|---|---|---|---|---|---|---|
| **S - Spray, SC - Sprühbare Creme** | **S** | **S** | **S** | **S** | **SC** | **S** | **S** | **S** | **S** | **S** | **S** | **S** |
| Eumulgin^{®} PRISMA | 0,5 | 0,5 | 0,2 | 0,3 | 0,2 | 0,2 | 0,2 | 0,3 | 0,3 | 0,3 | 0,3 | 0,55 |
| Eumulgin^{®} B2 | | | | | | 1 | | | | | | |
| Eumulgin^{®} HRE 40 | | | | | | | 1 | | | | | |
| Lanette^{®} E | | | 0.2 | 0,1 | | | | | | | | |
| Amphisol^{®} K | | | | 1 | | | | | | | | |
| Emulgade^{®} CM | | | | | | | 20 | | | | | |
| Emulgade^{®} SE-PF | | | | | | 4,5 | | | | | | |
| Emulgade^{®} PL 68/50 | | | 1 | | | | | | | | | |
| Dehyquart^{®} C 4046 | | | | | 6 | | | | | | | |
| Cutina^{®} MD | | | | | | | | 0,5 | | 0,25 | 0,25 | 0,25 |
| Cutina^{®} PES | 1 | 1 | 2.5 | 2,5 | 3 | 1 | 2 | | 0,25 | | | |
| Lanette^{®} O | | | | | | | | | 0,25 | 0,1 | | |
| Cosmedia^{®} DC | 1 | | | 2 | | 1,5 | 0,5 | | | | | |
| Antaron^{®}V 216 | | | 2 | | | | | | | | | |
| Lanolin, wasserfrei USP | | | | 1 | | | | | | | | |
| Myritol^{®} 331 | 10 | 10 | 8 | 8 | | | | | | | | |
| Finsolv^{®} TN | | | 1 | | | | | | | | | |
| Cetiol^{®} CC | 6 | 6 | 5 | 5 | 4 | | | 1 | 1 | 1 | 4,5 | 10 |
| Cetiol^{®} OE | | | 3 | | | 5 | | | | | | |
| Cetiol^{®} SN | | | | | 4 | | | | | | | |
| Dow Corning DC^{®} 244 | | | 1 | | | | | | | | | |
| Cetiol^{®} S | | | | | | 5 | | | | | | |
| Cetiol^{®} B | | | 1 | | | | | | | | | |
| Cetiol^{®} 868 | | | | | | | | 1 | 1 | 1 | | |
| Cetiol^{®} SB 45 | | | | | | | | | | | 0,5 | 0,5 |
| Cegesoft^{®} PS6 | | | | | | | | 1 | 1 | 1 | | |
| Mandelöl | | | 2 | | | | | | | | | |
| Panthenol | 1 | | | | | | | | | | | |
| Bisabolol | 0,2 | | | | | | | | | | | |
| Tocopherol/ Tocopherylacetat | 1 | | | | | | | | | | | |
| Neo Heliopan^{®} 303 | | | 2.2 | 9 | | | | | | | | |
| Neo Heliopan^{®} BB | | | 9 | | | | | | | | | |
| Neo Heliopan^{®} AV | 7.5 | 7.5 | | | | | | | | | | |
| Uvinul^{®} A PLUS | | | 7.5 | 2 | | | | | | | | |
| Tinosorb^{®} S | | | 3 | | | | | | | | | |
| Uvasorb^{®} HEB | | | 1 | | | | | | | | | |
| Parsol^{®} 1789 | 2 | 2 | | | | | | | | | | |
| Zinkoxid NDM | | | 1 | | | | | | | | | |
| Z-Cote HP 1 | | | | 5 | | | | | | | | |
| Eusolex^{®} T 2000 | | | 5 | | | | | | | | | |
| Veequm^{®} Ultra | | | | 1,2 | | | | | | | | |
| Keltrol^{®} T | | | 0.75 | 0,4 | 1 | | | | | | | |
| Cosmedia^{®} SP | 0.3 | 0.3 | 0.25 | 0,1 | | | | 0,25 | | 0,25 | 0,25 | 0,5 |
| Locron^{®} L | | | | | | 10 | | | | | | |
| Hydagen^{®} C.A.T. | | | | | | 2 | | | | | | |
| Ethanol | | | | | | | | | | | | |
| Butylenglykol | | | | | | | | | | | | |
| Citronensäure | | | | | | | | | | | | 0,15 |
| Glycerin | 5 | 5 | 5 | 3 | 2 | 5 | | | | | | |
| Konservierungsmittel q.s., NaOH q.s., Wasser ad 100 | | | | | | | | | | | | |

**Tabelle 8:O/W-Pflegeemulsionen**

| **Komponente** | **1** | **2** | **3** | **4** | **5** | **6** | **7** | **8** | **9** | **10** | **11** |
|---|---|---|---|---|---|---|---|---|---|---|---|
| **C - Creme, L - Lotion** | **C** | **C** | **C** | **L** | **C** | **L** | **L** | **C** | **L** | **C** | **C** |
| Eumulgin^{®} PRISMA | 0,5 | 1,5 | 1,0 | 1,0 | 0,3 | 0,8 | 0,8 | 0,5 | 0,14 | 1,0 | 1,5 |
| Eumulgin^{®} VL 75 | | | | | | | | | | 1.5 | |
| Dehymuls^{®} PGPH | | 0.6 | | | | | | | | | |
| Generol^{®} R | | | 0.5 | | | | | | | | |
| Eumulgin^{®} B2 | | | 0.1 | | | | | | | | |
| Hostaphat^{®} KL 340 N | | | | | | | | | 0.5 | | |
| Lanette^{®} E | | | | | | | | 0.6 | | | |
| Amphisol^{®} K | | 0.2 | | | | | | | | | |
| Natriumstearat | | | | | 0.5 | | | | | | |
| Emulgade^{®} PL 68/50 | | | | | | | | | | | 1.2 |
| Tego^{®} Care CG 90 | 0.7 | | | | | | | | | | |
| Tego^{®} Care 450 | | | | | 0.4 | | | | | | |
| Cutina^{®} PES | 2.5 | 2 | 3 | | 2 | | 2.5 | 1.7 | 2.5 | 1.5 | 1.2 |
| Cutina^{®} MD | | 1 | | 3 | 5 | | 2 | | | 6 | |
| Lanette^{®} 14 | | | | 1 | | | | 4 | | | 4 |
| Lanette^{®} O | 4.5 | | 4 | | 1 | 2 | | | | | 2 |
| Novata^{®} AB | | 1 | | | | | | | | | 1 |
| Emery^{®} 1780 | | | | | 0.5 | 0.5 | | | | | |
| Lanolin, wasserfrei, USP | | | | | | | 1.1 | | | | |
| Cosmedia^{®} DC | | 1.5 | 2 | | | 1.5 | 2 | | 1.5 | 1.5 | |
| Cetiol^{®} SB 45 | | | 1.5 | | | | 2 | | | | |
| Cegesoft^{®} C 17 | | | | | | | | | | | 2 |
| Myritol^{®} PC | | | | | 5 | | | | | | |
| Myritol^{®} 331 | 2 | 5 | 5 | | | 6 | | 12 | | | |
| Finsolv^{®} TN | | | 2 | | | 2 | | | 8 | | |
| Cetiol^{®} CC | 4 | 6 | | | | 4 | 4 | | | | 5 |
| Cetiol^{®} OE | | | | 5 | | | | | 4 | 3 | |
| Dow Corning DC^{®} 245 | | | 2 | | 5 | 1 | | | | | |
| Dow Corning DC^{®} 2502 | | | | | 2 | 1 | | | | | 5 |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol^{®} 868 | | | | | 2 | | 4 | | | | |
| Cetiol^{®} J 600 | 2 | | 3 | | 3 | 2 | | | | 5 | |
| Ceraphyl^{®} 45 | | | | | | | 3 | | | | |
| Mineralöl | | | | 9 | | | | | | | |
| Cetiol^{®} SN | | | 5 | | | | | | | | |
| Cetiol^{®} B | | | | | | | | 4 | | 2 | |
| Eutanol^{®} G | | 2 | | 3 | | | | | | | |
| Cetiol^{®} PGL | | | | | | | | | 5 | 5 | |
| Dry Flo^{®} Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N,N-Diethyl-m-toluamid | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} ultra | | | | | | | | | 1 | | |
| Keltrol^{®} T | | | 0.4 | | | | | | 0.5 | | |
| Cosmedia^{®} SP | | 0.3 | | 0.2 | 0.2 | | | | 0.2 | 0.3 | |
| Pemulen^{®} TR 2 | 0.3 | | | | | | | 0.3 | | | |
| Carbopol^{®} Ultrez 10 | | | 0.3 | | | 0.2 | | | | | |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser ad 100, Konservierungsmittel q.s., NaOH q.s., q.s., pH 6,5 - 7,5 | | | | | | | | | | | |

**Tabelle 9: O/W-Pflegeemulsionen**

| **Komponente** | **12** | **13** | **14** | **15** | **16** | **17** | **18** | **19** | **20** | **21** | **22** |
|---|---|---|---|---|---|---|---|---|---|---|---|
| **C - Creme, L - Lotion** | **C** | **C** | **L** | **C** | **L** | **C** | **C** | **L** | **L** | **L** | **C** |
| Eumulgin^{®} PRISMA | 1,5 | 1 | 0,5 | 1 | 0,2 | 0,5 | 1 | 0,6 | 0,6 | 0,3 | 1 |
| Eumulgin^{®} VL 75 | | | | | | | | | | | 1 |
| Generol^{®} R | | | | | | 0.3 | | | | | |
| Eumulgin^{®} B2 | | | | | | | | | | 1 | |
| Tween^{®} 60 | | | | | | | | | | 1 | |
| Cutina^{®} E 24 | | | | 0.5 | | | | | | | |
| Amphisol^{®} K | | 0.5 | | | | | | | | | |
| Natriumstearat | | | | | 1 | | | | | | |
| Emulgade^{®} PL 68/50 | | | | | | | | 1 | | | |
| Tego^{®} Care 450 | | | | | | | | | 1 | | |
| Cutina^{®} PES | | 2 | 1 | 3 | 2.5 | 2 | | 1.2 | 2.5 | 1.5 | 0.8 |
| Cutina^{®} MD | 3 | 1 | | 4 | | | 4 | | | | |
| Lanette^{®} 14 | | 2 | | | 1 | | | 2 | | 1 | |
| Lanette^{®} O | 2 | | | 2 | | 3 | 1 | | 1 | 1 | 6 |
| Novata^{®} AB | | | | | | | | | 1 | 1 | |
| Emery^{®} 1780 | | | | | | | | | | | 0.5 |
| Lanolin, wasserfrei, USP | | | | | | 4 | | | | | |
| Cosmedia^{®} DC | | | 1 | | | 1.5 | | | 1 | 1 | |
| Cetiol^{®} SB 45 | | | | | | | 2 | | | | |
| Cegesoft^{®} C 17 | 4 | | | | | | | | | | |
| Myritol^{®} PC | 6 | | | | | 5 | | | 5 | | |
| Myritol^{®} 331 | 5 | | 5 | | | | 7 | | | 10 | 3 |
| Finsolv^{®} TN | | 5 | | 4 | 5 | | | 3 | 3 | | 1 |
| Cetiol^{®} CC | | | 8 | 6 | | | 4 | 3 | | | 2 |
| Cetiol^{®} OE | | | | | 2 | | 2 | | 5 | | |
| Dow Corning DC^{®} 245 | | 2 | | | 1 | 8 | | | | 8 | 2 |
| Dow Corning DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | 2 | | | | | | | |
| Ceraphyl^{®} 45 | | | | | | | 3 | | | | |
| Cetiol^{®} SN | | | | 5 | | | | | | | |
| Cetiol^{®} B | | | | | | 5 | | 4 | | | 3 |
| Eutanol^{®} G | | 3 | | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Cosmedia^{®} SP | 0.1 | | 1 | | 0.2 | 0.2 | 0.2 | 0.2 | | | 0.5 |
| Carbopol^{®} ETD 2001 | | 0.3 | | 0.3 | | | | | | | |
| Pemulen^{®} TR 2 | | | | | | 0.3 | | | | | |
| Ethanol | | 5 | | 8 | | | | | | | 10 |
| Butylenglykol | 5 | | | 3 | 3 | | | | | 8 | |
| Glycerin | 2 | 4 | 3 | 3 | | 7 | 5 | 3 | 5 | | |
| Wasser ad 100" Konservierungsmittel q.s., NaOH (pH 6,5 - 7,5) | | | | | | | | | | | |

**Tabelle 10: O/W-Pflegeemulsionen**

| **Komponente** | **45** | **46** | **47** | **48** | **49** |
|---|---|---|---|---|---|
| **C - Creme, L - Lotion, SC = sprayable cream** | **SC** | **C** | **C** | **L** | **C** |
| Eumulgin^{®} PRISMA | 0,2 | 0,3 | 0,5 | 0,2 | 0,2 |
| Dehyquart^{®} C 4046 | 6 | | | 3 | |
| Cutina^{®} GMS-SE | | | 2 | | 5.5 |
| Cutina^{®} FS 45 | | | | | 1.5 |
| Eumulgin^{®} B2 | | 1 | | | |
| Cutina^{®} PES | 3 | 2 | 2 | 2 | 2 |
| Cutina^{®} MD | | 1,5 | | | |
| Cosmedia^{®} DC | | | | 0.5 | |
| Cegesoft^{®} PS 6 | | | | 4.5 | |
| Cegesoft^{®} SH | | 7 | 3 | | |
| Myritol^{®} 331 | | | 5 | 4.5 | |
| Cetiol^{®} CC | 4 | | 3 | 3 | |
| Cetiol^{®} OE | | 1 | | | |
| Silikonöl Wacker AK^{®} 350 | | | | | 0.5 |
| Paraffin Liquid | | | | | 6 |
| Isopropylpalmitate | | | | 2 | |
| Cetiol^{®} 868 | | 7 | 8 | | |
| Cetiol^{®} SN | 4 | | | | 3 |
| Eutanol^{®} G | | | | | 3 |
| Mandelöl | | 7 | | | |
| Panthenol | 1 | 0.2 | 1 | | |
| Bisabolol | 1 | | | | |
| Tocopherol / Tocopherylacetat | 0.2 | | | | |
| Keltrol^{®} T | 1 | | | | |
| Cosmedia^{®} SP | | 1 | 0.7 | 0.45 | |
| Glycerin | 2 | 5 | 5 | 5 | |
| Wasser, Konservierungsmittel, NaOH | ad 100, q.s., (pH 6,5 - 7,5) | | | | |

**Tabelle 11:**

| **Komponenten (INCI)** | **1** | **2** | **3** | **4** | **5** | **6** | **7** |
|---|---|---|---|---|---|---|---|
| Eumulgin^{®} PRISMA (Disodium Cetearyl Sulfosuccinate) | 0,1 | 0,05 | 0,2 | 0,15 | 0,2 | 0,9 | 1,1 |
| Glyceryl Stearate, Ceteareth-20, Ceteareth-12, Cetearyl Alcohol, Cetyl Palmitate (Emulgade^{®} SE) | 6 | | 4,5 | | | 6 | |
| Ceteareth-12 (Eumulgin^{®}B1) | | | | | | | |
| Ceteareth-20 (Eumulgin^{®}B2) | | | 1 | | | | |
| Aqua, Cetearyl Isononanoate, Glyceryl Stearate, Ceteareth-20, Ceteareth-12, Cetearyl Alcohol, Cetyl Palmitate (Emulgade^{®} CM) | | | | | 20 | | |
| Polyglyceryl-3 Diisostearate Lameform TGI | | 3 | | | | | |
| Cocoglycerides (Novata^{®} AB) | | | | | | | 4 |
| Stearyl alcohol ( Lanette 18) | | | | 10 | | | |
| Hydrogenated Castor Oil (Cutina^{®} HR) | | | | 3,7 | | | 6,5 |
| Polyglyceryl-2 Dipolyhydroxystearate (Dehymuls^{®} PGPH) | | 1 | | | | | |
| Sodium Cetearyl Sulfate (Lanette^{®} E) | 0,3 | | | | | 0,3 | |
| Pentaerythrityl Distearate (Cutina^{®} PES) | 1,5 | 2 | 1 | 4,7 | 2 | 5 | 4 |
| Behenyl Alcohol (Lanette^{®} 22) | 2 | | | | | 4 | |
| Dicaprylyl Carbonate (Cetiol^{®} CC) | | 7 | | | | | |
| Dicaprylylether ( Cetiol^{®} OE) | 6 | | 5 | 9 | | 6 | 9 |
| Cocoglycerides (Myritol^{®} 331) | | | | | | | |
| Diethylhexylcyclohexane (Cetiol^{®} S) | | | 5 | 14,7 | | | 35 |
| Cyclopentasiloxane | 3 | 5 | | 34 | | 3 | 14 |
| Cyclopentasiloxane and Dimethicone/ Vinyldimethicone Crosspolymer SFE 839 (GE Bayer) | | 3 | | | | | |
| Dimethicone | 1 | | | | | | |
| Hydrogenated Dimer Dilinoleyl / Dimethylcarbonate Copolymer (Cosmedia^{®} DC) | 0,5 | 1 | 1,5 | 1 | 0,5 | 2 | 1 |
| Triethyl Citrate (Hydagen^{®} C.A.T) | | | 2 | | | | |
| PEG-40 Hydrogenated Castor Oil | | | | | 1 | | |
| Tocopheryl Acetate | | | | 1 | | | |
| Aluminium Zirconium Tetrachlorohydrex GLY (Rezal 36) | 30 | 40 | | 22,9 | | 30 | 25 |
| Aluminum Chlorhydrate (Locron L) | | | 10 | | | | |
| Chitosan (Hydagen^{®} DCMF) | 0,05 | | | | | | |
| Glycolic Acid | 0,02 | | | | | | |
| Glycerin | | 5 | 5 | | | | |
| Propylene Carbonate ( Fluka) | | | | | | | 0,5 |
| Quaternium-18 Hectorite (Bentone 18) | | | | | | | 1 |
| Talc (Merck) | | | | | | 5 | 5 |
| MgSO4 x 7H2O | | 1 | | | | | |
| Wasser | Ad 100 | | | | | | |
| Perfume, Preservatives | q.s. | q.s. | q.s. | q.s. | q.s. | q.s. | q.s. |

| | | | | | | | |
|---|---|---|---|---|---|---|---|
| 1 - Antiperspirant / Deo Creme ; 2 - Antiperspirant Creme (W/O); 3 - Antiperspirant / Deo Spray; 4 - Antiperspirant Stift mit Vitamin E ; 5 - Deodorant Wipe - Formulierung ; 6 - Antiperspirant Creme ; 7- Antiperspirant Creme «Soft solid » | | | | | | | |

**Tabelle 12: O/W-Pflegeemulsionen**

| **Komponente** | **1** | **2** | **3** | **4** | **5** | **6** | **7** | **8** | **9** | **10** | **11** |
|---|---|---|---|---|---|---|---|---|---|---|---|
| **C - Creme, L - Lotion** | C | C | C | L | C | L | L | C | L | C | C |
| Eumulgin^{®} PRISMA | 0.5 | 1.5 | 1.0 | 1.0 | 0.3 | 0.8 | 0.8 | 0.5 | 0.14 | 1.0 | 1.5 |
| Emulgade^{®} Sucro | 2.5 | 2 | 3 | 2 | 2 | 2.5 | 2.5 | 1.7 | 2.5 | 1.5 | 1.2 |
| Generol^{®} R | | | 0.5 | | | | | | | | |
| Eumulgin^{®} B2 | | | 0.1 | | | | | | | | |
| Hostaphat^{®} KL 340 N | | | | | | | | | 0.5 | | |
| Lanette^{®} E | | | | | | | | 0.6 | | | |
| Amphisol^{®} K | | 0.2 | | | | | | | | | |
| Natriumstearat | | | | | 0.5 | | | | | | |
| Emulgade^{®} PL 68/50 | | | | | | | | | | | 1.2 |
| Tego^{®} Care CG | 0.7 | | | | | | | | | | |
| Tego^{®} Care 450 | | | | | 0.4 | | | | | | |
| Cutina^{®} PES | | | | | | | | | 2.5 | | |
| Cutina^{®} MD | | 1 | | 3 | 5 | | 2 | | | 6 | |
| Lanette^{®} 14 | | | | 1 | | | | 4 | | | 4 |
| Lanette^{®} O | 4.5 | | 4 | | 1 | 2 | | | | | 2 |
| Novata^{®} AB | | 1 | | | | | | | | | 1 |
| Emery^{®} 1780 | | | | | 0.5 | 0.5 | | | | | |
| Lanolin, wasserfrei, USP | | | | | | | 1.1 | | | | |
| Cosmedia^{®} DC | | 1.5 | 2 | | | 1.5 | 2 | | 1.5 | 1.5 | |
| Cetiol^{®} SB 45 | | | 1.5 | | | | 2 | | | | |
| Cegesoft^{®} C 17 | | | | | | | | | | | 2 |
| Myritol^{®} PC | | | | | 5 | | | | | | |
| Myritol^{®} 331 | 2 | 5 | 5 | | | 6 | | 12 | | | |
| Finsolv^{®} TN | | | 2 | | | 2 | | | 8 | | |
| Cetiol^{®} CC | 4 | 6 | | | | 4 | 4 | | | | 5 |
| Cetiol^{®} OE | | | | 5 | | | | | 4 | 3 | |
| Dow Corning DC^{®} 245 | | | 2 | | 5 | 1 | | | | | |
| Dow Corning DC^{®} 2502 | | | | | 2 | 1 | | | | | 5 |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol^{®} 868 | | | | | 2 | | 4 | | | | |
| Cetiol^{®} J 600 | 2 | | 3 | | 3 | 2 | | | | 5 | |
| Ceraphyl^{®} 45 | | | | | | | 3 | | | | |
| Mineralöl | | | | 9 | | | | | | | |
| Cetiol^{®} SN | | | 5 | | | | | | | | |
| Cetiol^{®} B | | | | | | | | 4 | | 2 | |
| Eutanol^{®} G | | 2 | | 3 | | | | | | | |
| Cetiol^{®} PGL | | | | | | | | | 5 | 5 | |
| Dry Flo^{®} Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N,N-Diethyl-m-toluamid | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} ultra | | | | | | | | | 1 | | |
| Keltrol^{®} T | | | 0.4 | | | | | | 0.5 | | |
| Cosmedia^{®} SP | | 0.3 | | 0.2 | 0.2 | | | | 0.2 | 0.3 | |
| Pemulen^{®} TR 2 | 0.3 | | | | | | | 0.3 | | | |
| Carbopol^{®} Ultrez 10 | | | 0.3 | | | 0.2 | | | | | |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel, NaOH | ad 100, q.s., pH 6,5 - 7,5 | | | | | | | | | | |

**Tabelle 13: O/W-Pflegeemulsionen**

| **Komponente** | **12** | **13** | **14** | **15** | **16** | **17** | **18** | **19** | **20** | **21** | **22** |
|---|---|---|---|---|---|---|---|---|---|---|---|
| **C - Creme, L - Lotion** | C | C | L | C | L | C | C | L | L | L | C |
| Eumulgin^{®} SG | 1.5 | 1 | 0.5 | 1 | 0.2 | 0.5 | 1 | 0.6 | 0.6 | 0.3 | 1 |
| Emulgade^{®} Sucro | 1 | 2 | 1 | 3 | 2.5 | 2 | 1 | 1.2 | 2.5 | 1.5 | 1.0 |
| Generol^{®} R | | | | | | 0.3 | | | | | |
| Eumulgin^{®} B2 | | | | | | | | | | 1 | |
| Tween^{®} 60 | | | | | | | | | | 1 | |
| Cutina^{®} E 24 | | | | 0.5 | | | | | | | |
| Amphisol^{®} K | | 0.5 | | | | | | | | | |
| Natriumstearat | | | | | 1 | | | | | | |
| Emulgade^{®} PL 68/50 | | | | | | | | 1 | | | |
| Tego^{®} Care 450 | | | | | | | | | 1 | | |
| Cutina^{®} PES | | | | | | | | | | | 0.8 |
| Cutina^{®} MD | 3 | 1 | | 4 | | | 4 | | | | |
| Lanette^{®} 14 | | 2 | | | 1 | | | 2 | | 1 | |
| Lanette^{®} O | 2 | | | 2 | | 3 | 1 | | 1 | 1 | 6 |
| Novata^{®} AB | | | | | | | | | 1 | 1 | |
| Emery^{®} 1780 | | | | | | | | | | | 0.5 |
| Lanolin, wasserfrei, USP | | | | | | 4 | | | | | |
| Cosmedia^{®} DC | | | 1 | | | 1.5 | | | 1 | 1 | |
| Cetiol^{®} SB 45 | | | | | | | 2 | | | | |
| Cegesoft^{®} C 17 | 4 | | | | | | | | | | |
| Myritol^{®} PC | 6 | | | | | 5 | | | 5 | | |
| Myritol^{®} 331 | 5 | | 5 | | | | 7 | | | 10 | 3 |
| Finsolv^{®} TN | | 5 | | 4 | 5 | | | 3 | 3 | | 1 |
| Cetiol^{®} CC | | | 8 | 6 | | | 4 | 3 | | | 2 |
| Cetiol^{®} OE | | | | | 2 | | 2 | | 5 | | |
| Dow Corninq DC^{®} 245 | | 2 | | | 1 | 8 | | | | 8 | 2 |
| Dow Corning DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | 2 | | | | | | | |
| Ceraphyl^{®} 45 | | | | | | | 3 | | | | |
| Cetiol^{®} SN | | | | 5 | | | | | | | |
| Cetiol^{®} B | | | | | | 5 | | 4 | | | 3 |
| Eutanol^{®} G | | 3 | | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Cosmedia^{®} SP | 0.1 | | 1 | | 0.2 | 0.2 | 0.2 | 0.2 | | | 0.5 |
| Carbopol^{®} ETD 2001 | | 0.3 | | 0.3 | | | | | | | |
| Pemulen^{®} TR 2 | | | | | | 0.3 | | | | | |
| Ethanol | | 5 | | 8 | | | | | | | 10 |
| Butylenglykol | 5 | | | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s., (pH 6,5 - 7,5) | | | | | | | | | | |

**Tabelle 14: Beispiele für getönte Tagespflege vom Typ O/W**

| **Inhaltstoff** | **1** | **2** | **3** | **4** | **5** | **6** | **7** | **8** |
|---|---|---|---|---|---|---|---|---|
| Eumulgin^{®} PRISMA | 0.5 | 1.0 | 2.0 | 0.5 | 0.5 | 2.0 | 2.0 | 1.0 |
| Emulgade^{®} Sucro | 1.0 | 1.0 | 1.5 | 1.5 | 2.0 | 1.0 | 2.0 | 1.5 |
| Cutina^{®} GMS-SE | 5.5 | | | | | | | 3.0 |
| Emulgade^{®} PL 68/50 | | 5.0 | | | | 2.0 | | |
| Eumulgin^{®} VL 75 | | | | 3.0 | | | 5.0 | |
| Tego Care^{®} 450 | | | | | | 2.0 | 2.0 | |
| Crodesta^{®} F-50 | | | | | 6.0 | | | |
| Amphisol^{®} K | | | | 2.0 | | | | |
| Lanette^{®} E | | 0.25 | | | 1.0 | 1.0 | | |
| Cutina^{®} FS 45 | 1.5 | | | | | | | |
| Eumulgin^{®} B 2 | | | 2.0 | | | | | 2.0 |
| Cutina^{®} PES | 1.0 | | 0.5 | 2.0 | | 0.2 | 0.5 | |
| Lanette^{®} O | | | 2.0 | | | | | 1.0 |
| Cutina^{®} MD | | 0.5 | 3.0 | 3.0 | | | | |
| Cetiol^{®} LC | 4.0 | 5.0 | | | | | | |
| Cosmedia^{®} DC | 0.5 | | | 1.0 | | | | 1.0 |
| Cetiol^{®} CC | 4.0 | | 4.0 | | 7.0 | 5.0 | 10.0 | |
| Tegosoft^{®} DEC | | 5.0 | 2.0 | 4.0 | 2.0 | 2.0 | | 6.0 |
| Dow Corning^{®} 245 | | 2.0 | | 2.0 | | | | 2.0 |
| Eutanol^{®} G 16 | 4.0 | | | | 3.0 | 3.0 | | |
| Myritol^{®} 331 | | 5.0 | | | 2.0 | 2.0 | 5.0 | |
| Uvinul^{®} T 150 | | | | 0.5 | | | | 0.5 |
| Uvasorb^{®} HEB | 2.0 | | | | | | 1.0 | 1.0 |
| Tinosorb^{®} M | | | 2.0 | | | | | 2.0 |
| Tinosorb^{®} S | | | | 3.0 | | | | 2.0 |
| Neo Heliopan^{®} AV | | | | 2.0 | | | 2.0 | |
| Heo Heliopan^{®} AP | | | | 1.0 | | | 1.0 | |
| Uvinul^{®} A plus | | | 1 | | | | 2.0 | 2.0 |
| Microna^{®} Matte White | 5.0 | 5.0 | | 5.0 | 5.0 | 5.0 | | 5.0 |
| Microna^{®} Matte Black | 0.3 | 0.3 | 0.1 | 0.3 | 0.3 | 0.3 | 0.4 | 0.3 |
| Microna^{®} Matte Yellow | 3.0 | 3.0 | | 3.0 | 3.0 | 3.0 | 2.0 | 3.0 |
| Microna^{®} Matte Red | 0.6 | 0.6 | 1.0 | 0.6 | 0.6 | 0.6 | 1.0 | 0.6 |
| Ronasphere^{®} LDP | 1.0 | 1.0 | | 1.0 | 1.0 | 1.0 | | 1.0 |
| Piqment White 6 | | | 6.0 | | | | 6.0 | |
| Dry Flow PC | | | | | | | 2.0 | 2.0 |
| Glycerin | 5.0 | 5.0 | 3.0 | 5.0 | 5.0 | 5.0 | 3.0 | |
| Cosmedia^{®} SP | | | 0.3 | | 0.2 | | | |
| Water, de-ionized, Preservative | ad 100 | | | | | | | |

**Tabelle 15: Beispiele für getönte Tagespflege vom Typ W/O**

| **Inhaltstoff** | **1** | **2** | **3** | **4** | **5** | **6** | **7** | **8** |
|---|---|---|---|---|---|---|---|---|
| Eumulgin^{®} SG | 0.1 | 0.2 | 0.15 | 0.3 | 0.1 | 0.05 | 0.1 | 0.01 |
| Emulgade^{®} Sucro | 0.1 | 0.2 | 0.1 | 0.3 | 0.2 | 0.4 | 0.1 | 0.1 |
| Dehymuls^{®} PGPH | 5.5 | | 4.0 | | | | | 3.0 |
| Lameform^{®} TGI | | 5.0 | | | | 2.0 | | |
| Abil^{®} EM 90 | | | | 3.0 | | | 5.0 | |
| Isolan^{®} GI 34 | | | | | | 2.0 | 2.0 | |
| Isolan^{®} PDI | | | | | 6.0 | | | |
| Admul^{®} WOL 1403 | | | | 2.0 | | | | |
| Dehymuls^{®} HRE 7 | | 1.0 | | | 1.0 | 1.0 | | |
| Monomuls^{®} 90-O18 | 1.5 | | | | | | | 2.0 |
| Cutina^{®} PES | 2.0 | 1.0 | | 4.0 | | 1.0 | 2.5 | 2.0 |
| Cera Bellina | | | 2.0 | | | | | 2.0 |
| Beeswax | | | 2.0 | | | 2.0 | | 1.0 |
| Microcristalline Wax | | 1.5 | 3.0 | 3.0 | | | | |
| Cetiol^{®} LC | 4.0 | 5.0 | | | | | | |
| Cosmedia^{®} DC | 1.0 | | | | 0.5 | | 1.0 | |
| Cetiol^{®} CC | 4.0 | | | | 7.0 | 5.0 | 10.0 | |
| Tegosoft^{®} DEC | | 5.0 | 2.0 | 4.0 | 2.0 | 2.0 | | 6.0 |
| Dow Corning^{®} 245 | | 2.0 | | 2.0 | | | | 2.0 |
| Eutanol^{®} G 16 | 4.0 | | | | 3.0 | 3.0 | | |
| Myritol^{®} 331 | | 5.0 | | | 2.0 | 2.0 | 5.0 | |
| Uvinul^{®} T 150 | | | | 0.5 | | | | 0.5 |
| Uvasorb^{®} HEB | | | 2.0 | | | | 1.0 | 1.0 |
| Tinosorb^{®} M | | | 2.0 | | | | | 2.0 |
| Tinosorb S | | | | 3.0 | | | | 2.0 |
| Neo Heliopan^{®} AV | | | | 2.0 | | | 2.0 | |
| Heo Heliopan^{®} AP | | | | 1.0 | | | 1.0 | |
| Uvinul^{®} A plus | | | 1.0 | | | | 2.0 | 2.0 |
| Microna^{®} Matte White | 5.0 | 5.0 | | 5.0 | 5.0 | 5.0 | | 5.0 |
| Microna^{®} Matte Black | 0.3 | 0.3 | 0.1 | 0.3 | 0.3 | 0.3 | 0.4 | 0.3 |
| Microna^{®} Matte Yellow | 3.0 | 3.0 | 3.5 | 3.0 | 3.0 | 3.0 | 2.0 | 3.0 |
| Microna^{®} Matte Red | 0,6 | 0,6 | 1,0 | 0,6 | 0,6 | 0,6 | 1,0 | 0,6 |
| Ronasphere^{®} LDP | 1.0 | 1.0 | | 1.0 | 1.0 | 1.0 | | 1.0 |
| Pigment White 6 | | | 6.0 | | | | 6.0 | |
| Dry Flow PC | | | | | | | 2.0 | 2.0 |
| Glycerin | 5.0 | 5.0 | 3.0 | 5.0 | 5.0 | 5.0 | 3.0 | |
| Water, de-ionized, Preservative | ad 100 | | | | | | | |

**Tabelle 16:**

| **Komponenten (INCI)** | **1** | **2** | **3** | **4** | **5** | **6** | **7** | **8** |
|---|---|---|---|---|---|---|---|---|
| Disodium Cetearyl Sulfosuccinate (Eumulgin^{®} PRISMA) | 0.1 | 0.05 | 0.2 | 0.15 | 0.2 | 0.9 | 1.1 | 0.05 |
| Sucrose Polystearate, Hydrogenated Polyisobutene (Emulgade^{®} Sucro) | 0.5 | 0.1 | 0.4 | 0.6 | 0.2 | 0.3 | 0.5 | 0.1 |
| Glyceryl Stearate, Ceteareth-20, Ceteareth-12, Cetearyl Alcohol, Cetyl Palmitate (Emulgade SE) | 6 | | 4.5 | | | 6 | | |
| Ceteareth-12 (Eumulgin^{®}B1) | | | | | | | | |
| Ceteareth-20 (Eumulgin^{®}B2) | | | 1 | | | | | |
| Aqua, Cetearyl Isononanoate, Glyceryl Stearate, Ceteareth-20, Ceteareth-12, Cetearyl Alcohol, Cetyl Palmitate (Emulgade^{®} CM) | | | | | 20 | | | |
| Polyglyceryl-3 Diisostearate (Lameform TGI) | | 3 | | | | | | 3 |
| Cocoglycerides (Novata® AB) | | | | | | | 4 | |
| Stearyl alcohol ( Lanette 18) | | | | 10 | | | | |
| Hydrogenated Castor Oil (Cutina^{®} HR) | | | | 3.7 | | | 6.5 | |
| Polyglyceryl-2 Dipolyhydroxystearate (Dehymuls^{®} PGPH) | | 1 | | | | | | |
| PEG-30 Digolyhydroxystearate (Dehymuls LE) | | | | | | | | 1 |
| Cetyl PEG/PPG-10/1 Dimethicone (Degussa) | | | | | | | | 0.5 |
| Sodium Cetearyl Sulfate (Lanette^{®} E) | 0.3 | | | | | 0.3 | | |
| Pentaerythrityl Distearate (Cutina^{®} PES) | 1.0 | 2 | 0.7 | 4.7 | 2 | 5 | 3.5 | 2 |
| Behenyl Alcohol (Lanette^{®} 22) | 2 | | | | | 4 | | |
| Dicaprylyl Carbonate (Cetiol^{®} CC) | | 7 | | | | | | 7 |
| Dicaprylylether (Cetiol^{®} OE) | 6 | | 5 | 9 | | 6 | 9 | |
| Cocoglycerides (Myritol^{®} 331) | | | | | | | | |
| Diethylhexylcyclohexane (Cetiol^{®} S) | | | 5 | 14.7 | | | 35 | |
| Cyclopentasiloxane | 3 | 5 | | 34 | | 3 | 14 | 5 |
| Cyclopentasiloxane and Dimethicone/ Vinyldimethicone Crosspolymer SFE 839 (GE Bayer) | | 3 | | | | | | 3 |
| Dimethicone | 1 | | | | | | | |
| Hydrogenated Dimer Dilinoleyl / Dimethylcarbonate Copolymer (Cosmedia^{®} DC) | 0.5 | 1 | 1.5 | 1 | 0.5 | 2 | 1 | 1 |
| Triethyl Citrate (Hydagen^{®} C.A.T) | | | 2 | | | | | |
| PEG-40 Hydrogenated Castor Oil | | | | | 1 | | | |
| Tocopheryl Acetate | | | | 1 | | | | |
| Aluminium Zirconium Tetrachlorohydrex GLY (Rezal 36) | 30 | 40 | | 22.9 | | 30 | 25 | 40 |
| Aluminum Chlorhydrate (Locron L) | | | 10 | | | | | |
| Chitosan (Hydagen^{®} DCMF) | 0.05 | | | | | | | |
| Glycolic Acid | 0.02 | | | | | | | |
| Glycerin | | 5 | 5 | | | | | 5 |
| Propylene Carbonate (Fluka) | | | | | | | 0.5 | |
| Quaternium-18 Hectorite (Bentone 18) | | | | | | | 1 | |
| Talc (Merck) | | | | | | 5 | 5 | |
| MgSO4x7H2O | | 1 | | | | | | 1 |
| Wasser | Ad 100 | | | | | | | |
| Perfume, Preservatives | q.s. | q.s. | q.s. | q.s. | q.s. | q.s. | q.s. | q.s. |

| | | | | | | | | |
|---|---|---|---|---|---|---|---|---|
| 1 - Antiperspirant / Deo Creme ; 2 - Antiperspirant Creme (W/0) ; 3 - Antiperspirant / Deo Spray ; 4 - Antiperspirant Stift mit Vitamin E ; 5 - Deodorant Wipe - Formulierung ; 6 - Antiperspirant Creme ; 7- Antiperspirant Creme «Soft Solid » ; 8- Antiperspirant Spray | | | | | | | | |

**Tabelle 17: O/W-Sonnenschutzemulsionen**

| **Komponente** | **1** | **2** | **3** | **4** | **5** | **6** | **7** | **8** | **9** | **10** | **11** |
|---|---|---|---|---|---|---|---|---|---|---|---|
| **C - Creme, L - Lotion, S - Spray** | **L** | **C** | **S** | **L** | **C** | **L** | **L** | **C** | **L** | **C** | **L** |
| Eumulgin^{®} PRISMA | 0.5 | 1.0 | 0.2 | 0.1 | 1.0 | 0.5 | 0.5 | 2.0 | 0.5 | 0.1 | 0.5 |
| Eumulgin^{®} Sucro | 2 | 2.5 | 2.5 | 1.0 | 2.5 | 2.5 | 2.5 | 2 | 2.5 | 1.7 | 1.5 |
| Tween^{®} 60 | | | | 0.2 | | | | | | | |
| Myrj^{®} 51 | | | | 0.5 | | | | | | | |
| Cutina^{®} E 24 | | | | 0.1 | | | | | | | |
| Hostaphat^{®} KL 340 N | | | | | | | | | 0.6 | | |
| Lanette^{®} E | | | 0.2 | | | | | | | | |
| Amphisol^{®} K | | | | | | | | | | 1 | |
| Natriumstearat | | | | | | | 1 | | | | |
| Emulgade^{®} PL 68/50 | | | 1 | | | | | | | | |
| Tego^{®} Care 450 | | | | | | | | | | 1 | |
| Cutina^{®} PES | | 0.2 | | | 0.4 | | | | | | |
| Cutina^{®} MD | 2 | | | 2 | | | 2 | | | 6 | |
| Lanette^{®} 14 | 1 | | | 1 | | | | 2 | | | 2 |
| Lanette^{®}O | 1 | 6 | | | 5 | 2 | | 2 | | | |
| Cosmedia^{®} DC | 1 | 1.5 | | 1 | 1 | | 2 | 2 | | | 2 |
| Antaron^{®}V 216 | | | 2 | | | 1.5 | | | 1 | 1 | |
| Emery 1780 | | | | | 0.5 | 0.5 | | | | | |
| Lanolin, wasserfrei USP | | | | | | | 5 | | | | |
| Myritol^{®} PC | | | | | 5 | | | | | | |
| Myritol^{®} 331 | 5 | | 8 | | | 6 | | 10 | | 2 | |
| Finsolv^{®} TN | | | 1 | | | | | 1 | 8 | | |
| Cetiol^{®} CC | | 2 | 5 | | | 4 | 4 | 2 | | 2 | |
| Cetiol^{®} OE | | | 3 | | | | | | 2 | 3 | |
| Dow Corning DC^{®} 244 | 4 | | 1 | | 5 | | | 2 | | | 2 |
| Dow Corning DC^{®} 2502 | | 1 | | | 2 | | | | | | |
| Squatol^{®} S | | | | | | | 4 | | | | |
| Silikonöl Wacker AK^{®} 350 | | 2 | | | | | | | | | |
| Cetiol^{®} 868 | | | | | 2 | | 4 | | | | 7 |
| Cetiol^{®} J 600 | | | | | 3 | 2 | | | | 5 | |
| Mineralöl | | | | 9 | | | | | | | |
| Cetiol^{®} B | | | 1 | | | | | | | 2 | |
| Eutanol^{®} G | | | | 2 | | | | | 4 | | |
| Eutanol^{®} G 16 | 4 | | | | | 4 | | | | | |
| Cetiol^{®} PGL | | 5 | | | | | | | | 5 | |
| Mandelöl | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl^{®} LS | | | | | | | | | | | |
| Neo Heliopan^{®} AP (Na-Salz) | | 1 | | | | | | | 1 | | |
| Neo Heliopan^{®} Hydro (Na-Salz) | 2 | | 2.2 | | | | | | 1 | | |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} A PLUS | | | | 2 | 1 | | | | | | |
| Uvinul^{®} T 150 | 2 | | | | 2.5 | | | 1 | | | |
| Tinosorb^{®} M | | | 3 | | | | 2 | | | | 3 |
| Tinosorb^{®} S | | | 1 | | | | 1,5 | | | | |
| Uvasorb^{®} HEB | | 1 | | | 1 | | | | | | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex^{®} T 2000 | | | | | 5 | | 3 | 3 | | | 4 |
| Veegum^{®} Ultra | 1.5 | | 0.75 | | | | | 1 | 1 | | |
| Keltrol^{®} T | 0.5 | | 0.25 | | | | | 0.5 | 0.5 | | |
| Cosmedia^{®} SP | | 0.5 | | | 0.5 | | 0.2 | 0.2 | | 0.2 | 0.2 |
| Carbopol^{®} 980 | | | | 0.2 | | 0.2 | | | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |
| Konservierungsmittel, NaOH, Wasser | q.s.ad 100 | | | | | | | | | | |

**Tabelle 18: O/W-Sonnenschutzemulsionen**

| **Komponente** | **12** | **13** | **14** | **15** | **16** | **17** | **18** | **19** | **20** | **21** | **22** |
|---|---|---|---|---|---|---|---|---|---|---|---|
| **C - Creme, L - Lotion** | L | C | L | C | L | C | S | C | C | L | L |
| Eumulgin^{®} SG | 0.4 | 1.0 | 0.3 | 1.5 | 0.6 | 1.2 | 0.3 | 0.6 | 2.0 | 0.4 | 0.4 |
| Emulgade^{®} Sucro | 2 | 1.8 | 2.5 | 1.5 | 1 | 2 | 2.5 | 3 | 2 | 1.5 | 1.5 |
| Cutina^{®} E 24 | | | | 0.5 | | | | | | | |
| Lanette^{®} E | | | | | | | 0.1 | | | | |
| Amphisol^{®} K | 0.5 | | | | | | 1 | | | | |
| Natriumstearat | | | | | 1 | | | | | | |
| Emulgade^{®} PL 68/50 | | 1.5 | | | | | | | | | |
| Tego^{®} Care 450 | | | | | | | | | 0.3 | | |
| Cutina^{®} PES | | 1 | | | 0.5 | | | 0.5 | | | |
| Cutina^{®} MD | 1 | | | 4 | 1 | 3 | | | | | 1 |
| Lanette^{®} 14 | | 2 | | | | | | | | 1 | |
| Lanette^{®} O | | | | 2 | | | | 2 | 1 | 1 | |
| Allianz^{®} OPT | 1 | | | 1 | 1 | | | 2 | | | 2 |
| Cosmedia^{®} DC | | 1.5 | 2 | | | 1.5 | 2 | | 1.5 | 1.5 | |
| Emery^{®} 1780 | | | | 1 | 1 | | | | | | |
| Lanolin, wasserfrei, USP | | | | | | 1 | 1 | | | | |
| Myritol^{®} PC | | | | | | | | | 5 | | |
| Myritol^{®} 331 | 12 | | 12 | | | 8 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Corninq DC^{®} 244 | | 2 | | | 1 | | | | | | |
| Dow Corninq DC^{®} 2502 | | 1 | | | 1 | | | 3 | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Mineralöl | | | | 10 | | | | | | | |
| Cetiol^{®} B | 4 | | 4 | | | | | 4 | | | |
| Eutanol^{®} G | | 3 | | | | 3 | | | | | |
| Eutanol^{®} G 16 S | 10 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz ) | | | | | | | | | | 3 | |
| Eusolex^{®} OCR | 6 | | 9 | | 5 | 7 | 9 | | 4 | | 7 |
| Neo Heliopan^{®} AP (Na-Salz) | | | | 0.5 | | 1 | | | | | |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} A PLUS | | | | | 1 | | 2 | | | | |
| Uvinul^{®} T 150 | 1 | | | | | | | | 1.3 | 1 | 1 |
| Tinosorb^{®} M | | | 6,5 | | | | | | | 4 | |
| Tinosorb^{®} S | | | 1 | | 2 | | | | | | |
| Uvasorb^{®} HEB | 1 | | | | | | | | | | 2 |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Cosmedia^{®} SP | | | 0.2 | 0.3 | | | 0.1 | | | 0.2 | |
| Pemulen^{®} TR 2 | | 0.3 | | 0.3 | | | | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 19: W/O-Sonnenschutzemulsionen**

| **Komponente** | **23** | **24** | **25** | **26** | **27** | **28** | **29** | **30** | **31** | **32** | **34** |
|---|---|---|---|---|---|---|---|---|---|---|---|
| **C - Creme, L - Lotion** | **C** | **L** | **C** | **L** | **C** | **L** | **L** | **L** | **L** | **C** | **C** |
| Eumulgin^{®} SG | 0.05 | 0.1 | 0.2 | 0.1 | 0.2 | 0.1 | 0.3 | 0.1 | 0.1 | 0.1 | 0.2 |
| Emulgade^{®} Sucro | 0.2 | 0.4 | 0.3 | 0.2 | 0.5 | 0.1 | 0.4 | 0.5 | 0.2 | 0.3 | 0.4 |
| Dehymuls^{®} PGPH | 4 | 2 | 1 | 3 | 3 | 1 | 1 | 2 | | 4 | 1 |
| Dehymuls^{®} LE | | | | | | | | | 2 | | |
| Monomuls^{®} 90-O18 | | | 2 | | | | | | | | |
| Lameform^{®} TGI | 2 | | 4 | | 3 | | | | | 1 | 3 |
| Abil^{®} EM 90 | | | | | | | 4 | | | | |
| Isolan^{®} PDI | | | | | | 4 | | 2 | 2 | | |
| Zinc Stearate | 1 | | | 1 | 1 | | | 1 | 1 | 1 | |
| Beeswax | 1 | | 5 | 1 | | | | 5 | 5 | 7 | 5 |
| Tego^{®} Care CG | | | | | 1 | | | | | | 0.5 |
| Cutina^{®} PES | 2 | 3 | 2 | 1 | 2 | 1 | 1 | 1 | 1 | 1 | 4 |
| Prisorine^{®} 3505 | | | 1 | | | 1 | 1 | | | | 1 |
| Cosmedia DC | 3 | 4 | 2 | 1 | 1 | 2 | 2 | 2 | 2 | 1 | 1 |
| Myritol^{®} 331 | 2 | | | | 3 | 6 | | | | | 8 |
| Finsolv^{®} TN | | | | 5 | | | 5 | | | | |
| Cetiol^{®} CC | 10 | 4 | 2 | | 4 | 2 | | | | 3 | 5 |
| Tegosoft DEC | | 4 | | 3 | 2 | | 5 | 5 | 5 | 3 | |
| Cetiol^{®} OE | | | | | 4 | | 5 | | | 2 | |
| Dow Corning^{®} DC 244 | | | 3 | | | | 2 | | | 4 | |
| Dow Corning^{®} DC 2502 | 1 | | 1 | | 2 | 1 | | | | | 1 |
| Silikonöl Wacker AK 350 | | 1 | | 4 | | | | 3 | 3 | | |
| Cetiol^{®} PGL | | 3 | | | | 4 | | | | | |
| Copherol^{®} F 1300 | 1 | | | | | | | | | | |
| Magnesiumsulfat x 7 Wasser | 1 | | | | | | | | | | |
| Neo Heliopan^{®} Hydro (Na-Salz) | 2 | | 2.2 | | 3 | 3 | | | | | 2 |
| Neo Heliopan^{®} 303 | | 5 | | | | | | | | | 4 |
| Uvasorb^{®} HEB | 1 | | | 1 | 1 | | | | | | 2 |
| Neo Heliopan^{®} MBC | 2 | | | | | 2 | 2 | 2 | 2 | | |
| Uvinul^{®} A plus | | | | | 2 | | | | | 3 | |
| Neo Heliopan^{®} AP (Na-Salz) | | 2 | 2 | | 1 | | | | | | 6 |
| Neo Heliopan^{®} AV | 3 | | 4 | 6 | 4 | 7.5 | 4 | 5 | 5 | | 1 |
| Uvinul^{®} T 150 | 1 | 1 | | | 2.5 | | | 1 | 1 | | |
| Parsol^{®} 1789 | 2 | 1 | | | | | 2 | | | 2 | |
| Zinkoxid NDM | | | | | | 10 | | 3 | | | 4 |
| Tinosorb^{®} M | | 3 | | 3 | | | | 2 | 2 | 2 | |
| Tinosorb^{®} S | | 3 | | 3 | | | | 2 | 2 | 2 | |
| Eusolex^{®} T Aqua | | | 8 | | | | | 5 | | | |
| Eusolex^{®} T 2000 | | | | | 5 | | 3 | 3 | 3 | | 4 |
| Ethanol | | | | | | | | | | 8 | |
| Glycerin | 5 | 3 | 3 | 3 | 5 | 3 | 2 | 3 | 3 | 4 | 3 |
| Wasser ad 100, Konservierungsmittel q.s. | | | | | | | | | | | |

**Sporty Looking Face Cream**

| **Phase** | **Component** | | **%** | **Function** |
|---|---|---|---|---|
| I. | EUMULGIN® PRISMA | Disodium Cetearyl Sulfosuccinate | 0,10 | Emulsifier (W/O) |
| | ULTRAGEL™ 300 | Polyquaternium-37 (EU 2006/257/EC) Polvquaternium-37 (CTFA) | 0,90 | Polymer, cationic |
| | CUTINA® PES | Pentaerythrityl Distearate | 2,00 | Consistency factor |
| | CUTINA® MD | Glyceryl Stearate | 2,00 | Consistency factor |
| | CETIOL® CC | Dicaprylyl Carbonate | 2,00 | Emollient |
| | CETIOL® SENSOFT | Propylheptyl Caprylate | 2,00 | Emollient |
| | COPHEROL® F 1300 C | Tocopherol | 0,50 | Active ingredient |
| | Eusolex OCR (Merck) | Octocrylene | 2,00 | UV filter, UV-B |
| | Parsol 1789 (DSM Nutritional Products, Inc.) | Butyl Methoxydibenzoylmethane | 0,60 | UV filter, UV-A |
| II. | Water, demin. | Aqua | 72,70 | |
| | Glycerin | Glycerin | 5,00 | Active, moisturizing |
| III. | Dihydroxyaceton | Dihydroxyaceton | 1,50 | Active ingredient |
| | Water, demin. | Aqua | 5,00 | |
| IV. | Ethanol (denatured) | Ethanol | 3,00 | Active, cooling |
| | HERBALIA® GINKGO CG | Ginkgo Biloba Extract (EU); Ginkgo Biloba Leaf Extract | 0,20 | Active ingredient |
| | | (US) | | |
| | Tapioca Starch (National Starch) | Tapioca Starch | 0,5 | Skin feel modifier |
| | Preservative | Preservative | q.s. | |
| | Perfume | Perfume | q.s. | |
| ph-value (as is) 4.0 | | | | |
| Viscosity (Brookfield RVF, 23°C, spindle TE; 4 rpm, Helipath) 50.000mPas | | | | |

### Selftanning Aprés -Lotion

| **Phase** | **Component** | | **%** | **Function** |
|---|---|---|---|---|
| I. | EUMULGIN® PRISMA | Disodium Cetearyl Sulfosuccinate | 0,08 | Emulsifier (O/W) |
| | ULTRAGEL® 300 | Polyquaternium-37 | 0,75 | Polymer, cationic |
| | CUTINA® PES | Pentaerythrityl Distearate | 2,00 | Consistency factor |
| | CUTINA® MD | Glyceryl Stearate | 2,00 | Consistency factor |
| | CETIOL® SB 45 | Butyrospermum Parkii (Shea Butter) | 1,50 | Emollient |
| | CETIOL® CC | Dicaprylyl Carbonate | 2,00 | Emollient |
| | CETIOL® 868 | Ethylhexyl Stearate | 1,00 | Emollient |
| | CETIOL® SenSoft | Propylheptyl Caprylate (proposed) | 2,00 | Emollient |
| II. | Glycerin | Glycerin | 5,00 | Active, moisturizing |
| | Water, demin. | Aqua | 70,67 | |
| III. | Water, demin. | Aqua | 5,00 | |
| | Dihydroxyaceton (Merck) | Dihydroxyaceton | 1,50 | Active ingredient |
| IV. | Ethanol (denatured) | ) Ethanol | 5,00 | Active, cooling |
| | ALOVERIA® | Aloe Barbadensis (EU); Aloe Barbadensis Leaf Extract (US) | 1,00 | Active ingredient |
| | Tapioca Starch (National Starch) | Tapioca Starch | 0,50 | Active ingredient |
| | Preservative | Preservative | q.s. | |
| | Perfume | Perfume | q.s. | |
| | Viscosity (Brookfield RVF, 23 °C, spindle 5, 10 rpm) 12400 mPas | | | |
| | pH-value (as is) 4,0 | | | |

### Selftanning Body-Lotion

| **Phase** | **Component** | | **%** | **Function** |
|---|---|---|---|---|
| I. | EUMULGIN® PRISMA | Disodium Cetearyl Sulfosuccinate | 0,08 | Emulsifier (W/O) |
| | ULTRAGEL® 300 | Polyquaternium-37 | 0,70 | Polymer, cationic |
| | CUTINA® PES | Pentaerythrityl Distearate | 2,00 | Consistency factor |
| | CUTINA® MD | Glyceryl Stearate | 2,00 | Consistency factor |
| | MYRITOL® 331 | Cocoglycerides | 3,00 | Emollient |
| | CETIOL® CC | Dicaprylyl Carbonate | 3,00 | Emollient |
| | COPHEROL® F 1300 C | Tocopherol | 0,50 | Active ingredient |
| II. | Glycerin | Glycerin | 5,00 | Active, moisturizing |
| | Water, demin. | Aqua | 75,02 | |
| III. | Dihydroxyaceton (Merck) | Dihydroxyaceton | 3,00 | Active ingredient |
| | Water, demin. | Aqua | 5,00 | |
| IV. | Tapioca Starch (National Starch) | Tapioca Starch | 0,50 | Active ingredient |
| | PLANTACTIV® CENTELLA | Asiaticoside (and) | 0,20 | Active ingredient |
| | | Asiatic Acid (and) | | |
| | | Madecassic Acid | | |
| | Preservative | Preservative | q.s. | |
| | Perfume | Perfume | q.s. | |
| | Viscosity (Brookfield RVF, 23 °C, spindle 5, 10 rpm) 14000 mPas | | | |
| | pH-value (as is) 3,9 | | | |

### Kosmetische Zubereitungen:

| | **1** | **2** | **3** | **4** | **5** | **6** | **7** |
|---|---|---|---|---|---|---|---|
| Eumulgin® Prisma | 0,25 | 0,25 | 0,25 | 0,25 | 0,25 | 0,25 | 0,25 |
| Cetiol® SenSoft | 4,00 | 4,00 | 4,00 | 4,00 | 4,00 | 3,00 | 4,00 |
| Cetiol® LC | 5,00 | 5,00 | 5,00 | 5,00 | 5,00 | 3,00 | 4,00 |
| Myritol® 331 | 5,00 | 5,00 | 5,00 | 5,00 | 5,00 | 5,00 | 5,00 |
| Cegesoft® PS 6 | | 5,00 | 5,00 | 5,00 | 5,00 | 5,00 | 3,00 |
| Zinc Oxide NDM | | 5,00 | 5,00 | 5,00 | 5,00 | 5,00 | |
| Copherol® 1250 | 0,50 | 0,50 | 0,50 | 0,50 | 0,50 | 0,50 | 0,50 |
| Cutina® PES | | | 3,00 | 3,00 | 3,00 | 3,00 | 3,00 |
| Cegesoft® SBE | | | | | | 2,00 | 2,00 |
| Eusolex® OCR | | | | | | | 10,00 |
| Parsol® 1789 | | | | | | | 5,00 |
| Parsol® SLX | | | | | | | 3,00 |
| Eusolex® T-AVO | | | | | | | 8,00 |
| Glycerin 99,5% | 5,00 | 5,00 | 5,00 | 5,00 | 5,00 | 5,00 | 5,00 |
| Water | 57,15 | 40,15 | 44,15 | 45,15 | 44,65 | 45,65 | 35,65 |
| Rheocare® TTN | 2,00 | 4,00 | 2,00 | 1,00 | 1,50 | 1,50 | 0,50 |
| Neo Heliopan® Hydro (15%) | 10,00 | 10,00 | 10,00 | 10,00 | 10,00 | 10,00 | 5,00 |
| Neo Heliopan® AP (15%) | 10,00 | 10,00 | 10,00 | 10,00 | 10,00 | 10,00 | 5,00 |
| Phenonip® XB | 1,00 | 1,00 | 1,00 | 1,00 | 1,00 | 1,00 | 1,00 |
| Euxy® K 100 | 0,10 | 0,10 | 0,10 | 0,10 | 0,10 | 0,10 | 0,10 |
| Ethanol | | 5,00 | | | | | |
| NaOH (50%) | q.s. | q.s. | q.s. | q.s. | q.s. | q.s. | q.s. |
| pH | 7,2 | 8,1 | 8,1 | 7,5 | 8,4 | 8,0 | 6,9 |
| Viscosity (Brookfield, RVF, 23°C, spindle TE with helipath, 4 rpm) | | | 362500 | | 362500 | 162500 | 16250 0 |
| Viscosity (Brookfield, RVF, 23°C, spindle 5, 10 rpm) | 6000 | 12000 | | 5200 | | | |

### Kosmetische Zubereitungen,:

| | **8** | **9** | **10** | **11** | **12** | **13** | **14** |
|---|---|---|---|---|---|---|---|
| Eumulgin® Prisma | 0,25 | 0,25 | 0,25 | 0,25 | 0,25 | 0,25 | 0,25 |
| Cetiol® SenSoft | 4,00 | 4,00 | 4,00 | 4,00 | 4,00 | 3,00 | 4,00 |
| Cetio®I LC | 5,00 | 5,00 | 5,00 | 5,00 | 5,00 | 3,00 | 4,00 |
| Myritol® 331 | 5,00 | 5,00 | 5,00 | 5,00 | 5,00 | 5,00 | 5,00 |
| Cegesoft® PS 6 | | 5,00 | 5,00 | 5,00 | 5,00 | 5,00 | 3,00 |
| Zinc Oxide NDM | | 5,00 | 5,00 | 5,00 | 5,00 | 5,00 | |
| Copherol® 1250 | 0,50 | 0,50 | 0,50 | 0,50 | 0,50 | 0,50 | 0,50 |
| Cutina® PES | | | 3,00 | 3,00 | 3,00 | 3,00 | 3,00 |
| Cegesoft® SBE | | | | | | 2,00 | 2,00 |
| Eusolex® OCR | | | | | | | 10,00 |
| Parsol® 1789 | | | | | | | 5,00 |
| Parsol® SLX | | | | | | | 3,00 |
| Eusolex® T-AVO | | | | | | | 8,00 |
| Glycerin 99,5% | 5,00 | 5,00 | 5,00 | 5,00 | 5,00 | 5,00 | 5,00 |
| Water | 57,15 | 40,15 | 42,15 | 44,15 | 43,65 | 44,65 | 34,65 |
| Rheocare® TTN | 2,00 | 4,00 | 4,00 | 2,00 | 2,50 | 2,50 | 1,50 |
| Neo Heliopan® Hydro (15%) | 10,00 | 10,00 | 10,00 | 10,00 | 10,00 | 10,00 | 5,00 |
| Neo Heliopan® AP (15%) | 10,00 | 10,00 | 10,00 | 10,00 | 10,00 | 10,00 | 5,00 |
| Phenonip® XB | 1,00 | 1,00 | 1,00 | 1,00 | 1,00 | 1,00 | 1,00 |
| Euxyl® K 100 | 0,10 | 0,10 | 0,10 | 0,10 | 0,10 | 0,10 | 0,10 |
| Ethanol | | 5,00 | | | | | |
| NaOH (50%) | q.s. | q.s. | q.s. | q.s. | q.s. | q.s. | q.s. |
| pH | 7,2 | 8,2 | 8,2 | 7,6 | 8,3 | 8,4 | 6,8 |
| Viscosity (Brookfield, | | | 362500 | | 300000 | 162500 | 287500 |
| RVF, 23°C, spindle TE with helipath, 4 rpm) | | | | | | | |
| Viscosity (Brookfield, RVF, 23°C, spindle 5, 10 rpm) | 3200 | 6000 | | 5200 | | | |

### Kosmetische Zubereitungen:

| | **15** | **16** | **17** | **18** | **19** | **20** | **21** |
|---|---|---|---|---|---|---|---|
| Eumulgin® Prisma | 0,25 | 0,25 | 0,25 | 0,25 | 0,25 | 0,25 | 0,25 |
| Cetiol® SenSoft | 4,00 | 4,00 | 4,00 | 4,00 | 4,00 | 3,00 | 4,00 |
| Cetiol®LC | 5,00 | 5,00 | 5,00 | 5,00 | 5,00 | 3,00 | 4,00 |
| Myritol® 331 | 5,00 | 5,00 | 5,00 | 5,00 | 5,00 | 5,00 | 5,00 |
| Cegesoft® PS 6 | | 5,00 | 5,00 | 5,00 | 5,00 | 5,00 | 3,00 |
| Zinc Oxide NDM | | 5,00 | 5,00 | 5,00 | 5,00 | 5,00 | |
| Copherol ®1250 | 0,50 | 0,50 | 0,50 | 0,50 | 0,50 | 0,50 | 0,50 |
| Cutina® PES | | | 3,00 | 3,00 | 3,00 | 3,00 | 3,00 |
| Cegesoft® SBE | | | | | | 2,00 | 2,00 |
| Eusolex® OCR | | | | | | | 10,00 |
| Parsol® 1789 | | | | | | | 5,00 |
| Parsol® SLX | | | | | | | 3,00 |
| Eusolex® T-AVO | | | | | | | 8,00 |
| Glycerin 99,5% | 5,00 | 5,00 | 5,00 | 5,00 | 5,00 | 5,00 | 5,00 |
| Water | 57,15 | 40,15 | 42,15 | 44,15 | 43,65 | 44,65 | 34,65 |
| Rheocare® TTN | 2,00 | 4,00 | 4,00 | 2,00 | 2,50 | 2,50 | 1,50 |
| Neo Heliopan® Hydro (15%) | 10,00 | 10,00 | 10,00 | 10,00 | 10,00 | 10,00 | 5,00 |
| Neo Heliopan® AP (15%) | 10,00 | 10,00 | 10,00 | 10,00 | 10,00 | 10,00 | 5,00 |
| Phenonip® XB | 1,00 | 1,00 | 1,00 | 1,00 | 1,00 | 1,00 | 1,00 |
| Euxyl® K 100 | 0,10 | 0,10 | 0,10 | 0,10 | 0,10 | 0,10 | 0,10 |
| Ethanol | | 5,00 | | | | | |
| NaOH (50%) | q.s. | q.s. | q.s. | q.s. | q.s. | q.s. | q.s. |
| pH | 7,2 | 7,5 | 7,7 | 7,6 | 8,0 | 7,9 | 6,8 |
| Viscosity (Brookfield, RVF, 23°C, spindle TE | | | 162500 | | 162500 | 150000 | 150000 |
| with helipath, 4 rpm) | | | | | | | |
| Viscosity (Brookfield, RVF, 23°C, spindle 5, 10 rpm) | 1800 | 3000 | | 3000 | | | |

### Appendix - Bestandteile: Handelsnamen und INCI Bezeichnung

AMP-95, INCI: Aminomethyl Propanol, Dow Chemical Co; Abil^{®} EM 90, INCI: Cetyl Dimethicone Copolyol, Tego Cosmetics (Goldschmidt); Allianz^{®} OPT, INCI: Acrylates/C12-22 Alkyl Methacrylate Copolymer, Rohm und Haas; Amphisol^{®} K, INCI: Potassium Cetyl Phosphate, Hoffmann La Roche; Admul® WOL 1403, INCI: Polyricinoleate of polyglycerol, Quest; Antaron^{®} V 220, INCI: PVP/Eicosene Copolymer, GAF General Aniline Firm Corp. (IPS-Global); Antaron^{®} V 216, INCI: PVP/Hexadecene Copolymer, GAF General Aniline Firm Corp. (IPS-Global); Arlacel^{®} 83, INCI: Sorbitan Sesquioleate, Uniqema (ICI Surfacants); Arlacel^{®} P 135, INCI: PEG-30 Dipolyhydroxystearate, Uniqema (ICI Surfacants); Carbopol^{®} 980, INCI: Carbomer, Goodrich; Carbopol^{®} 2984, INCI: Carbomer, Noveon, Inc.; Carbopol^{®} ETD 2001, INCI: Carbomer, Noveon, Inc.; Carbopol^{®} Ultrez 10, INCI: Carbomer; Noveon, Inc.; Cegesoft^{®} C17, Myristyl Lactate, Cognis GmbH; Cegesoft^{®} PFO, INCI: Passiflora Incarnata (EU); Cognis GmbH; Cegesoft^{®} PS 6, INCI: Olus, Cognis GmbH, Cegesoft® SH, INCI: Shorea Stenoptera Seed Butter Cognis GmbH; Ceraphyl^{®} 45, INCI: Diethylhexyl Malate, International Specialty Products; Cetiol^{®} 868, INCI: Ethylhexyl Stearate, Hersteller: Cognis GmbH; Cetiol^{®} A, INCI: Hexyl Laurate, Cognis GmbH; Cetiol^{®} B, INCI: Dibutyl Adipate, Cognis GmbH; Cetiol^{®} CC, INCI: Dicaprylyl Carbonate; Cognis GmbH; Cetiol^{®} J 600, INCI: Oleyl Erucate, Cognis GmbH; Cetiol^{®} LC, INCI: Coco-Caprylate/Caprate, Cognis GmbH; Cetiol® MM, INCI: Myristyl Myristate, Cognis GmbH; Cetiol^{®} OE, INCI: Dicaprylyl Ether, Cognis GmbH, Cetiol^{®} PGL, INCI: Hexyldecanol, Hexyldecyl Laurate, Cognis GmbH; Cetiol^{®} S, INCI: Diethylhexylcyclohexane, Cognis GmbH; Cetiol^{®} SB 45, INCI: Shea Butter Butyrospermum Parkii (Linne), Cognis GmbH; Cetiol^{®} SN, INCI: Cetearyl Isononanoate, Cognis GmbH; Cetiol®Sensoft INCI: Propylheptyl Caprylate; Copherol^{®} F 1300 C, INCI: Tocopherol, Cognis GmbH; Copherol 1250 C, INCI: Tocopheryl Acetate, Cognis GmbH; Cosmedia^{®} DC, INCI: Hydrogenated Dimer Dilinoleyl / Dimethylcarbonate Copolymer; Cognis GmbH; Cosmedia^{®} SP, INCI: Sodium Polyacrylate; Cognis GmbH; Crodesta® F-50, INCI Sucrosedistearate, Croda; Cutina^{®} E 24, INCI: PEG-20 Glyceryl Stearate; Cognis GmbH; Cutina^{®} HR, INCI: Hydrogenated Castor Oil, Cognis GmbH; Cutina^{®} MD, INCI:Glyceryl Stearate, Cognis GmbH; Cutina^{®} PES, INCI: Pentaerythrityl Distearate, Cognis GmbH; Cutina® FS-45, INCI: Palmitic Acid, Stearic Acid, Cognis GmbH; Cutina® GMS-SE, INCI Glyceryl Stearate SE, Cognis GmbH; Cutina® LM conc, INCI: Polyglyceryl-2 Dipolyhydroxystearate, Octyldodecanol, Copernicia Cerifera (Carnauba) Wax, Euphorbia Cerifera (Candelilla) Wax, Beeswax, Cetearyl Glucoside, Cetearyl Alcohol, Cognis GmbH; Dehymuls^{®} FCE, INCI: Dicocoyl Pentaerythrityl Distearyl Citrate, Cognis GmbH; Dehymuls^{®} HRE 7, INCI:PEG-7 Hydrogenated Castor Oil, Cognis GmbH; Dehymuls^{®} PGPH, INCI:Polyglyceryl-2 Dipolyhydroxystearate, Cognis GmbH; Dehymuls® LE, INCI: PEG-30 Dipolyhydroxystearate, Cognis GmbH; Dow Corning^{®} 244 Fluid, INCI: Cyclomethicone, Dow Corning; Dow Corning^{®}1503 Fluid, INCI: Dimethicone and Dimethiconol, Dow Corning; Dow Corning^{®} 246 Fluid, Cyclopentasiloxane, Dow Corning; Dow Corning^{®} 2502, INCI:Cetyl Dimethicone, Dow Corning; Dow Corning DC® 245 INCI: Cyclopentasiloxane, Dow Corning, Dehyquart® C 4046, INCI: Cetearyl Alcohol, Dipalmitoylethyl Hydroxyeethylmonium Methosulfate, Ceteareth-20, Cognis GmbH; Dry^{®}Flo Plus, INCI:Aluminium Starch Octenylsuccinate, National Starch; Dry® Flo PC, INCI: Aluminum Starch Octenylsuccinate, Akzo Nobel; Elfacos^{®}ST 37, INCI: PEG-22 Dodecyl Glycol Copolymer, Akzo-Nobel; Elfacos^{®}ST 9, INCI:PEG-45 Dodecyl Glycol Copolymer, Akzo-Nobel; Emery^{®} 1780, INCI: Lanolin Alcohol, Cognis Corp.; Emulgade^{®} CM, INCI: Cetearyl Isononanoate and Ceteareth-20 and Cetearyl Alcohol and Glyceryl Stearate and Glycerin and Ceteareth-12 and Cetyl Palmitate, Cognis GmbH; Emulgade^{®}PL 68/50, INCI: Cetearyl Glucoside, Cetearyl Alcohol, Cognis GmbH; Emulgade^{®} SE - PF, INCI: Glyceryl Stearate (and) Ceteareth-20 (and) Ceteareth-12 (and) Cetearyl Alcohol (and) Cetyl Palmitate; Cognis GmbH, Emulgade^{®}SUCRO, INCI: Sucrose Polystearate (and) Hydrogenated Polyisobutene, Cognis GmbH; Eumulgin^{®}B1, INCI: Ceteareth-12, Cognis GmbH, Eumulgin^{®} B 2, INCI: Ceteareth- 20, Cognis GmbH; Eumulgin^{®}HRE 40, INCI: PEG-40 Hydrogenated Castor Oil, Cognis GmbH; Eumulgin^{®} Prisma INCI: Disodium Cetearyl Sulfosuccinate; Eumulgin^{®}SG, INCI: Sodium Stearoyl Glutamate, Cognis GmbH; Eumulgin^{®} VL 75, INCI: Lauryl Glucoside (and) Polyglyceryl-2 Dipolyhydroxystearate (and) Glycerin; Cognis GmbH; Eusolex^{®} OCR, INCI: Octocrylene, Merck; Eusolex® 2292: INCI: Ethylhexyl Methoxycinnamate and BHT; Merck; Eusolex^{®}T-AVO, INCI: Titanium Dioxide (and) Silica, Merck; Eusolex^{®} T 2000, INCI: Titanium Dioxide, Alumina, Simethicone, Merck; Eusolex^{®}T AQUA, INCI: Water and Titanium Dioxide and Alumina and Sodium Metaphosphate and Phenoxyethanol and Sodium Methylparaben, Merck; Eutanol^{®}G, INCI: Octyldodecanol, Cognis GmbH; Eutanol^{®}G 16, INCI: Hexyldecanol, Cognis GmbH; Eutanol^{®}G 16 S, INCI: Hexyldecyl Stearate, Cognis GmbH; Euxyl® K100: INCI Benzyl Alcohol and Methylchloroisothiazolinone and Methylisothiazolinone; Finsolv^{®} TN, INCI: C 12/15 Alkyl Benzoate, Findex (Nordmann/Rassmann); Fitoderm®, INCI Squalane, Cognis GmbH; Generol^{®} R, INCI: Brassica Campestris (Rapseed) Sterols, Cognis GmbH; Glucate^{®}DO, INCI: Methyl Glucose Dioleate, NRC Nordmann/Rassmann; Hispagel^{®} 200, INCI: Glycerin, Glyceryl Polyacrylate, Cognis GmbH; Hostaphat^{®} KL 340 N, INCI: Trilaureth-4 Phosphate, Clariant; Hydagen^{®} C.A.T.,INCI Triethyl Citrate, Cognis GmbH; Hydagen^{®}CMF, INCI: Chitosan Glycolate, Cognis GmbH; Hydagen^{®}DCMF, INCI: Chitosan, Cognis GmbH; Imwitor® 372 P INCI: Gylceryl Stearate Citrate; Insect Repellent^{®}3535, INCI: Ethyl Butylacetylaminopropionate, EMD Chemicals Inc; Isolan^{®}PDI, INCI: Diisostearoyl Polyglyceryl-3 Diisostearate, Goldschmidt AG ; Isolan® GPS, INCI: Polyglyceryl-4 Diisostearate/Polyhydroxystearate/Sebacate, Evonik Goldschmidt; Isolan® GI 34, INCI: Polyglyceryl-4 Isostearate, Evonik Goldschmidt; Irwinol® LS 9319, INCI: Octyldecanol, Irvingia Gabonensis Kernel Butter, Hydrogenated CocoGlycerides, Keltrol^{®}T, INCI: Xanthan Gum, CP Kelco; Lameform^{®}TGI, INCI: Polyglyceryl-3 Diisostearate, Cognis GmbH; Lanette^{®}14, INCI: Myristyl Alcohol, Cognis GmbH; Lanette^{®}18, INCI: Stearyl Alcohol, Cognis GmbH; Lanette^{®}22, INCI: Behenyl Alcohol, Cognis GmbH; Lanette^{®}E, INCI: Sodium Cetearyl Sulfate, Cognis GmbH; Lanette^{®}O, INCI: Cetearyl Alcohol, Cognis GmbH; Locron^{®} L, INCI: Aluminium Chlorhydrate, Clariant; Lucentite^{®} SAN, INCI: Quaternium-18 Hectorit, Co-Op Chemical Co., Ltd.; Microna® Matte White ((INCI: Titanium Dioxide, Zinc Oxide); Microna® Matte Black (INCI: Iron Oxide; Mica); Microna® Matte Yellow (INCI: Iron Oxide; Mica); Microna® Matte Red (INCI: Iron Oxide; Mica), Cosmetic white C47056 (INCI: Titanium Dioxide, Mica); FDC Yellow 6 Al Lake C705270 (INCI: Colour Index 15985); DC Red 7 Ca Lake C 19003 (INCI: Colour Index 15850); Irodin 100 Silverpearl, (INCI: Mica, Titanium dioxide); Colophane Claire type Y (INCI: Colophonium); Mexoryl®XL: 2-(2H-Benzotriazol-2-yl)-4-methyl-6-(2-methyl-3-(1,3,3,3-tetramethyl-1-(trimethylsilyloxy) disiloxanyl)propyl) phenol INCI: Drometrizole trisiloxane; Monomuls^{®} 90-0 18, INCI: Glyceryl Oleate, Cognis GmbH; Monomuls® 90 L 12, INCI: Glyceryl Laurate, Cognis GmbH; Myrj^{®} 51, INCI: PEG-30-Sterate, Uniqema; Myritol^{®} 312, INCl: Caprylic/Capric Triglyceride, Cognis GmbH; Myritol^{®}331, INCI: Cocoglycerides, Cognis GmbH; Myritol^{®}PC, INCI:PropyleneGlycol Dicaprylate/ Dicaprate, Cognis GmbH; Neo Heliopan^{®} 303, INCI: Octocrylene, Symrise; Neo Heliopan^{®}AP, INCI: Disodium Phenyl Dibenzimidazole Tetrasulfonate, Symrise; Neo Heliopan^{®}AV, INCI: Ethylhexyl Methoxycinnamate, Symrise; Neo Heliopan^{®} BB, INCI: Benzophenone-3, Symrise; Neo Heliopan^{®} E 1000, INCI: Isoamyl-p-Methoxycinnamate, Symrise; Neo Heliopan^{®}Hydro, INCI: Phenylbenzimidazole Sulfonic Acid, Symrise; Neo Heliopan® HMS INCI: Homosalate; Neo Heliopan^{®} MBC, INCI: 4-Methylbenzylidene Camphor, Symrise; Neo Heliopan^{®} OS, INCI: Ethylhexyl Salicylate, Symrise; Novata^{®} AB, INCI:Cocoglycerides, Cognis GmbH; Parsol^{®} 1789, INCI: Butyl Methoxydibenzoylmethane, Hoffmann-La Roche (Givaudan); Phenonip® XB: INCI: Phenoxyethanol and Methylparaben and Propylparaben and Ethylparaben; Pemulen^{®} TR-2 Polymer, INCI: Acrylates / C10-30 Alkylacrylate Crosspolymer, Noveon, Inc.; Photonyl^{®}LS, INCI: Arginine, Disodium Adenosine Triphosphate, Mannitol, Pyridoxine HCL, Phenylalanine, Tyrosine, Laboratoires Serobiologiques (Cognis); Prisorine^{®} 3505, INCI: Isostearic Acid; Uniqema; Prisorine^{®} 3758, INCl: Hydrogenated Polyisobutene, Uniqema; Rezal 36G, INCI: Aluminum Zirconium Tetrachlorohydrex GLY, Reheis, Inc; Rheocare® C Plus, INCI Carbomer, Cognis GmbH; Rheocare® TTN, INCI Acrylates Copolymer, Cognis GmbH; Ronasphere® LDP (INCI: Silica, Titaniumdioxide, Iron Oxides); Squatol® S, INCl: Hydrogenated Polyisobutene, BASF Corp.; Poloxamer® 101, INCI: Poloxamer, BASF SE; SFE^{®}839, INCI: Cyclopentasiloxane and Dimethicone/Vinyl Dimethicone Crosspolymer, GE Silicones; Silikonöl Wacker AK^{®}350, INCI: Dimethicone, Wacker; Tego^{®}Care 450, INCI: Polyglyceryl-3 Methylglucose Distearate, Goldschmidt; Tego^{®}Care CG 90, INCI: Cetearyl Glucoside, Goldschmidt; Tegosoft^{®} DEC, INCI: Diethylhexyl Carbonate, Goldschmidt; Tinosorb^{®} S, INCI: Bis-Ethylhexyloxyphenol Methoxyphenyl Triazine; Ciba Specialty Chemicals Corporation; Tinosorb^{®} M, INCI: Methylene Bis-Benzotriazolyl Tetramethylbutylphenol, Ciba Specialty Chemicals Corporation; Tween^{®} 60, INCI: Polysorbate 60, Uniqema (ICI Surfactants), Uvasorb^{®} HEB, INCI: Diethylhexyl Butamido Triazone, 3V Inc.; Ultragel® 300 INCI: Polyquaternium-37; Unirep^{®} U-18, INCI: Dimethyl Phthalate and Diethyl Toluamide and Ethyl Hexanediol, Induchem AG; Uvinul^{®} T 150, INCI: Ethylhexyl Triazone, BASF; Uvinul^{®} A plus, INCI: Diethylamino Hydroxybenzoyl Hexyl Benzoate, BASF; Veegum^{®} Ultra, INCI: Magnesium Aluminium Silicate, R. T. Vanderbilt Company, Inc; Veegum^{®} Plus, INCI: Magnesium Aluminum Silicate and Cellulose Gum, R. T. Vanderbilt Company, Inc; Z-Cote^{®} HP 1, INCI: Zinc Oxide and Triethoxy-caprylylsilane, BASF, Zinc Oxide NDM, INCI: Zinc Oxide, Symrise.

## Patentansprüche

1. Mischung von Alkylsulfosuccinatmonoestern der allgemeinen Formel (I) und/oder (II) 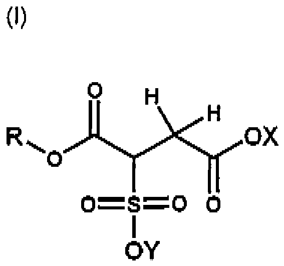 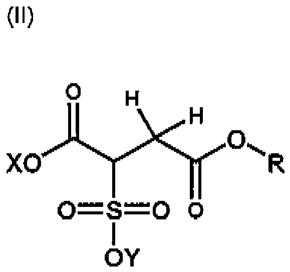
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, **dadurch gekennzeichnet, dass** die Mischung
- 30 bis 70 Gew.-% C16-Alkylsulfosuccinatmonoester und
- 30 bis 70 Gew.-% C16-Alkylsulfosuccinatmonoester enthält
wobei die Gew.-% bezogen sind auf die Gesamtmenge der Alkylsulfosuccinatmonoester der Formel (I) und (II).

2. Mischung nach Anspruch 1, **dadurch gekennzeichnet, dass** der Gehalt an Fettalkoholen kleiner gleich 10 Gew.-%, vorzugsweise kleiner gleich 5 Gew.-% - bezogen auf die Gesamtmenge der Alkylsulfosuccinatmonoester der Formel (I) und (II).

3. Verwendung einer Mischung nach einem der vorgenannten Ansprüche in kosmetischen und/oder pharmazeutischen Zubereitungen.

4. Verwendung einer Mischung nach mindestens einem der Ansprüche 1 oder 2 als grenzflächenaktive Substanz, insbesondere als Emulgator oder Dispergator, insbesondere als Emulgator oder Dispergator für UV-Lichtschutzfilter.

5. Kosmetische und/oder pharmazeutische Zubereitung, enthaltend in einem kosmetisch und/oder pharmazeutisch geeigneten Träger 0,1 bis 50, vorzugsweise 0,5 bis 10 Gew.-% bezogen auf die Zubereitung - einer Mischung von Alkylsulfosuccinatmonoester der allgemeinen Formel (I) und/oder (II) 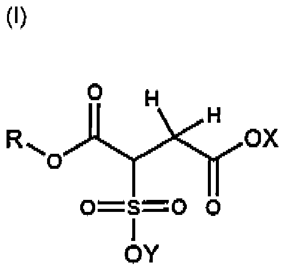 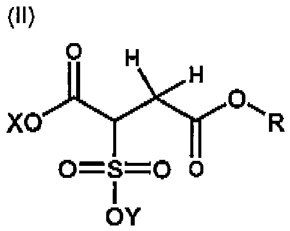
worin R für einen linearen oder verzweigten, gesättigten oder ungesättigten Alkylrest mit 6 bis 22 C-Atomen steht, X und Y unabhängig voneinander ein Wasserstoffatom oder ein zur Bildung eines wasserlöslichen Salzes befähigtes Kation darstellen, das ausgewählt ist aus der Gruppe bestehend aus Alkalimetall, Erdalkalimetall, Ammonium und organischem Ammonium, **dadurch gekennzeichnet, dass** die Mischung
- 30 bis 70 Gew.-% C16-Alkylsulfosuccinatmonoester und
- 30 bis 70 Gew.-% C18-Alkylsulfosuccinatmonoester enthält
wobei die Gew.-% bezogen sind auf die Gesamtmenge der Alkylsulfosuccinatmonoester der Formel (I) und (II).

6. Kosmetische und/oder pharmazeutische Zubereitung nach Anspruch 5, enthaltend weiterhin mindestens einen UV-Lichtschutzfilter

7. Kosmetische Zubereitung zur Färbung von Haut und Haar nach Anspruch 5, enthaltend weiterhin mindestens einen direktziehenden Farbstoff oder ein Oxidationsfarbstoffvorprodukt.

8. Kosmetische und/oder pharmazeutische Zubereitung nach Anspruch 5, enthaltend weiterhin mindestens einen Antiperspirant /Desodorant Wirkstoff.

9. Kosmetische und/oder pharmazeutische Zubereitung nach Anspruch 5, enthaltend weiterhin mindestens ein Pigment und/oder Farbstoff.

10. Kosmetische und/oder pharmazeutische Zubereitungen nach Anspruch 5, enthaltend weiterhin mindestens eine (weitere) grenzflächenaktive Substanz und/oder eine Wachskomponenten und/oder ein Polymer und/oder einen Ölkörper.

## Claims

1. Mixture of alkyl sulfosuccinate monoesters of the general formula (I) and/or (II) 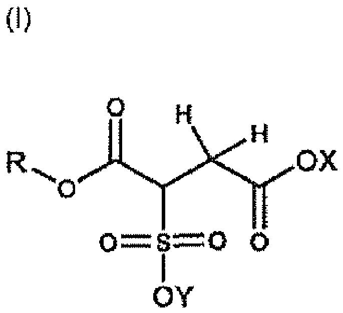 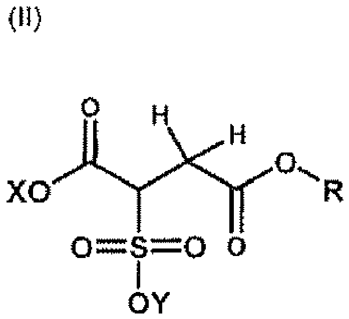
in which R is a linear or branched, saturated or unsaturated alkyl radical having 6 to 22 carbon atoms, X and Y are each independently a hydrogen atom or a cation which is capable of forming a water-soluble salt and is selected from the group consisting of alkali metal, alkaline earth metal, ammonium and organic ammonium, **characterized in that** the mixture comprises
- 30 to 70% by weight of C16-alkyl sulfosuccinate monoester and
- 30 to 70% by weight of C18-alkyl sulfosuccinate monoester,
where the percentages by weight are based on the total amount of the alkyl sulfosuccinate monoesters of the formulae (I) and (II).

2. Mixture according to Claim 1, **characterized in that** the content of fatty alcohols is less than or equal to 10% by weight, preferably less than or equal to 5% by weight, based on the total amount of the alkyl sulfosuccinate monoesters of the formulae (I) and (II).

3. Use of a mixture according to either of the preceding claims in cosmetic and/or pharmaceutical formulations.

4. Use of a mixture according to at least one of Claims 1 and 2 as an interface-active substance, especially as an emulsifier or dispersant, especially as an emulsifier or dispersant for UV photoprotective filters.

5. Cosmetic and/or pharmaceutical formulation comprising, in a cosmetically and/or pharmaceutically suitable carrier, 0.1 to 50% by weight, preferably 0.5 to 10% by weight, based on the formulation, of a mixture of alkyl sulfosuccinate monoesters of the general formula (I) and/or (II) 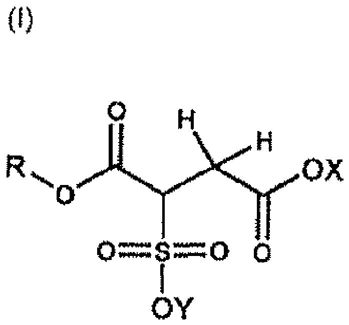 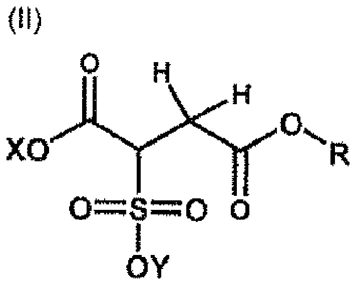
in which R is a linear or branched, saturated or unsaturated alkyl radical having 6 to 22 carbon atoms, X and Y are each independently a hydrogen atom or a cation which is capable of forming a water-soluble salt and is selected from the group consisting of alkali metal, alkaline earth metal, ammonium and organic ammonium, **characterized in that** the mixture comprises
- 30 to 70% by weight of C16-alkyl sulfosuccinate monoester and
- 30 to 70% by weight of C18-alkyl sulfosuccinate monoester,
where the percentages by weight are based on the total amount of the alkyl sulfosuccinate monoesters of the formulae (I) and (II).

6. Cosmetic and/or pharmaceutical formulation according to Claim 5, further comprising at least one UV photoprotective filter.

7. Cosmetic formulation for coloring skin and hair according to Claim 5, further comprising at least one direct dye or an oxidation dye precursor.

8. Cosmetic and/or pharmaceutical formulation according to Claim 5, further comprising at least one active antiperspirant/deodorant ingredient.

9. Cosmetic and/or pharmaceutical formulation according to Claim 5, further comprising at least one pigment and/or dye.

10. Cosmetic and/or pharmaceutical formulations according to Claim 5, further comprising at least one (further) interface-active substance and/or a wax component and/or a polymer and/or an oily substance.

## Revendications

1. Mélange de monoesters sulfosuccinate d'alkyle de formule (s) générale(s) (I) et/ou (II) 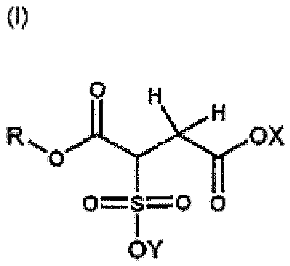 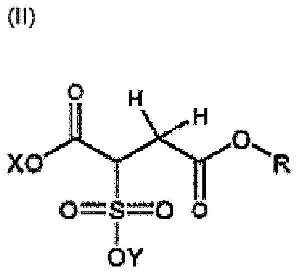
formules dans lesquelles R représente un radical alkyle linéaire ou ramifié, saturé ou insaturé, ayant de 6 à 22 atomes de carbone, X et Y représentent indépendamment l'un de l'autre un atome d'hydrogène ou un cation apte à la formation d'un sel hydrosoluble, qui est choisi dans le groupe constitué par un métal alcalin, un métal alcalino-terreux, le cation ammonium et un cation ammonium organique, **caractérisé en ce que** le mélange contient
- 30 à 70 % en poids de monoesters sulfosuccinate d'alkyle en C₁₆ et
- 30 à 70 % en poids de monoesters sulfosuccinate d'alkyle en C₁₈
les pourcentages en poids étant par rapport à la quantité totale des monoesters sulfosuccinate d'alkyle de formules (I) et (II).

2. Mélange selon la revendication 1, **caractérisé en ce que** la teneur en alcools gras est inférieure ou égale à 10 % en poids, de préférence inférieure ou égale à 5 % en poids - par rapport à la quantité totale des monoesters sulfosuccinate d'alkyle de formules (I) et (II).

3. Utilisation d'un mélange selon l'une quelconque des revendications précédentes, dans des préparations cosmétiques et/ou pharmaceutiques.

4. Utilisation d'un mélange selon au moins l'une quelconque des revendications 1 et 2 en tant que substance tensioactive, en particulier en tant qu'émulsifiant ou dispersant, en particulier en tant qu'émulsifiant ou dispersant pour des filtres photoprotecteurs anti-UV.

5. Préparation cosmétique ou pharmaceutique, contenant dans un véhicule cosmétiquement et/ou pharmaceutiquement approprié 0,1 à 50, de préférence 0,5 à 10 % en poids - par rapport à la préparation - d'un mélange de monoesters sulfosuccinate d'alkyle de formule (s) générale (s) (I) et/ou (II) 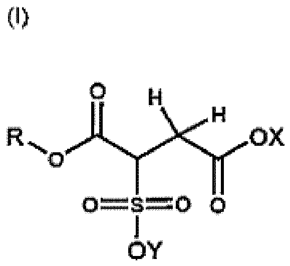 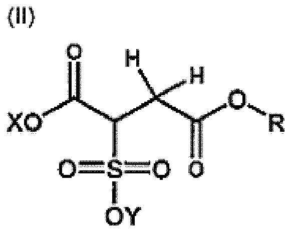
formules dans lesquelles R représente un radical alkyle linéaire ou ramifié, saturé ou insaturé, ayant de 6 à 22 atomes de carbone, X et Y représentent indépendamment l'un de l'autre un atome d'hydrogène ou un cation apte à la formation d'un sel hydrosoluble, qui est choisi dans le groupe constitué par un métal alcalin, un métal alcalino-terreux, le cation ammonium et un cation ammonium organique, **caractérisée en ce que** le mélange contient
- 30 à 70 % en poids de monoesters sulfosuccinate d'alkyle en C₁₆ et
- 30 à 70 % en poids de monoesters sulfosuccinate d'alkyle en C₁₈
les pourcentages en poids étant par rapport à la quantité totale des monoesters sulfosuccinate d'alkyle de formules (I) et (II).

6. Préparation cosmétique et/ou pharmaceutique selon la revendication 5, contenant en outre au moins un filtre photoprotecteur anti-UV.

7. Préparation cosmétique pour la coloration de la peau et des cheveux selon la revendication 5, contenant en outre au moins un colorant direct ou un précurseur de colorant d'oxydation.

8. Préparation cosmétique et/ou pharmaceutique selon la revendication 5, contenant en outre au moins une substance active antisudorale/ déodorante.

9. Préparation cosmétique et/ou pharmaceutique selon la revendication 5, contenant en outre au moins un pigment et/ou un colorant.

10. Préparations cosmétiques et/ou pharmaceutiques selon la revendication 5, contenant en outre au moins une (autre) substance tensioactive et/ou un composant cireux et/ou un polymère et/ou un corps huileux.
